# EUROPEAN PATENT APPLICATION

(11) **EP 4 631 956 A1**
(43) Date of publication of application: **15.10.2025**
(21) Application number: 23894017.5
(22) Date of filing: 24.11.2023
(51) Int. Cl.: C07K 5/062, C07D 207/02, A61K 38/00, A61P 35/00

(54) **LINKER, LINKER-CONTAINING ANTIBODY DRUG CONJUGATE, AND PREPARATION METHOD THEREFOR AND USE THEREOF**

(30) Priority: 25.11.2022 CN 202211489916
(71) Applicant: Chengdu Chipscreen Newway Biosciences Co., Ltd., Chengdu, Sichuan 610219 (CN); Shenzhen Chipscreen Biosciences Co., Ltd., Guangdong 518057 (CN)
(72) Inventor: LIU, Li, Chengdu, Sichuan 610219 (CN); LIU, Gang, Chengdu, Sichuan 610219 (CN); LIU, Bin, Chengdu, Sichuan 610219 (CN); PAN, Desi, Chengdu, Sichuan 610219 (CN); LU, Xianping, Chengdu, Sichuan 610219 (CN)
(74) Representative: Müller, Christian Stefan Gerd
(86) International application number: PCT/CN2023/133953
(87) International publication number: WO 2024/109928

(57) **Abstract**

Provided is a linker-containing antibody drug conjugate. Also involved are a pharmaceutical composition comprising the antibody conjugate, a use of a linker, and a use of the antibody conjugate in the preparation for a drug for preventing and/or treating diseases.

## Description

### TECHNICAL FIELD

The present disclosure relates to a linker for an antibody-drug conjugate, an antibody-drug conjugate prepared with the linker, a pharmaceutical composition comprising the antibody-drug conjugate, and use of the antibody-drug conjugate in the treatment and/or prevention of a disease.

### BACKGROUND

At present, traditional chemotherapy remains the primary treatment option for malignant tumors. It predominantly employs cytotoxic drugs and generally has shortcomings including poor targeting property, narrow safety window, strong toxic and side effects, susceptibility to drug resistance development, etc. The emergence of antibody-drug conjugates (ADCs) has provided a novel therapeutic approach for malignant tumors. The structure of an ADC comprises three key parts: an antibody, a small-molecule cytotoxic drug (cytotoxin), and a linker that achieves the organic conjugation of the two. ADCs utilize the targeting property of antibodies to concentrate cytotoxic drugs at target tumor sites so as to achieve enhanced efficacy and reduced toxicity, such that the released cytotoxic drugs can further kill adjacent tumor cells through the bystander effect. ADCs combine the dual advantages of high targeting property of monoclonal antibody drugs and high activity of cytotoxins in tumor tissues. They can kill tumor cells efficiently while demonstrating fewer side effects compared to chemotherapeutic drugs and superior therapeutic efficacy compared to traditional antibody-based tumor drugs. The characteristics of ADCs, including high activity, low toxic and side effects, and long duration of action, provide an innovative strategy for the "precision treatment" of tumors. Epigenetic dysregulation is frequently associated with human diseases, particularly cancer. Aberrant epigenetic regulation in cancer encompasses DNA methylation, histone methylation, histone acetylation, non-coding RNAs, and mRNA methylation. Epigenetic modifications can alter gene expression without changing the DNA sequence. Transcriptional abnormalities of oncogenes or tumor suppressor genes mediated by epigenetic enzymes are closely associated with the development, progression, and prognosis of tumors. Based on the reversible nature of epigenetic mechanisms, small-molecule compounds targeting epigenetic regulation have emerged as promising therapeutic drugs. These compounds target epigenetic regulatory enzymes, including histone modifiers (methylation and acetylation), DNA methylases, enzymes specifically recognizing post-translational modifications, chromatin remodeling enzymes, and post-transcriptional regulators (Jin Y, Liu T, Luo H, Liu Y, Liu D. Targeting Epigenetic Regulatory Enzymes for Cancer Therapeutics: Novel Small-Molecule Epidrug Development. Front Oncol. 2022;12:848221. Published 2022 Mar 28. doi: 10.3389/fonc.2022.848221). Epigenetic targeted drugs have demonstrated clinical efficacy in hematological malignancies and therapeutic potential in solid tumors (Jin N, George TL, Otterson GA, et al. Advances in epigenetic therapeutics with focus on solid tumors. Clin Epigenetics. 2021;13(1):83.). Peripheral T-cell lymphoma (PTCL) represents a paradigmatic epigenetic-related disease, exhibiting unique sensitivity to histone deacetylase (HDAC) and DNA methyltransferase (DNMT) inhibitors under both monotherapy and combination therapy (Scotto L, Kinahan C, Douglass E, et al. Targeting the T-Cell Lymphoma Epigenome Induces Cell Death, Cancer Testes Antigens, Immune-Modulatory Signaling Pathways. Mol Cancer Ther. 2021;20(8):1422-1430).

While demonstrating clinical efficacy in hematological tumors, epigenetic drugs as monotherapy have not shown significant therapeutic efficacy in solid tumors. Recent trials have indicated that these epigenetic drugs exhibit potential therapeutic efficacy when used in combination with chemotherapy or hormonal therapy. Given the novel mechanisms and great therapeutic potential of these drugs, it is important to reconsider optimal patient selection, medication regimens, study designs, and outcome measures (Juo YY, Gong XJ, Mishra A, et al. Epigenetic therapy for solid tumors: from bench science to clinical trials. Epigenomics. 2015;7(2):215-235. doi: 10.2217/epi.14.73).

Chidamide (tucidinostat) is a histone deacetylase (HDAC) inhibitor that has been used in clinical treatment. Studies have found that chidamide treatment in pancreatic cancer cell lines significantly reduces the expression of class I HDACs, Caspase-3, and p21, while increasing the expression ratio of Bax/Bcl-2. The study results suggest that chidamide may inhibit proliferation of pancreatic tumor cells by down-regulating the expression of class I HDACs and p21, while promoting mitochondrial apoptosis pathway-dependent cell death in a dose-dependent manner (Zhao B, He T. Chidamide, a histone deacetylase inhibitor, functions as a tumor inhibitor by modulating the ratio of Bax/Bcl-2 and P21 in pancreatic cancer. Oncol Rep. 2015;33(1):304-310. doi: 10.3892/or.2014.3595).

Research teams have discovered that chidamide can increase histone acetylation of the PD-L1 gene by activating the transcription factor STAT1. The HDAC gene family is frequently amplified in patients with soft tissue sarcoma. Based on pharmacogenomic target profiling, 8 of 11 (73%) liposarcoma patients exhibited broad amplification of the HDAC gene family. Analysis of the TCGA sarcoma cohort further validated HDAC gene family amplification in 76.65% (197/257) of cases. Expression of class I HDACs is associated with poor prognosis in patients with soft tissue sarcoma, and suppressing their expression can promote cell apoptosis and up-regulation of programmed cell death ligand 1 (PD-L1). The class I HDAC inhibitor chidamide can significantly increase PD-L1 expression in the tumor microenvironment, enhance CD8⁺ T cell infiltration, and reduce the number of MDSCs. In a mouse model, the combination of chidamide and an anti-PD-1 antibody significantly promotes tumor regression and improves survival rates. Furthermore, the combination of chidamide and the anti-PD-1 antibody toripalimab demonstrates therapeutic efficacy with tolerable side effects in patients with advanced and metastatic sarcomas (Que Y, Zhang XL, Liu ZX, et al. Frequent amplification of HDAC genes and efficacy of HDAC inhibitor chidamide and PD-1 blockade combination in soft tissue sarcoma. J Immunother Cancer. 2021;9(2):e001696. doi: 10.1136/jitc-2020-001696).

In order to meet more unmet clinical needs in the treatment of solid tumors, it is necessary to develop antibody-drug conjugates based on histone deacetylase inhibitors.

### SUMMARY

### Problems to be solved by the present disclosure

Firstly, to develop diversified stable ADCs that meet various clinical needs, novel linker-based stable ADCs still require further development. Through persistent efforts, the inventors of the present application have designed a linker having a structure represented by general formula (I).

Secondly, the inventors of the present application provide a conjugate having a structure represented by general formula (II) that exhibits excellent anti-tumor efficacy.

### Solutions for solving the problems

The inventors of the present application have attempted to conduct in-depth studies and found that a linker having a structure represented by general formula (I) and a conjugate having a structure represented by formula (II) can achieve the intended objects, thereby completing the present disclosure.

That is, the present disclosure relates to the following linker, and a conjugate comprising the linker, a stereoisomer thereof, a tautomer thereof, a pharmaceutically acceptable salt thereof, a deuterated compound thereof, or a solvate thereof.

In a first aspect, the present disclosure relates to a compound represented by formula I, a stereoisomer thereof, a tautomer thereof, a pharmaceutically acceptable salt thereof, a deuterated compound thereof, or a solvate thereof: 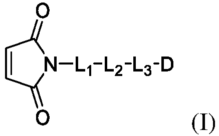 wherein
L₁ is selected from 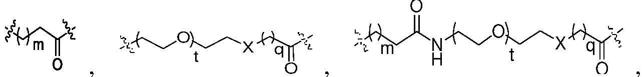 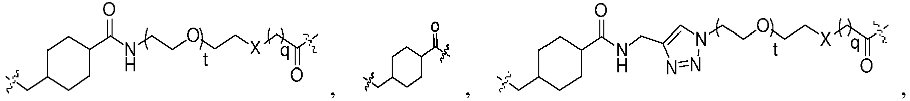 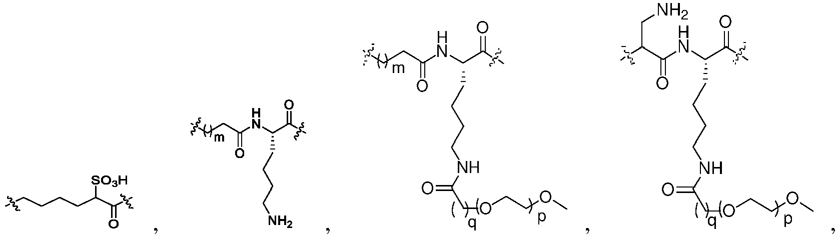 and 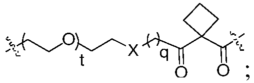
preferably, the carbon end of L₁ is linked to the N of succinimide, and the carbonyl end of L₁ is linked to L₂;
m and t are each independently selected from: 0, 1, 2, 3, 4, 5, 6, 7, and 8;
q is 0, 1, 2, or 3;
p is 0, 1, 2, 3, 4, 5, 6, 7, 8, 9, 10, 11, 12, 13, 14, or 15;
X is selected from: CH₂, O, and NH;
L₂ is absent, or is an amino acid residue, or is a peptide residue consisting of 2-10 amino acid residues;
preferably, the amino end of L₂ is linked to L₁, and the carbonyl end of L₂ is linked to L₃;
preferably, the amino acid includes, but is not limited to, phenylalanine (F), glycine (G), valine (V), lysine (K), serine (S), glutamic acid (E), asparagine (N), arginine (R), alanine (A), citrulline, and cysteine (C);
L₃ is absent, or is selected from 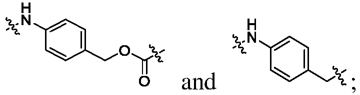
   preferably, the amino end of L₃ is linked to L₂, and the carbonyl end or carbon end of L₃ is linked to D;
   D is a histone deacetylase inhibitor drug selected from a thiol-based histone deacetylase inhibitor, a hydroxamic acid-based histone deacetylase inhibitor, and a benzamide-based histone deacetylase inhibitor.

In some embodiments, L₁ is selected from:

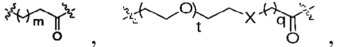

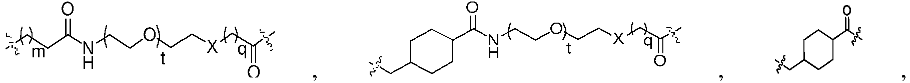

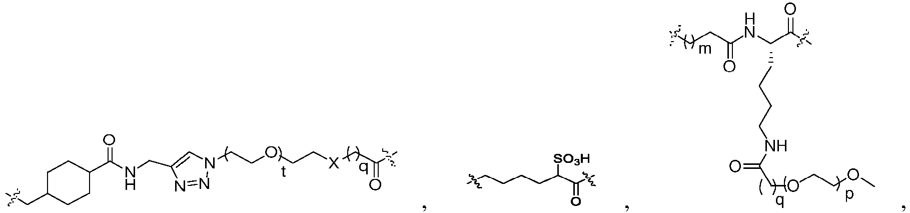

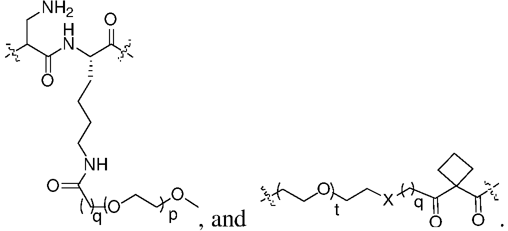

In some embodiments, L₁ is selected from: 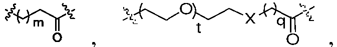 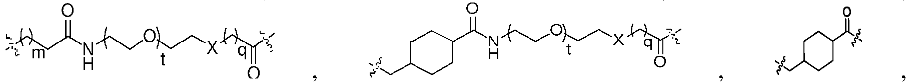 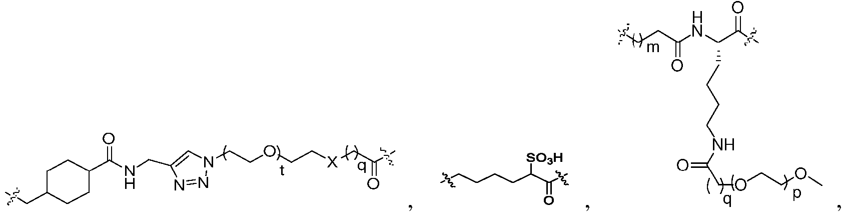 and

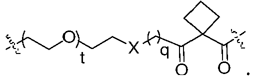

In some embodiments, m is selected from: 0, 1, 2, 3, 4, and 5. In some embodiments, m is selected from 1, 3, and 4.

In some embodiments, t is selected from: 0, 1, 2, 3, 4, 5, 6, and 7. In some embodiments, t is selected from 1, 2, 3, and 7.

In some embodiments, q is selected from 0, 1, and 2.

In some embodiments, p is selected from 10, 11, 12, 13, 14, and 15. In some embodiments, p is selected from 11, 12, and 13. In some embodiments, p is 12.

In some embodiments, X is selected from O and NH.

In some embodiments, L₁ is selected from

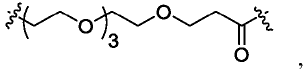

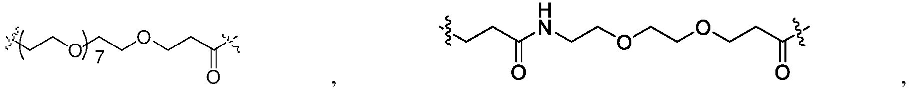

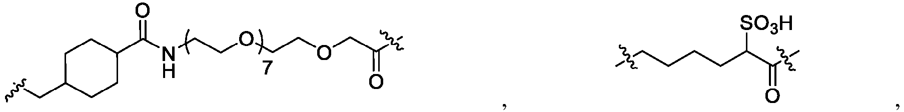

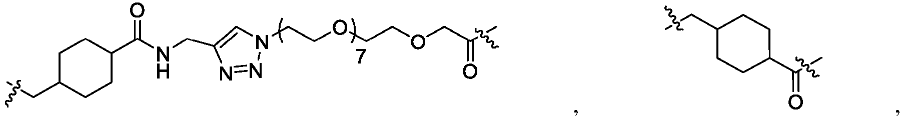

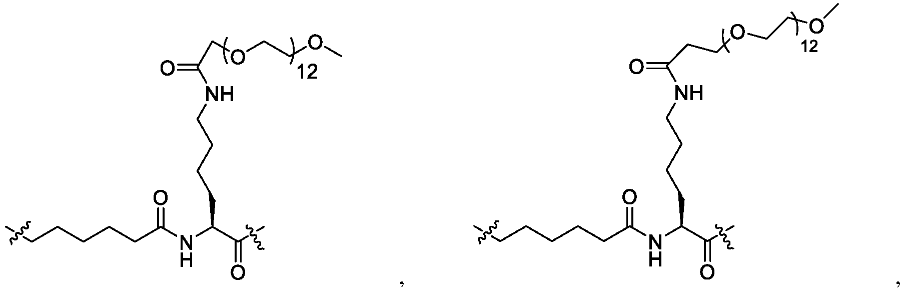

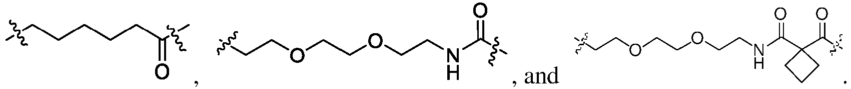

In some embodiments, L₁ is selected from

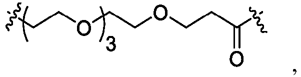

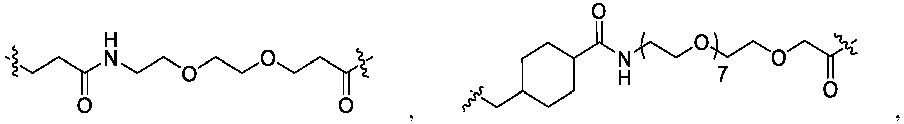

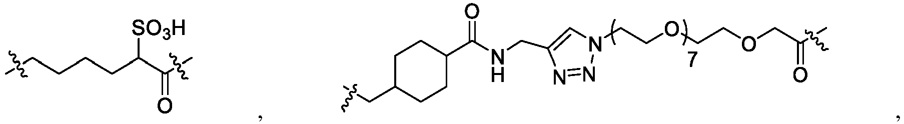

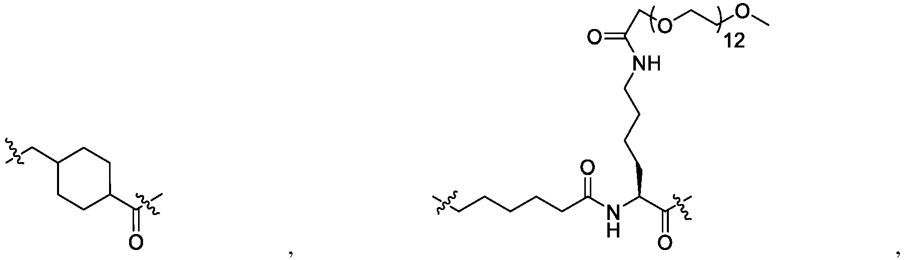

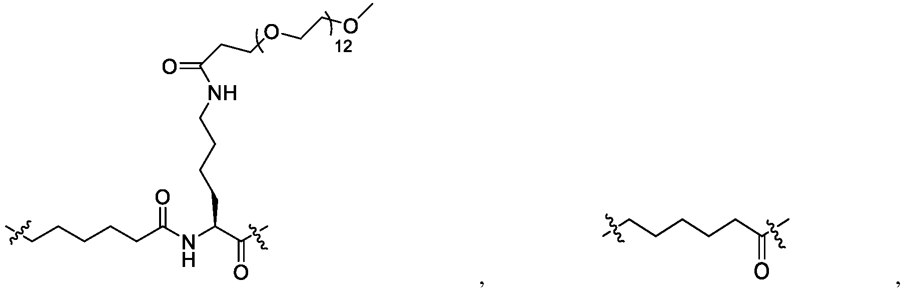

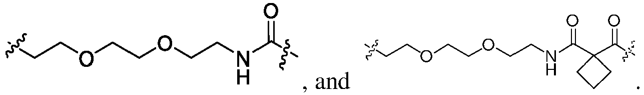

In some embodiments, L₁ is selected from

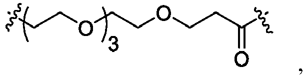

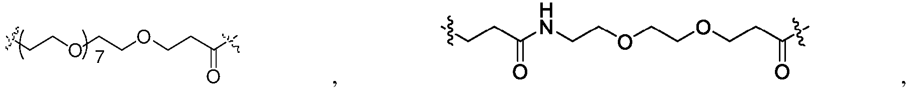

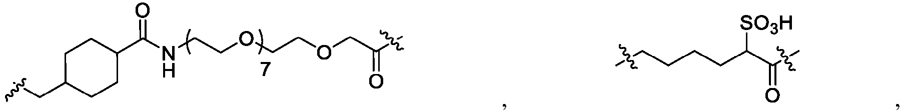

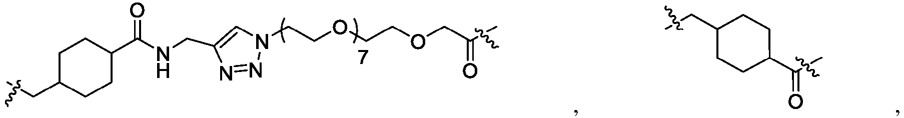

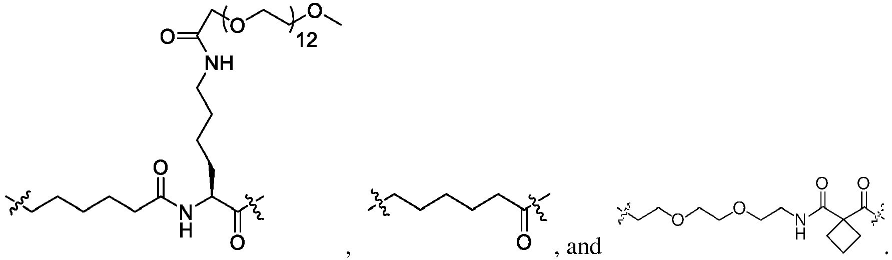

In some embodiments, L₁ is selected from 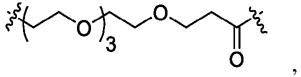 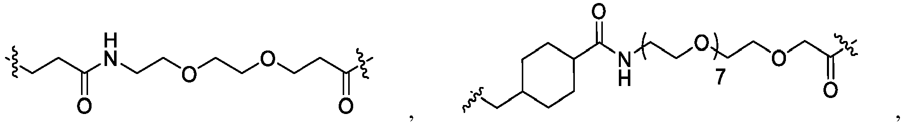 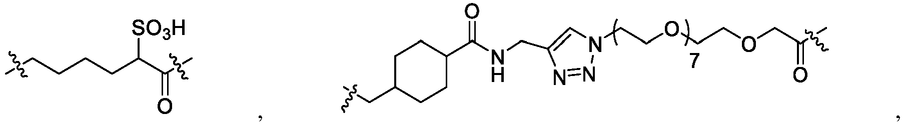 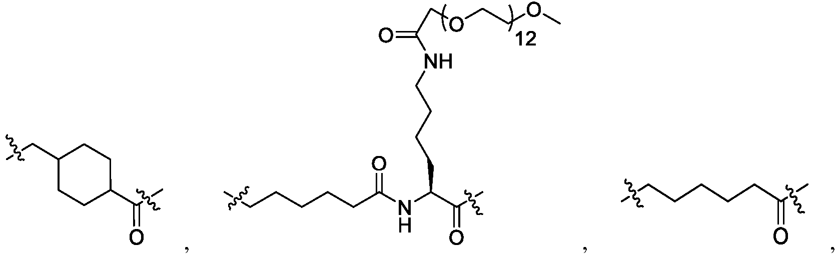 and

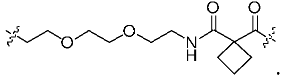

In some embodiments, L₁ is

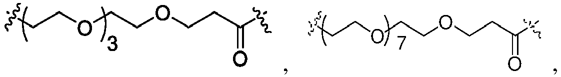

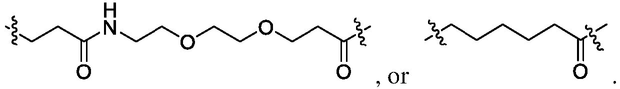

In some embodiments, L₁ is

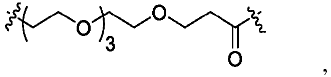

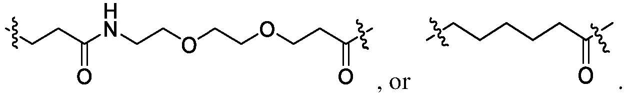

In some embodiments, L₁ is

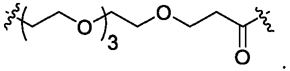

In some embodiments, L₁ is

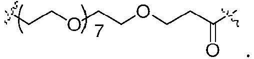

In some embodiments, L₂ is absent, or is an amino acid residue, or is a peptide residue consisting of 2-4 amino acid residues.

In some embodiments, the amino acid includes, but is not limited to, phenylalanine (F), glycine (G), valine (V), alanine (A), citrulline, and cysteine.

In some embodiments, the amino acid includes, but is not limited to, phenylalanine (F), glycine (G), valine (V), alanine (A), and citrulline.

In some embodiments, L₂ is absent, or is a citrulline residue, or is selected from the following peptide residues: valine residue-alanine residue, valine residue-citrulline residue, glycine amino-glycine residue-phenylalanine residue-glycine residue, valine residue-alanine residue-phenylalanine residue-glycine residue, glycine residue-glycine residue-glycine residue, glycine residue-phenylalanine residue-glycine residue, and valine residue-cysteine-phenylalanine residue-glycine residue.

In some embodiments, L₂ is absent, or is a citrulline residue, or is selected from the following peptide residues: valine residue-alanine residue, valine residue-citrulline residue, glycine amino-glycine residue-phenylalanine residue-glycine residue, and valine residue-alanine residue-phenylalanine residue-glycine residue.

In some embodiments, L₂ is absent, or is selected from

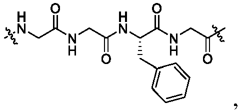

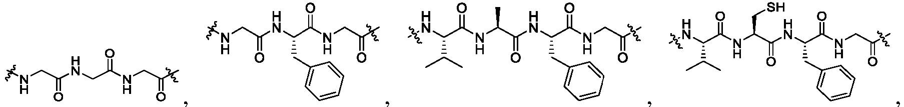

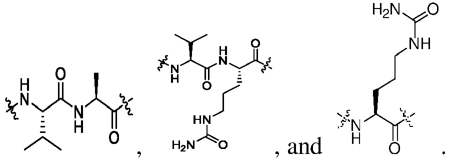

In some embodiments, L₂ is absent, or is selected from

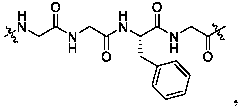

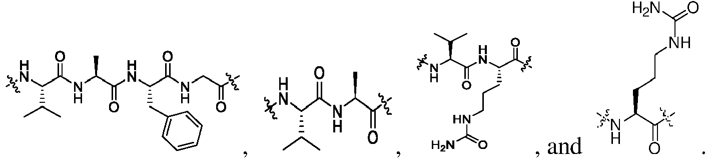

In some embodiments, L₂ is absent, or is selected from

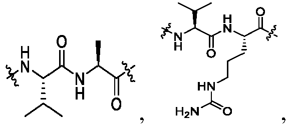

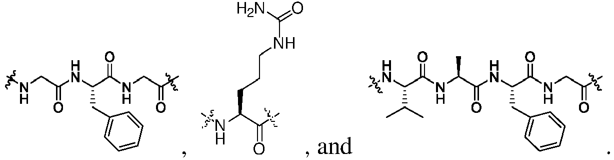

In some embodiments, L₂ is absent, or is selected from

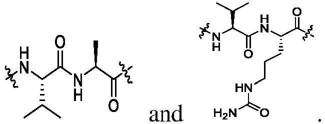

In some embodiments, L₂ is selected from

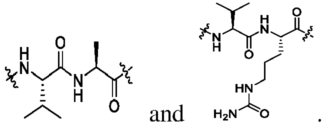

In some embodiments, L₂ is

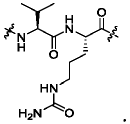

In some embodiments, L₃ is

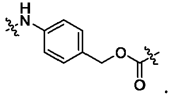

In some embodiments, D is a benzamide-based histone deacetylase inhibitor.

In some embodiments, D is selected from 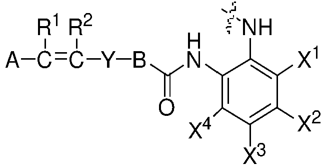 and 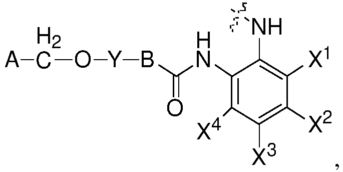 wherein
A is phenyl or pyridyl, and the phenyl or pyridyl is each independently and optionally substituted with 1-4 substituents selected from: halogen, C₁-C₄ alkyl (e.g., methyl and ethyl), and C₁-C₄ haloalkyl (e.g., trifluoromethyl);
preferably, A is phenyl or pyridyl, and the phenyl or pyridyl is each independently and optionally substituted with 1-2 substituents selected from: halogen, C₁-C₄ alkyl (e.g., methyl and ethyl), and trifluoromethyl;
more preferably, A is pyridyl, and the pyridyl is optionally substituted with 1-2 substituents selected from: C₁-C₄ alkyl;
further preferably, A is pyridyl;
most preferably, A is 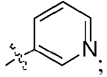
B is phenylene;
preferably, B is 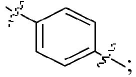
Y is -CO-NH-CH₂-;
preferably, the methylene end of Y is linked to B, and the carbonyl end of Y is linked to the alkenyl carbon or O;
R¹ and R² are each independently selected from hydrogen and C₁-C₄ alkyl;
preferably, R¹ and R² are both hydrogen;
any one of X¹, X², X³ , and X⁴ is selected from hydrogen, halogen, and C₁-C₄ alkyl, and the remaining three are hydrogen;
preferably, any one of X¹, X², X³, and X⁴ is selected from hydrogen and halogen, and the remaining three are hydrogen;
more preferably, any one of X¹, X², X³, and X⁴ is selected from hydrogen and fluorine, and the remaining three are hydrogen;
most preferably, X² is selected from hydrogen and fluorine, and X¹, X³, and X⁴ are hydrogen.

In some embodiments, D is selected from 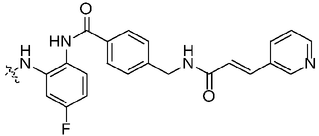 and

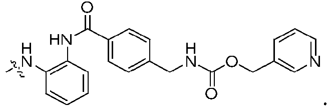

In some embodiments, D is

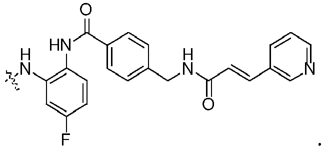

In some embodiments, the compound is selected from:

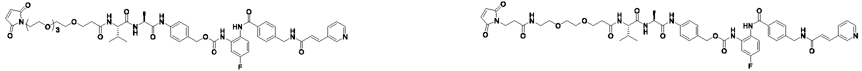

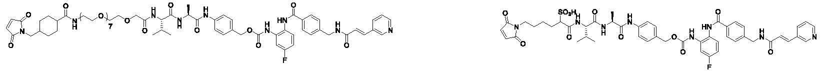

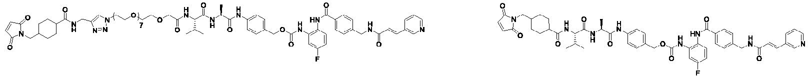

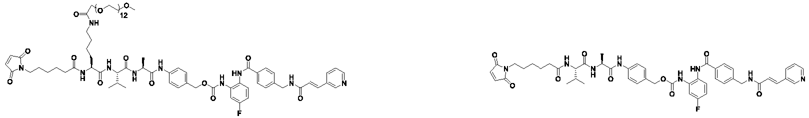

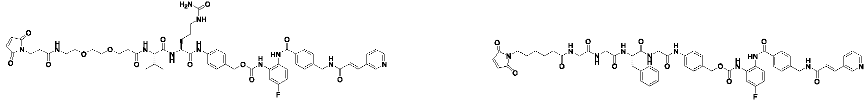

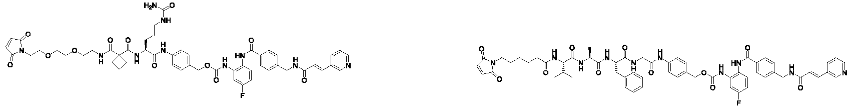

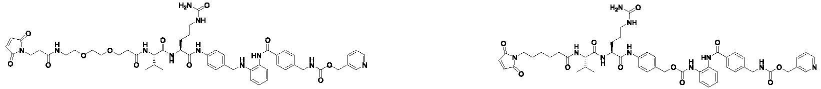

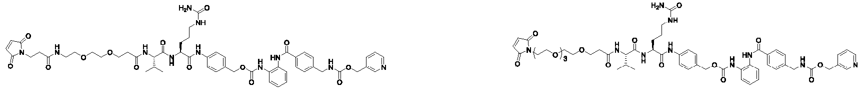

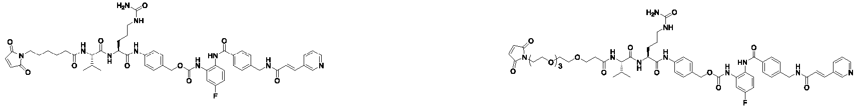

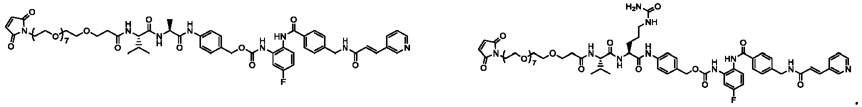

In a second aspect, the present disclosure relates to use of the compound, the stereoisomer thereof, the tautomer thereof, the pharmaceutically acceptable salt thereof, the deuterated compound thereof, or the solvate thereof described above in the preparation of a ligand-drug conjugate (e.g., an antibody-drug conjugate).

In a third aspect, the present disclosure relates to a ligand-drug conjugate represented by formula II, a stereoisomer thereof, a tautomer thereof, a pharmaceutically acceptable salt thereof, a deuterated compound thereof, or a solvate thereof: 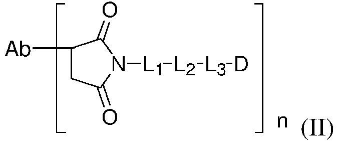 wherein
Ab is a ligand selected from a protein, an antibody, a polypeptide, an enzyme, and a small molecule;
n is a number between 0.5 and 8.5; for example, n is a number between 0.8 and 5, a number between 1 and 4, a number between 2 and 6, a number between 3 and 7, a number between 4 and 8, a number between 3.5 and 8.5, a number between 3.5 and 4.5, or a number between 6.5 and 8.5; preferably, n is about 1, 2, 3, 4, 5, 6, 7, or 8; preferably, n is about 2, 3, 4, 5, 6, 7, or 8; preferably, n is about 3, 4, 5, 6, 7, or 8;
L₁ is selected from 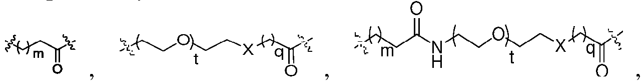 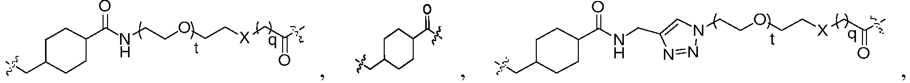 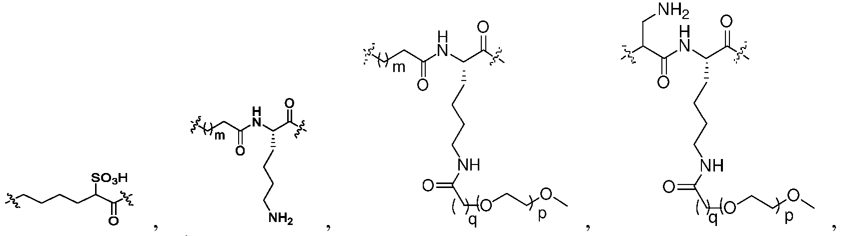 and 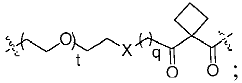
   preferably, the carbon end of L₁ is linked to the N of succinimide, and the carbonyl end of L₁ is linked to L₂;
   m and t are each independently selected from: 0, 1, 2, 3, 4, 5, 6, 7, and 8;
   q is 0, 1, 2, or 3;
   p is 0, 1, 2, 3, 4, 5, 6, 7, 8, 9, 10, 11, 12, 13, 14, or 15;
   X is selected from: CH₂, O, and NH;
   L₂ is absent, or is an amino acid residue, or is a peptide residue consisting of 2-10 amino acid residues;
   preferably, the amino end of L₂ is linked to L₁, and the carbonyl end of L₂ is linked to L₃;
   preferably, the amino acid includes, but is not limited to, phenylalanine (F), glycine (G), valine (V), lysine (K), serine (S), glutamic acid (E), asparagine (N), arginine (R), alanine (A), citrulline, and cysteine (C);
   L₃ is absent, or is selected from 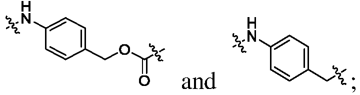
   preferably, the amino end of L₃ is linked to L₂, and the carbonyl end or carbon end of L₃ is linked to D;
   D is a histone deacetylase inhibitor drug selected from a thiol-based histone deacetylase inhibitor, a hydroxamic acid-based histone deacetylase inhibitor, and a benzamide-based histone deacetylase inhibitor.

In some embodiments, L₁ is selected from:

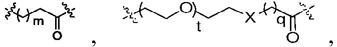

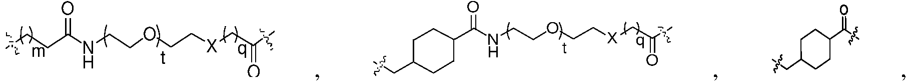

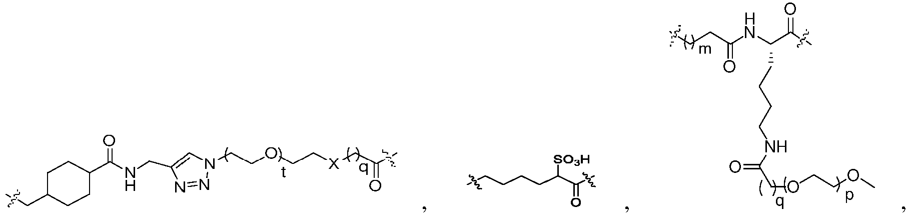

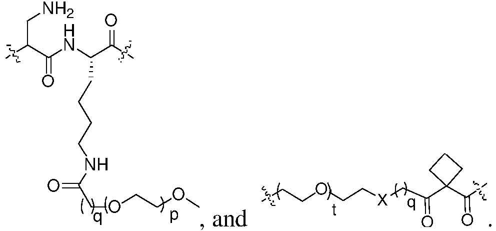

In some embodiments, L₁ is selected from: 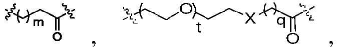 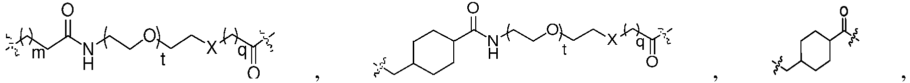 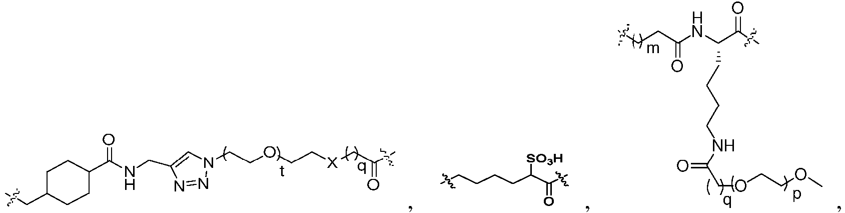 and

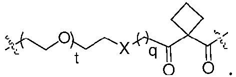

In some embodiments, m is selected from: 0, 1, 2, 3, 4, and 5. In some embodiments, m is selected from 1, 3, and 4.

In some embodiments, t is selected from: 0, 1, 2, 3, 4, 5, 6, and 7. In some embodiments, t is selected from 1, 2, 3, and 7.

In some embodiments, q is selected from 0, 1, and 2.

In some embodiments, p is selected from 10, 11, 12, 13, 14, and 15. In some embodiments, p is selected from 11, 12, and 13. In some embodiments, p is 12.

In some embodiments, X is selected from O and NH.

In some embodiments, L₁ is selected from

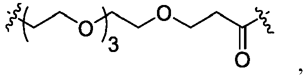

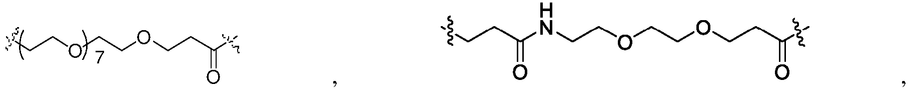

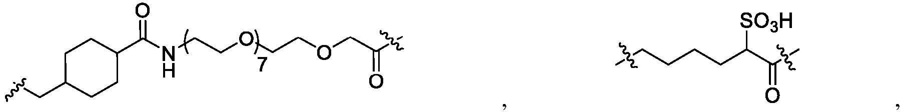

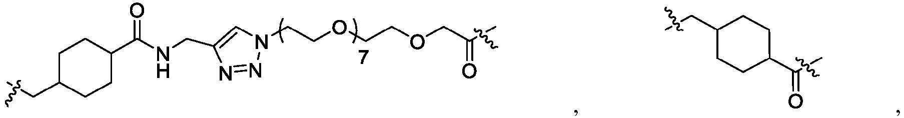

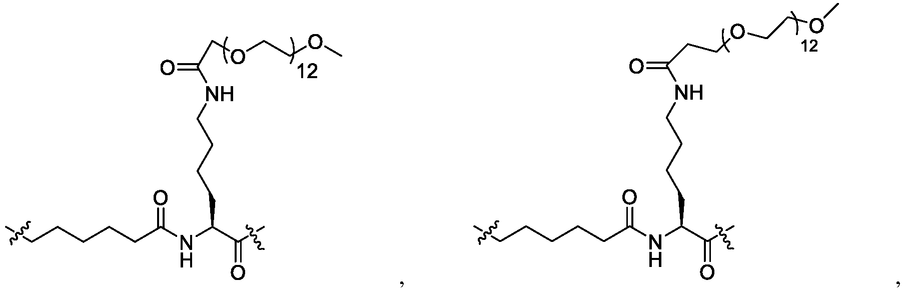

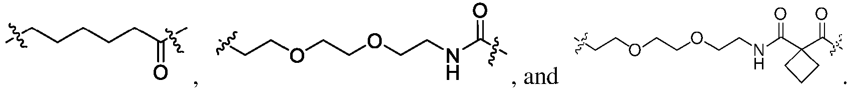

In some embodiments, L₁ is selected from

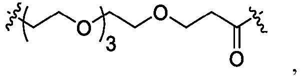

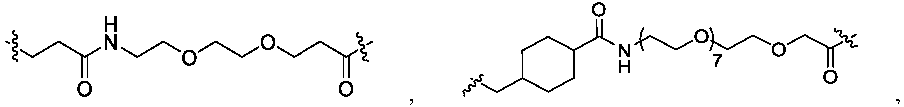

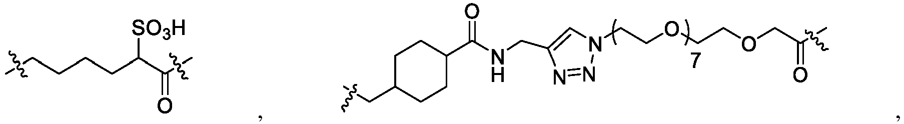

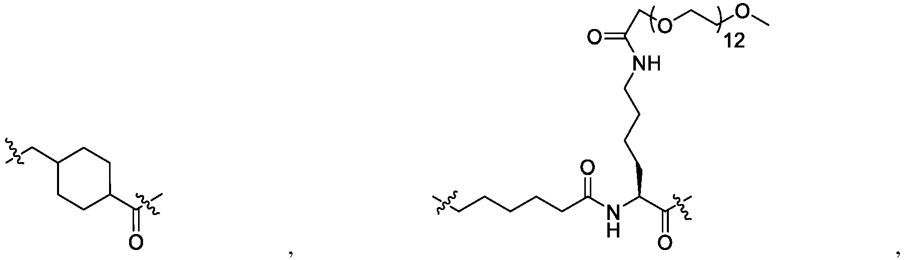

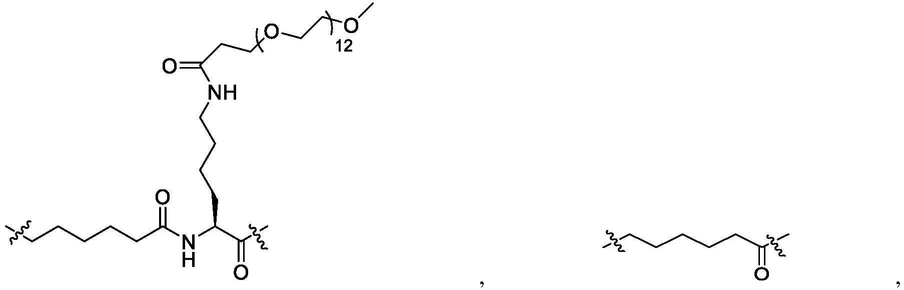

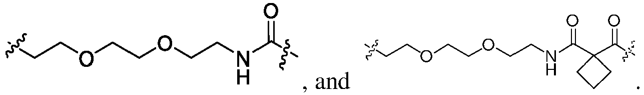

In some embodiments, L₁ is selected from

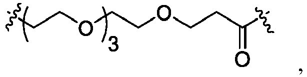

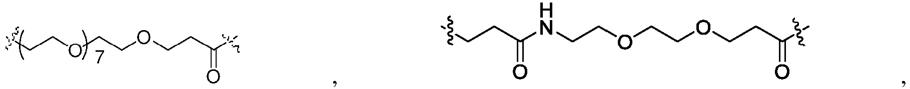

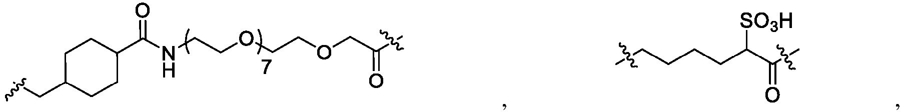

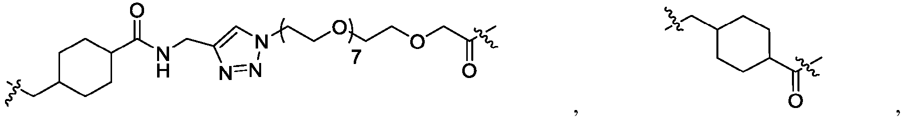

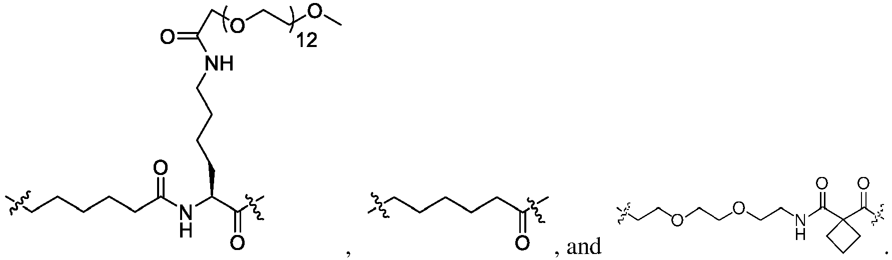

In some embodiments, L₁ is selected from 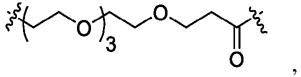 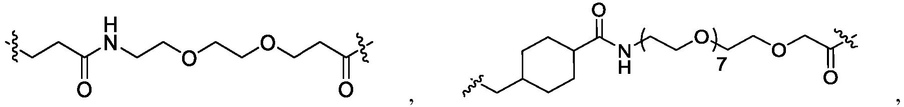 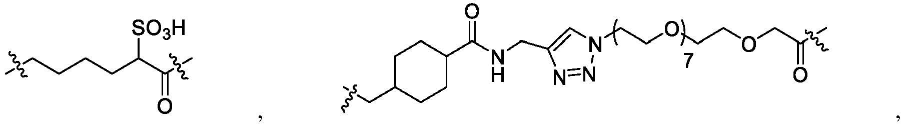 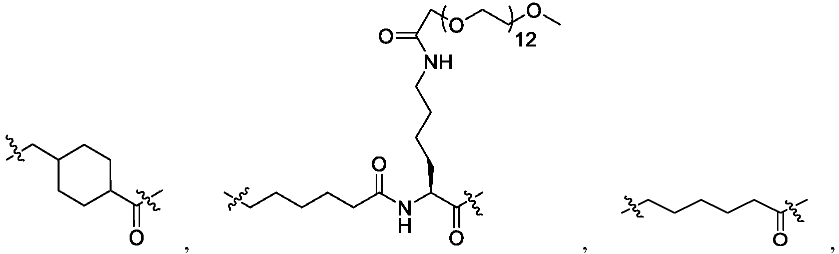 and

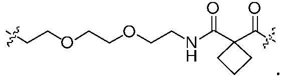

In some embodiments, L₁ is

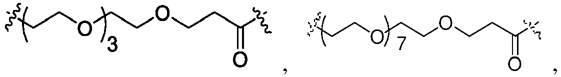

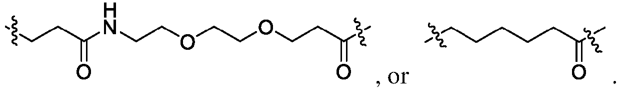

In some embodiments, L₁ is

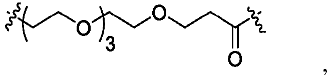

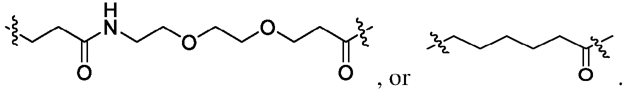

In some embodiments, L₁ is

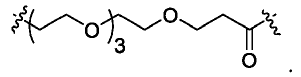

In some embodiments, L₁ is

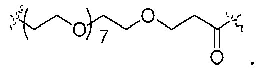

In some embodiments, L₂ is absent, or is an amino acid residue, or is a peptide residue consisting of 2-4 amino acid residues.

In some embodiments, the amino acid includes, but is not limited to, phenylalanine (F), glycine (G), valine (V), alanine (A), citrulline, and cysteine.

In some embodiments, the amino acid includes, but is not limited to, phenylalanine (F), glycine (G), valine (V), alanine (A), and citrulline.

In some embodiments, L₂ is absent, or is a citrulline residue, or is selected from the following peptide residues: valine residue-alanine residue, valine residue-citrulline residue, glycine amino-glycine residue-phenylalanine residue-glycine residue, valine residue-alanine residue-phenylalanine residue-glycine residue, glycine residue-glycine residue-glycine residue, glycine residue-phenylalanine residue-glycine residue, and valine residue-cysteine-phenylalanine residue-glycine residue.

In some embodiments, L₂ is absent, or is a citrulline residue, or is selected from the following peptide residues: valine residue-alanine residue, valine residue-citrulline residue, glycine amino-glycine residue-phenylalanine residue-glycine residue, and valine residue-alanine residue-phenylalanine residue-glycine residue.

In some embodiments, L₂ is absent, or is selected from

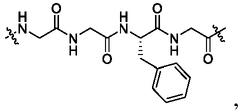

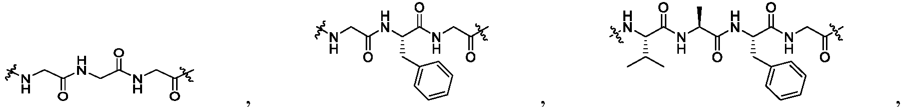

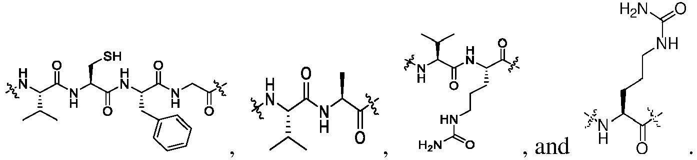

In some embodiments, L₂ is absent, or is selected from

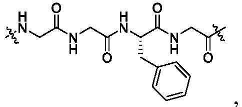

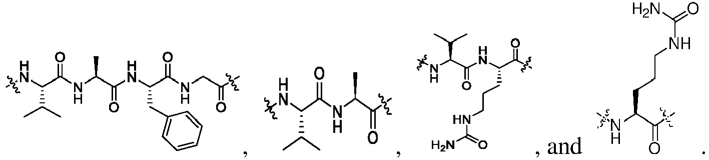

In some embodiments, L₂ is absent, or is selected from

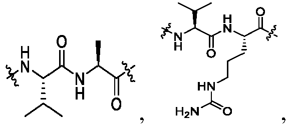

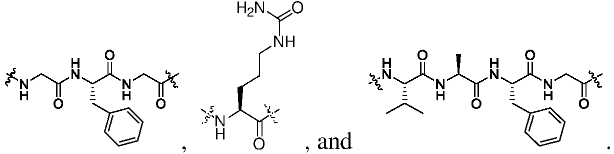

In some embodiments, L₂ is absent, or is selected from

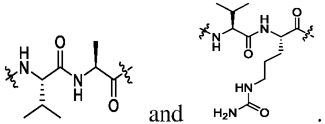

In some embodiments, L₂ is selected from

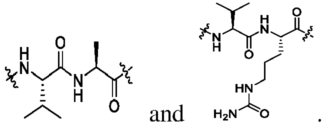

In some embodiments, L₂ is

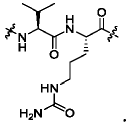

In some embodiments, L₃ is

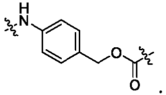

In some embodiments, D is a benzamide-based histone deacetylase inhibitor.

In some embodiments, D is selected from 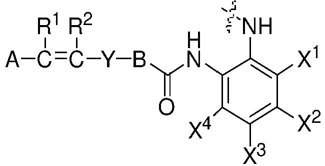 and 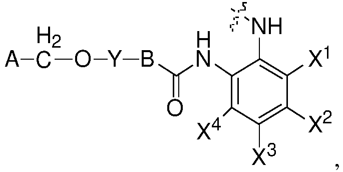 wherein
A is phenyl or pyridyl, and the phenyl or pyridyl is each independently and optionally substituted with 1-4 substituents selected from: halogen, C₁-C₄ alkyl (e.g., methyl and ethyl), and C₁-C₄ haloalkyl (e.g., trifluoromethyl);
preferably, A is phenyl or pyridyl, and the phenyl or pyridyl is each independently and optionally substituted with 1-2 substituents selected from: halogen, C₁-C₄ alkyl (e.g., methyl and ethyl), and trifluoromethyl;
more preferably, A is pyridyl, and the pyridyl is optionally substituted with 1-2 substituents selected from: C₁-C₄ alkyl;
further preferably, A is pyridyl;
most preferably, A is 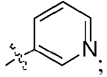
B is phenylene;
preferably, B is 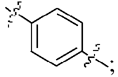
Y is -CO-NH-CH₂-;
preferably, the methylene end of Y is linked to B, and the carbonyl end of Y is linked to the alkenyl carbon or O;
R¹ and R² are each independently selected from hydrogen and C₁-C₄ alkyl;
preferably, R¹ and R² are both hydrogen;
any one of X¹, X², X³ , and X⁴ is selected from hydrogen, halogen, and C₁-C₄ alkyl, and the remaining three are hydrogen;
preferably, any one of X¹, X², X³, and X⁴ is selected from hydrogen and halogen, and the remaining three are hydrogen;
more preferably, any one of X¹, X², X³, and X⁴ is selected from hydrogen and fluorine, and the remaining three are hydrogen;
most preferably, X² is selected from hydrogen and fluorine, and X¹, X³, and X⁴ are hydrogen;
more preferably, D is selected from 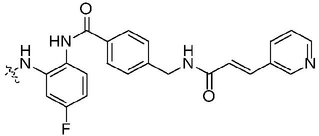 and

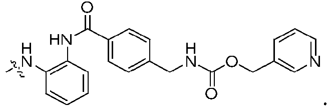

In some embodiments, D is

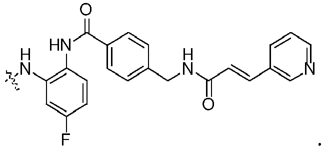

In some embodiments, Ab is an antibody selected from, without limitation: an anti-EGFR antibody, an anti-CD20 antibody, and an anti-PD-L1 antibody.

In some embodiments, Ab is selected from, without limitation, cetuximab, panitumumab, necitumumab, rituximab, tositumomab (tositumomab + iodine 131 tositumomab), ofatumumab, obinutuzumab, ocrelizumab, atezolizumab, avelumab, durvalumab, and cemiplimab (cemiplimab-rwlc).

In some embodiments, Ab is atezolizumab or avelumab.

In some embodiments, the ligand-drug conjugate is selected from:

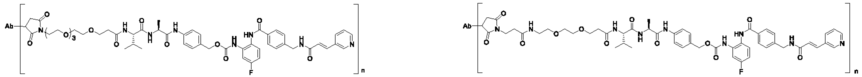

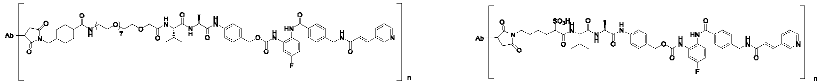

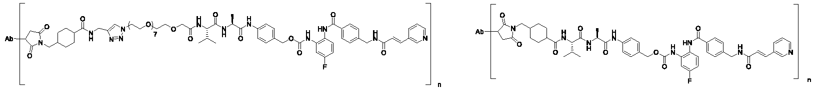

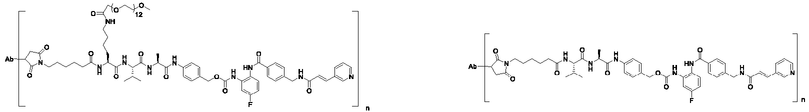

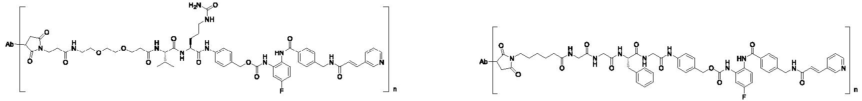

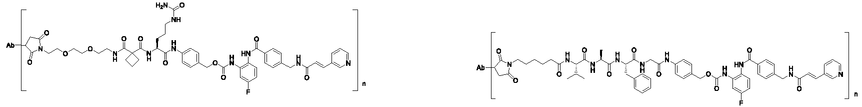

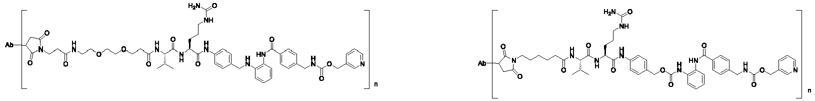

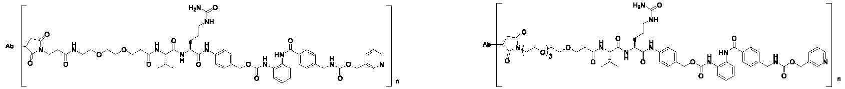

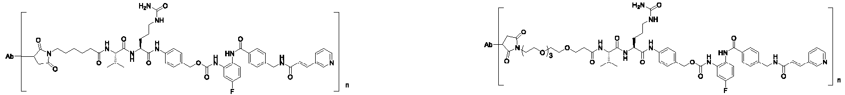

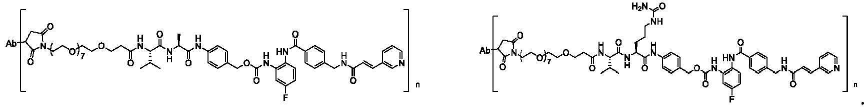

In some embodiments, the ligand-drug conjugate is selected from:

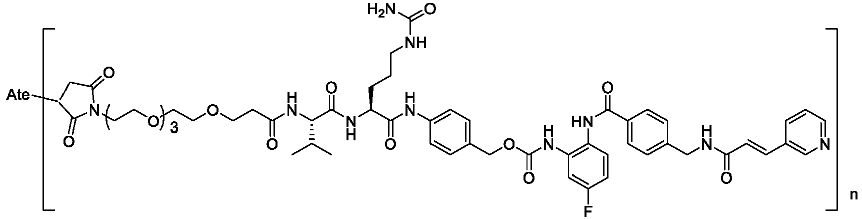

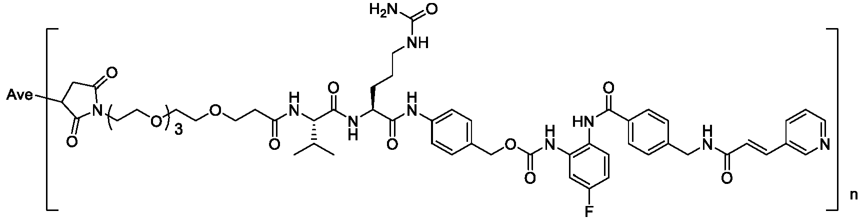

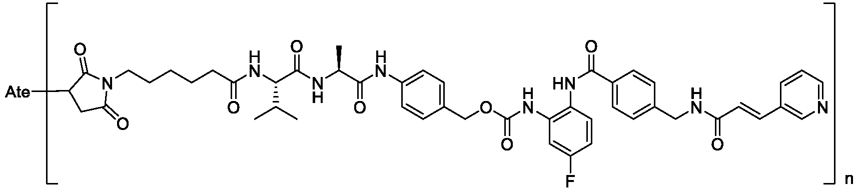

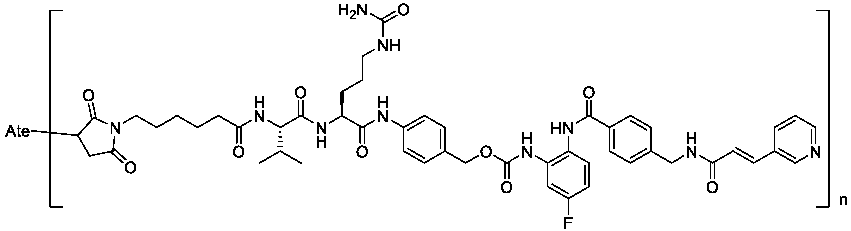

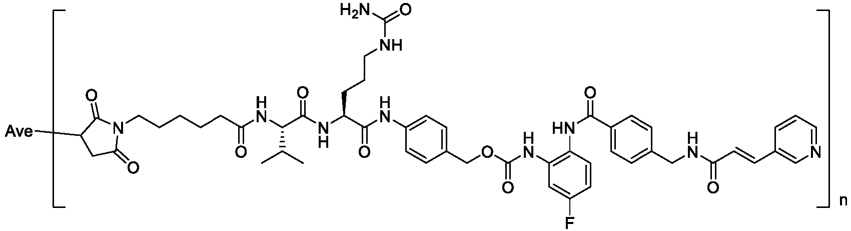

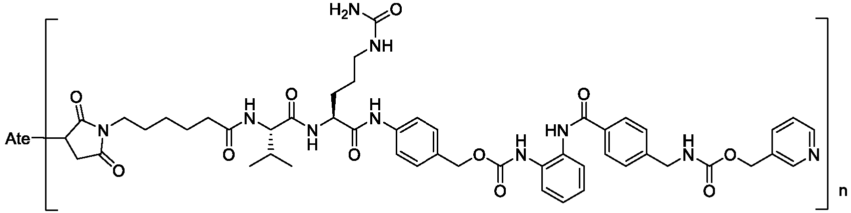

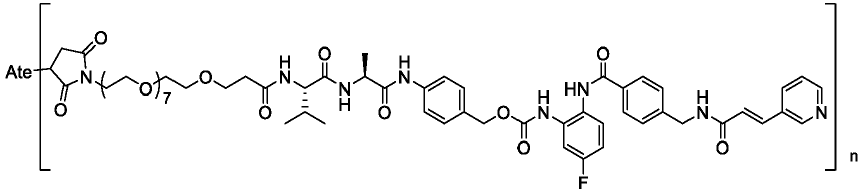

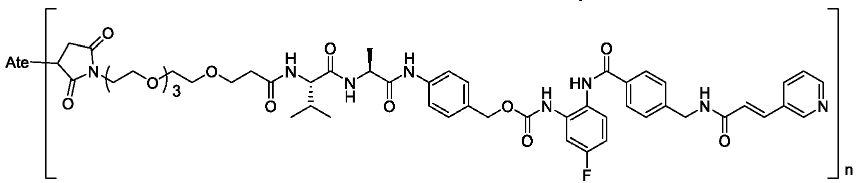

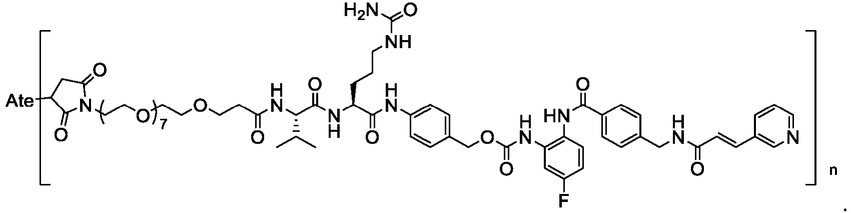

In a fourth aspect, the present disclosure relates to a pharmaceutical composition, which comprises the compound, the stereoisomer thereof, the tautomer thereof, the pharmaceutically acceptable salt thereof, the deuterated compound thereof, or the solvate thereof described above, or comprises the ligand-drug conjugate, the stereoisomer thereof, the tautomer thereof, the pharmaceutically acceptable salt thereof, the deuterated compound thereof, or the solvate thereof described above, and optionally further comprises a pharmaceutically acceptable carrier or excipient.

In a fifth aspect, the present disclosure relates to use of the compound, the stereoisomer thereof, the tautomer thereof, the pharmaceutically acceptable salt thereof, the deuterated compound thereof, or the solvate thereof described above, or the ligand-drug conjugate, the stereoisomer thereof, the tautomer thereof, the pharmaceutically acceptable salt thereof, the deuterated compound thereof, or the solvate thereof described above, or the pharmaceutical composition described above in the preparation of a medicament for treating or preventing a disease.

In some embodiments, the disease is a tumor.

In a sixth aspect, the present disclosure relates to the compound, the stereoisomer thereof, the tautomer thereof, the pharmaceutically acceptable salt thereof, the deuterated compound thereof, or the solvate thereof described above, or the ligand-drug conjugate, the stereoisomer thereof, the tautomer thereof, the pharmaceutically acceptable salt thereof, the deuterated compound thereof, or the solvate thereof described above, or the pharmaceutical composition described above, for use in the treatment or prevention of a disease.

In some embodiments, the disease is a tumor.

In a seventh aspect, the present disclosure relates to a method for treating or preventing a disease, which comprises administering to a subject an effective amount of the compound, the stereoisomer thereof, the tautomer thereof, the pharmaceutically acceptable salt thereof, the deuterated compound thereof, or the solvate thereof described above, or the ligand-drug conjugate, the stereoisomer thereof, the tautomer thereof, the pharmaceutically acceptable salt thereof, the deuterated compound thereof, or the solvate thereof described above, or the pharmaceutical composition described above.

In some embodiments, the disease is a tumor.

In an eighth aspect, the present disclosure relates to a method for preparing a compound represented by formula I, a stereoisomer thereof, a tautomer thereof, a pharmaceutically acceptable salt thereof, a deuterated compound thereof, or a solvate thereof, which comprises the following steps:
reacting a compound represented by formula I-1 with D' to give the compound represented by formula I; or reacting a compound represented by formula I-2 with H-L₂-L₃-D to give the compound represented by formula I; or reacting a compound represented by formula I-3 with H-L₂-L₃-D to give the compound represented by formula I: 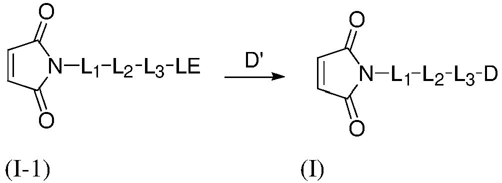 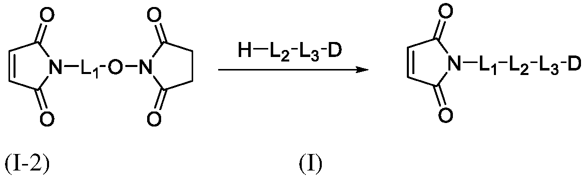 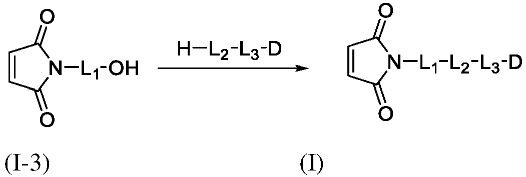 preferably, the method comprises the following steps:
reacting a compound represented by formula I-1 with D' to give the compound represented by formula I; or reacting a compound represented by formula I-2 with H-L₂-L₃-D to give the compound represented by formula I: 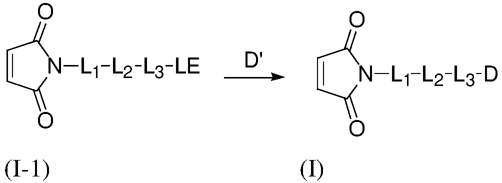 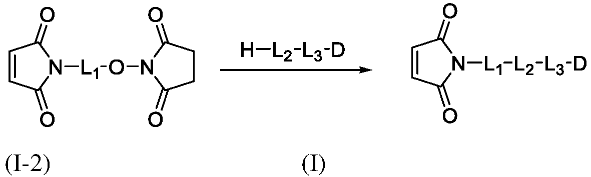
wherein LE is a leaving group; preferably, LE is halogen, 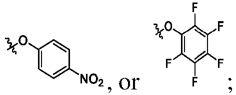
D' is a histone deacetylase inhibitor drug selected from a thiol-based histone deacetylase inhibitor, a hydroxamic acid-based histone deacetylase inhibitor, and a benzamide-based histone deacetylase inhibitor;
preferably, D' is a benzamide-based histone deacetylase inhibitor;
more preferably, D' is selected from 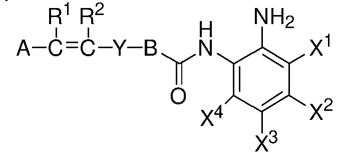 and 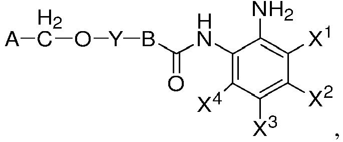 wherein
   A is phenyl or pyridyl, and the phenyl or pyridyl is each independently and optionally substituted with 1-4 substituents selected from: halogen, C₁-C₄ alkyl (e.g., methyl and ethyl), and C₁-C₄ haloalkyl (e.g., trifluoromethyl);
   preferably, A is phenyl or pyridyl, and the phenyl or pyridyl is each independently and optionally substituted with 1-2 substituents selected from: halogen, C₁-C₄ alkyl (e.g., methyl and ethyl), and trifluoromethyl;
   more preferably, A is pyridyl, and the pyridyl is optionally substituted with 1-2 substituents selected from: C₁-C₄ alkyl;
   further preferably, A is pyridyl;
   most preferably, A is 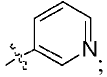
   B is phenylene;
   preferably, B is 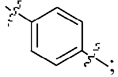
   Y is -CO-NH-CH₂-;
   preferably, the methylene end of Y is linked to B, and the carbonyl end of Y is linked to the alkenyl carbon or O;
   R¹ and R² are each independently selected from hydrogen and C₁-C₄ alkyl;
   preferably, R¹ and R² are both hydrogen;
   any one of X¹, X², X³ , and X⁴ is selected from hydrogen, halogen, and C₁-C₄ alkyl, and the remaining three are hydrogen;
   preferably, any one of X¹, X², X³, and X⁴ is selected from hydrogen and halogen, and the remaining three are hydrogen;
   more preferably, any one of X¹, X², X³, and X⁴ is selected from hydrogen and fluorine, and the remaining three are hydrogen;
   most preferably, X² is selected from hydrogen and fluorine, and X¹, X³, and X⁴ are hydrogen;
   more preferably, D' is selected from 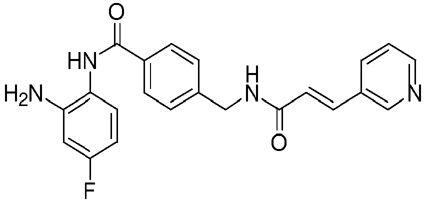 (i.e.,
   chidamide) and 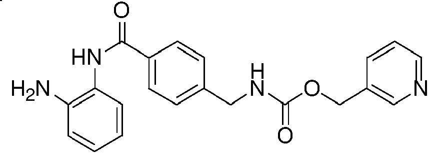 (i.e., entinostat);
   further preferably, D' is 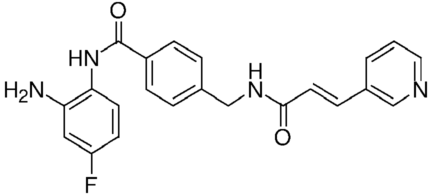 (i.e., chidamide);
   L₁, L₂, L₃, and D are as defined above.

In a ninth aspect, the present disclosure relates to a method for preparing a ligand-drug conjugate represented by formula II, a stereoisomer thereof, a tautomer thereof, a pharmaceutically acceptable salt thereof, a deuterated compound thereof, or a solvate thereof, which comprises the following steps:
reacting a compound represented by formula I, a stereoisomer thereof, a tautomer thereof, a pharmaceutically acceptable salt thereof, a deuterated compound thereof, or a solvate thereof with Ab to give the ligand-drug conjugate represented by formula II, the stereoisomer thereof, the tautomer thereof, the pharmaceutically acceptable salt thereof, the deuterated compound thereof, or the solvate thereof, wherein
preferably, the reaction is conducted at a pH of 5-10 and a temperature of 0-40 °C; 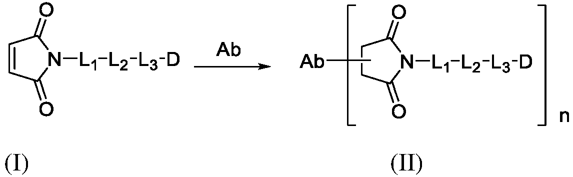 wherein
   Ab, n, L₁, L₂, L₃, and D are as defined above.

In some embodiments, the compound represented by formula I is selected from:

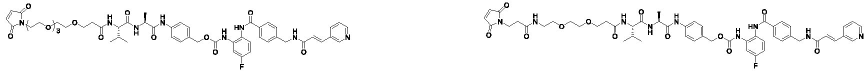

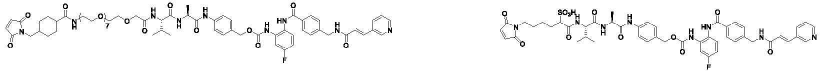

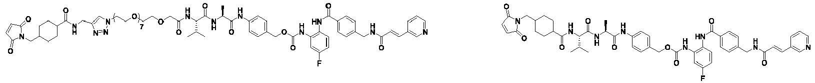

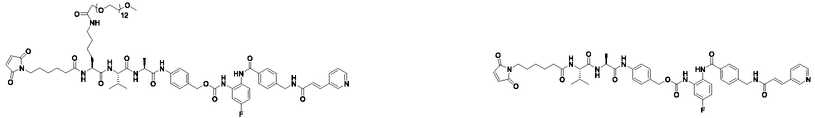

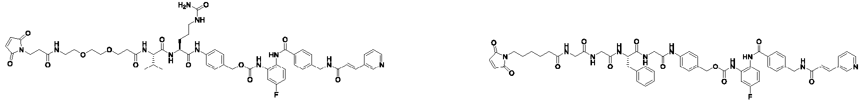

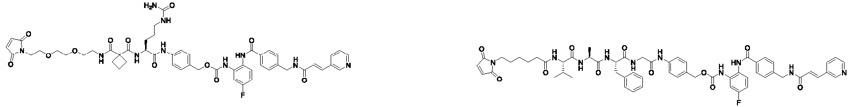

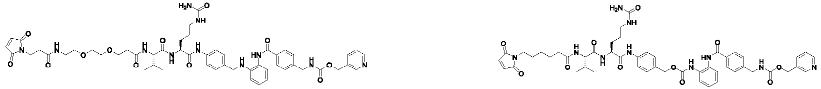

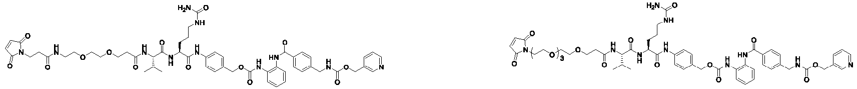

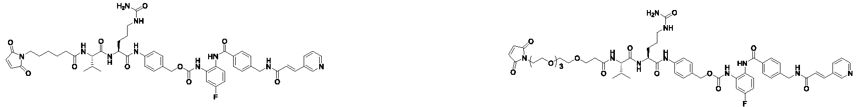

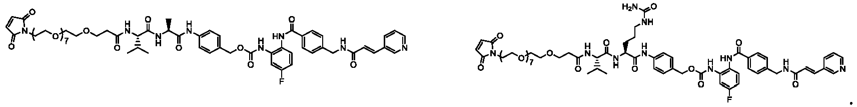

In some embodiments, the ligand-drug conjugate represented by formula II is selected from:

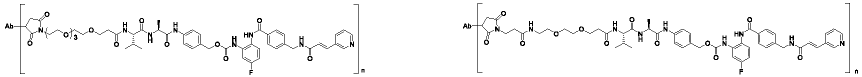

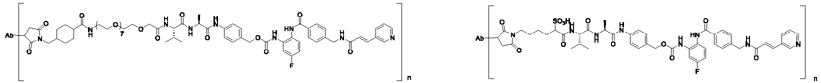

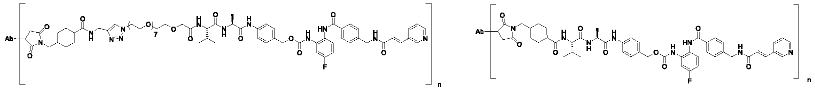

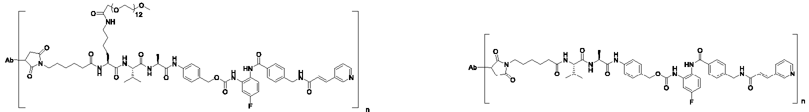

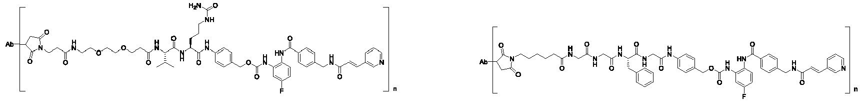

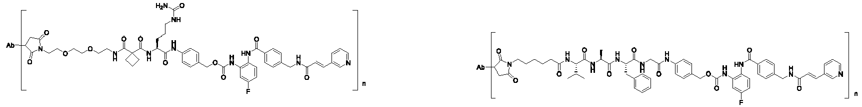

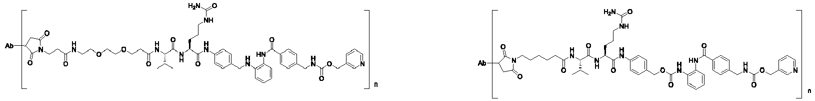

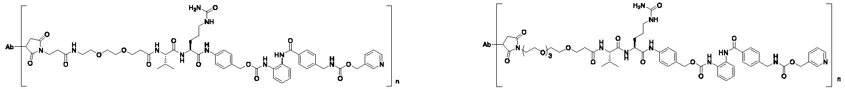

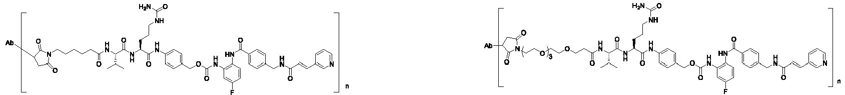

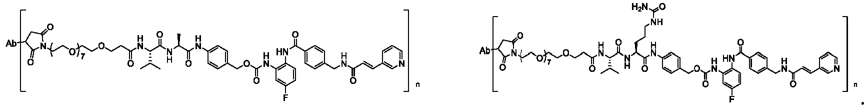

In some embodiments, the ligand-drug conjugate represented by formula II is selected from:

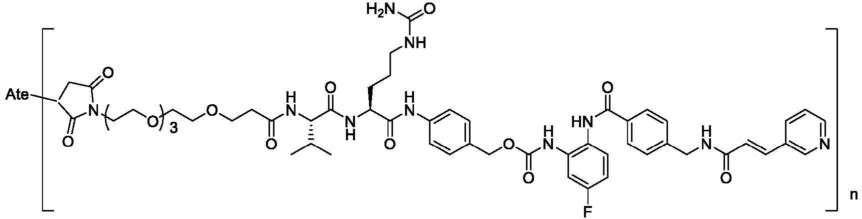

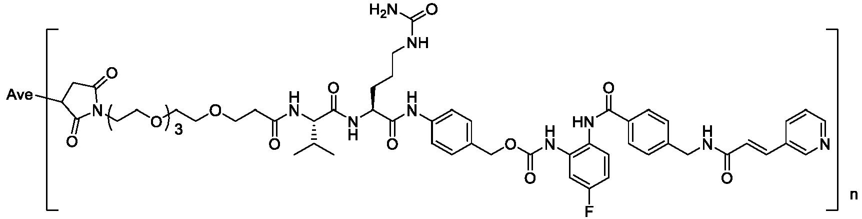

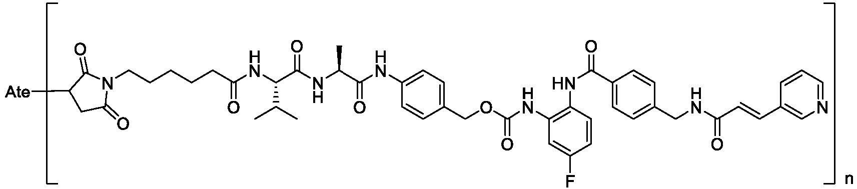

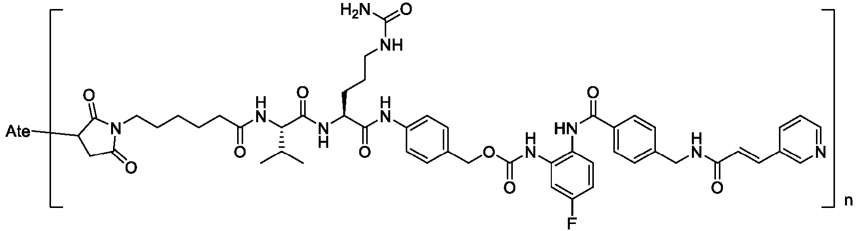

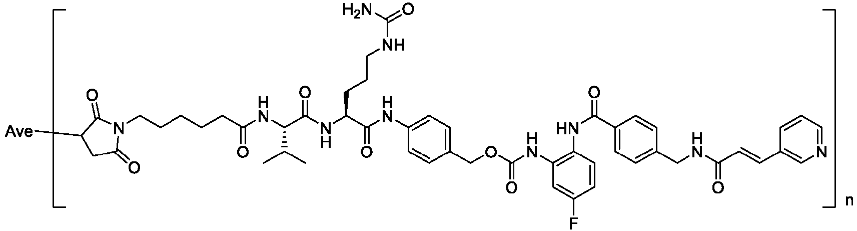

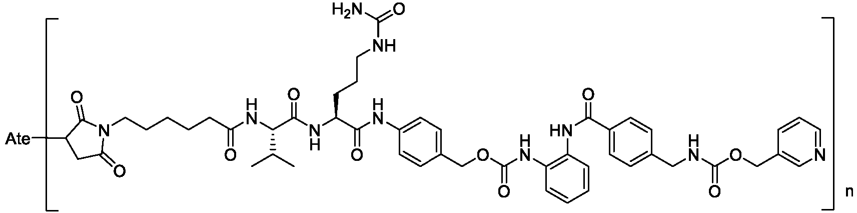

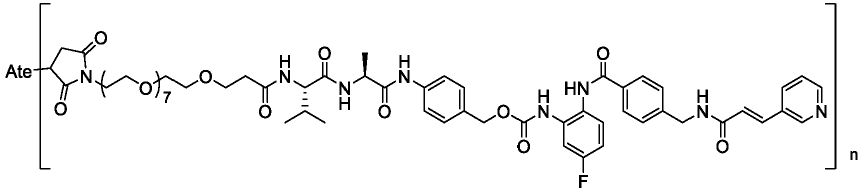

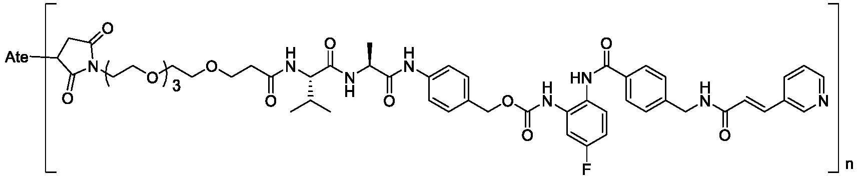

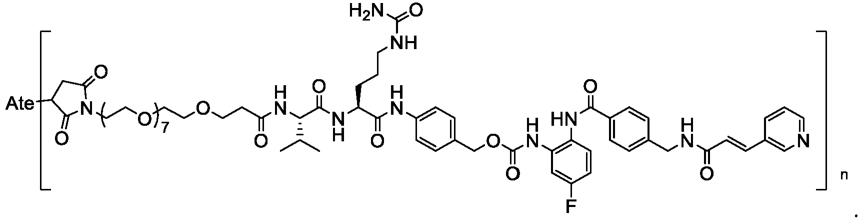

The beneficial effects of the present disclosure are as follows:
1. The present disclosure provides a novel stable ADC linker, as well as an antibody conjugate with excellent anti-tumor efficacy.
2. In an *in-vitro* tumor cell inhibition experiment, the conjugate containing the novel stable linker of the present disclosure exhibits a significant cell growth inhibitory effect compared to a naked antibody, a small-molecule drug, and combined use thereof.
3. In an *in-vivo* mouse efficacy experiment, the conjugate containing the novel stable linker of the present disclosure exhibits a significant tumor growth inhibitory effect compared to a naked antibody and combined use.
4. The conjugate containing the novel stable linker of the present disclosure has good plasma stability.
5. The conjugate containing the novel stable linker of the present disclosure shows excellent internalization.
6. The conjugate containing the novel stable linker of the present disclosure exhibits a significant promoting effect on cellular histone acetylation.

### BRIEF DESCRIPTION OF THE DRAWINGS

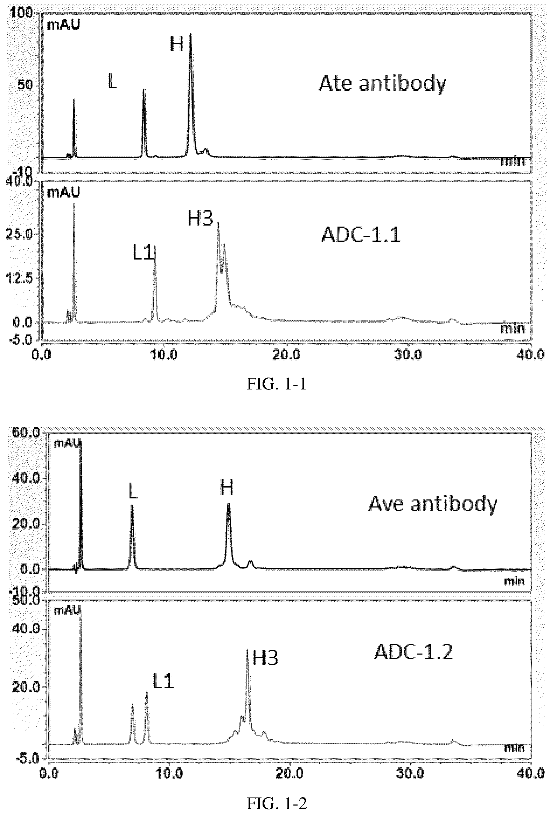

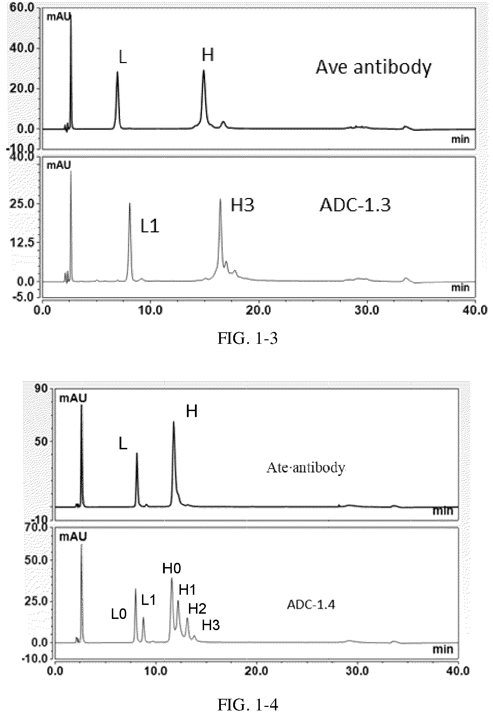

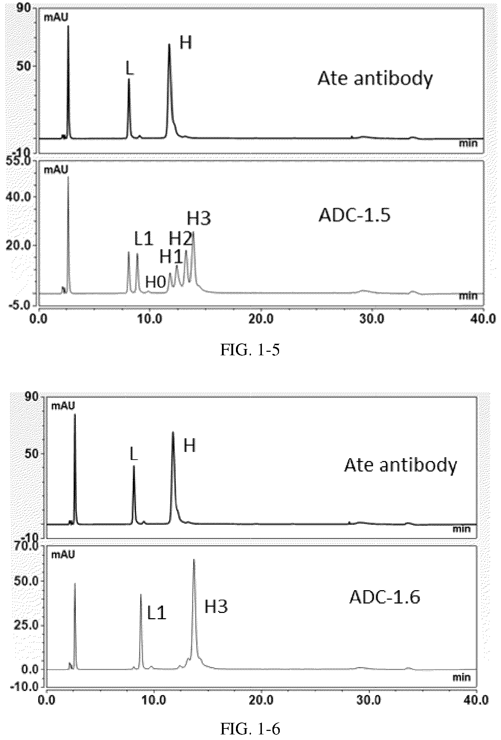

FIG. 1-1 shows the RP-HPLC chromatogram of ADC-1.1;
FIG. 1-2 shows the RP-HPLC chromatogram of ADC-1.2;
FIG. 1-3 shows the RP-HPLC chromatogram of ADC-1.3;
FIG. 1-4 shows the RP-HPLC chromatogram of ADC-1.4;
FIG. 1-5 shows the RP-HPLC chromatogram of ADC-1.5;
FIG. 1-6 shows the RP-HPLC chromatogram of ADC-1.6;
FIG. 2 shows the RP-HPLC chromatogram of ADC-2;
FIG. 3-1 shows the RP-HPLC chromatogram of ADC-3.1;
FIG. 3-2 shows the RP-HPLC chromatogram of ADC-3.2;
FIG. 3-3 shows the RP-HPLC chromatogram of ADC-3.3;

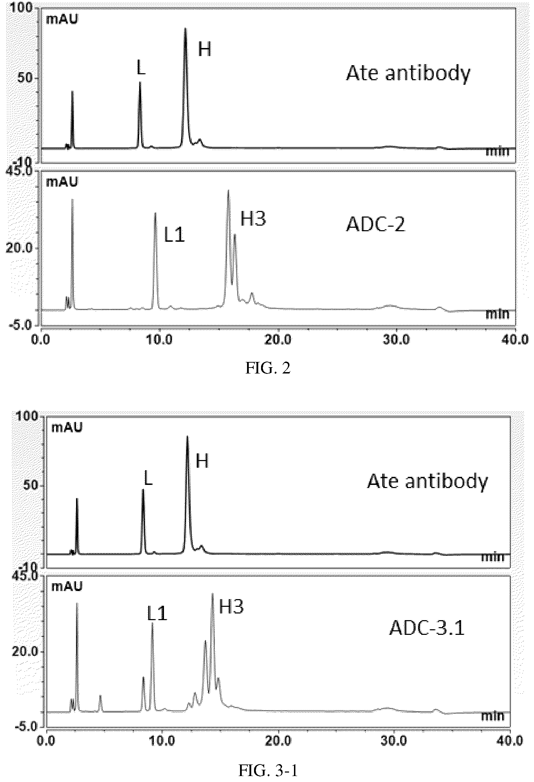

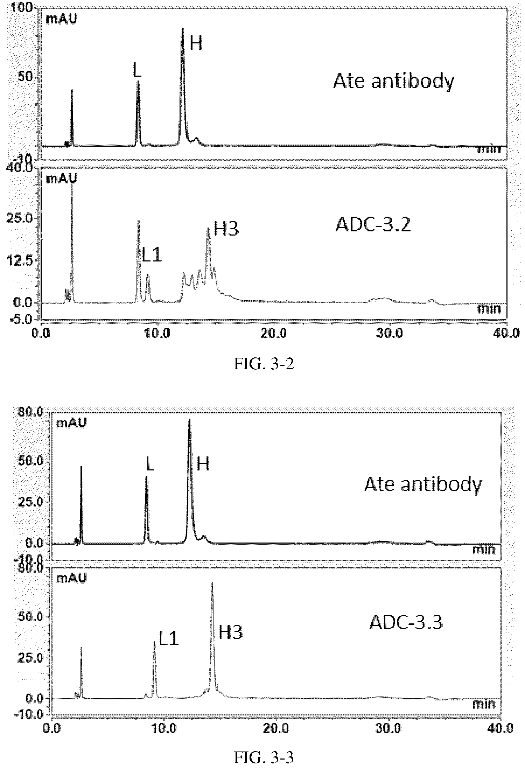

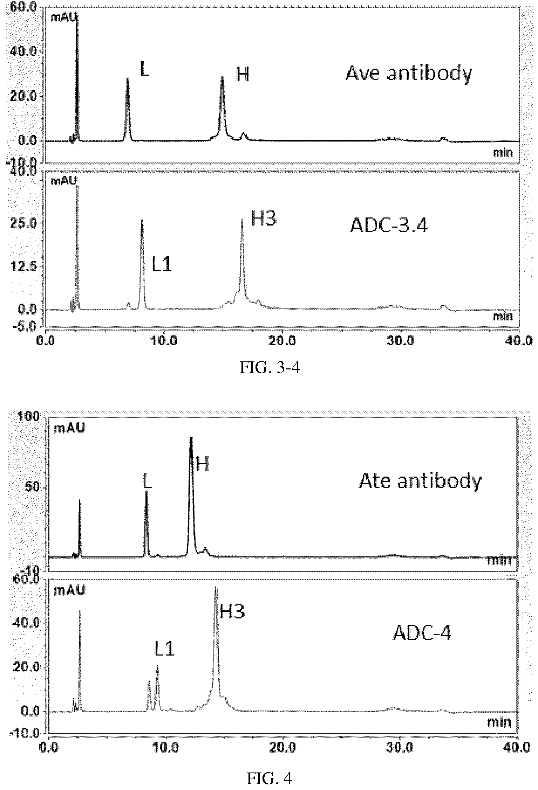

FIG. 3-4 shows the RP-HPLC chromatogram of ADC-3.4;
FIG. 4 shows the RP-HPLC chromatogram of ADC-4;

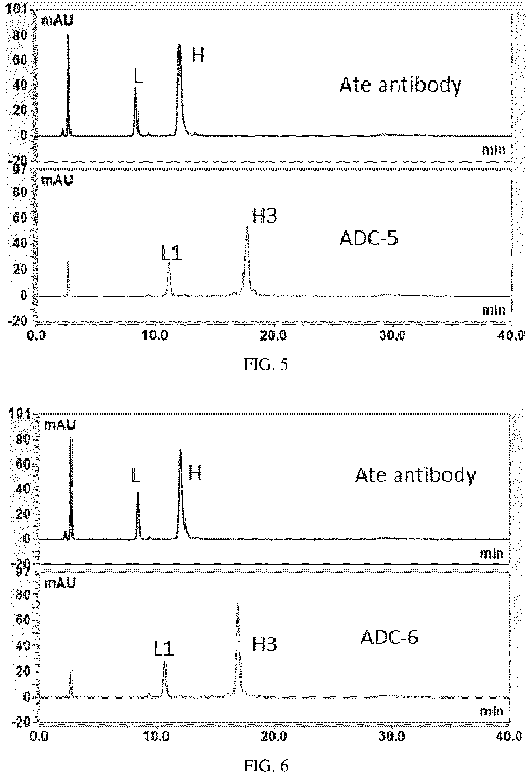

FIG. 5 shows the RP-HPLC chromatogram of ADC-5;
FIG. 6 shows the RP-HPLC chromatogram of ADC-6;

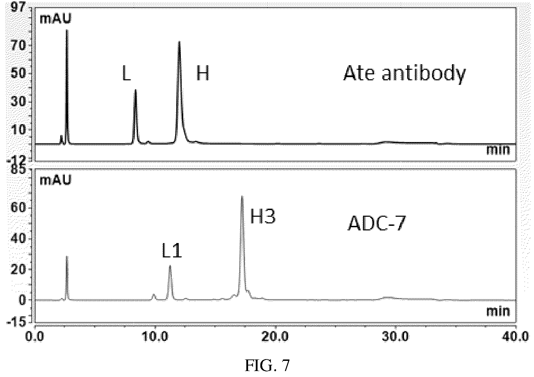

FIG. 7 shows the RP-HPLC chromatogram of ADC-7;

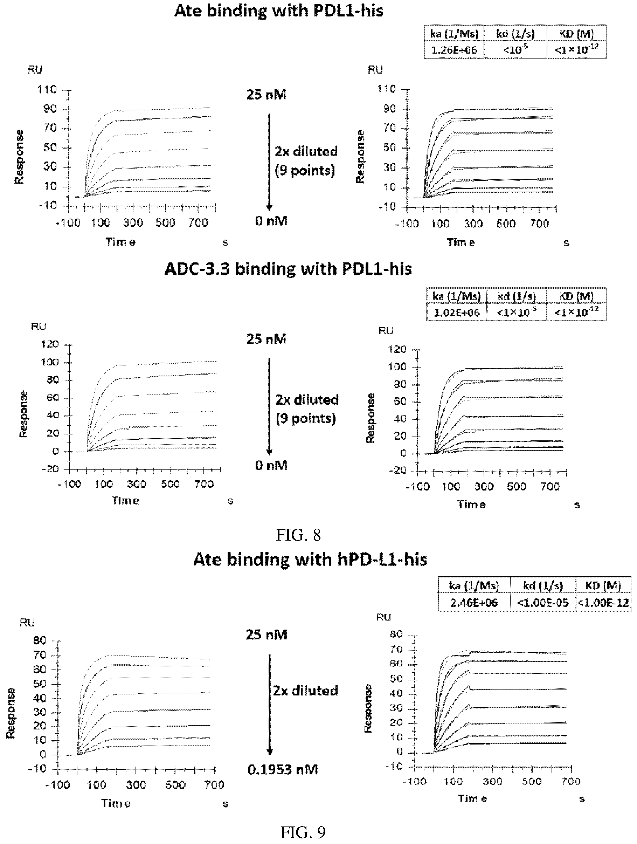

FIG. 8 shows the assay results of the affinity of the Ate antibody and ADC-3.3 for hPD-L1-his;
FIG. 9 shows the assay results of the affinity of the Ate antibody for hPD-L1-his;

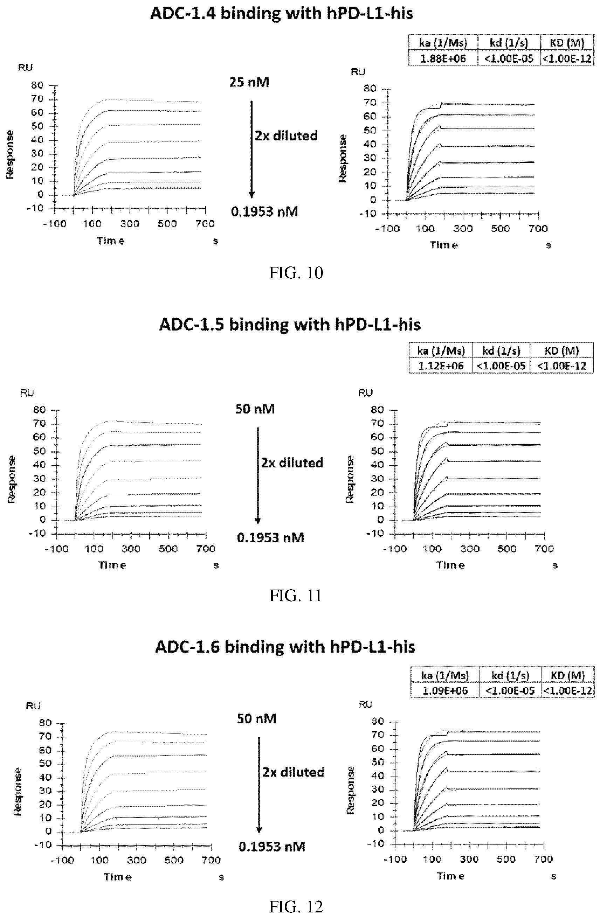

FIG. 10 shows the assay results of the affinity of ADC-1.4 for hPD-L1-his;
FIG. 11 shows the assay results of the affinity of ADC-1.5 for hPD-L1-his;
FIG. 12 shows the assay results of the affinity of ADC-1.6 for hPD-L1-his;

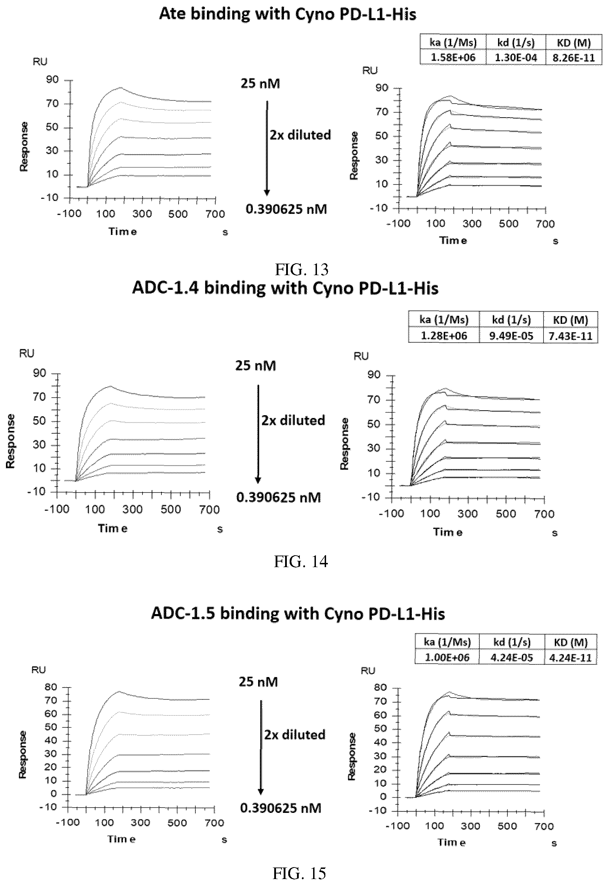

FIG. 13 shows the assay results of the affinity of the Ate antibody for Cyno PD-L1-his;
FIG. 14 shows the assay results of the affinity of ADC-1.4 for Cyno PD-L1-his;
FIG. 15 shows the assay results of the affinity of ADC-1.5 for Cyno PD-L1-his;

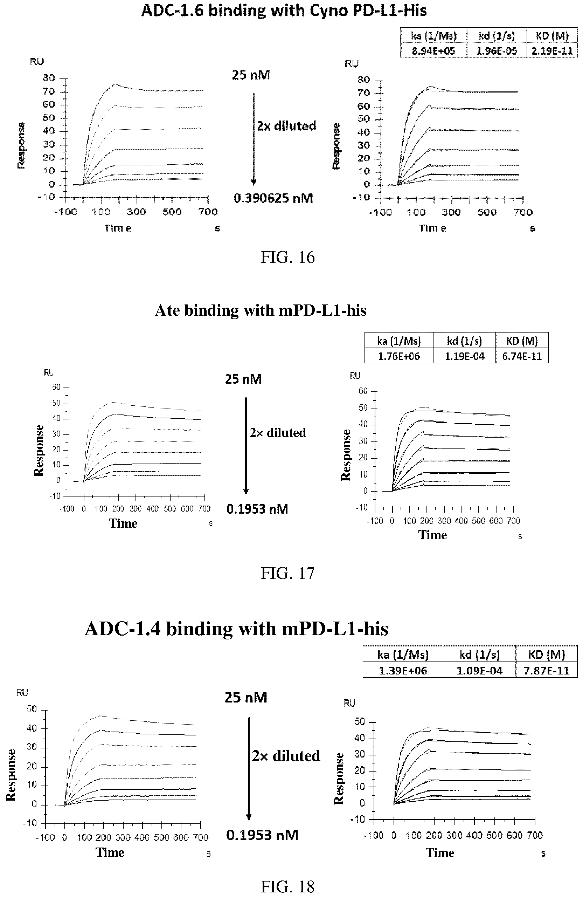

FIG. 16 shows the assay results of the affinity of ADC-1.6 for Cyno PD-L1-his;
FIG. 17 shows the assay results of the affinity of the Ate antibody for mPD-L1-his;
FIG. 18 shows the assay results of the affinity of ADC-1.4 for mPD-L1-his;

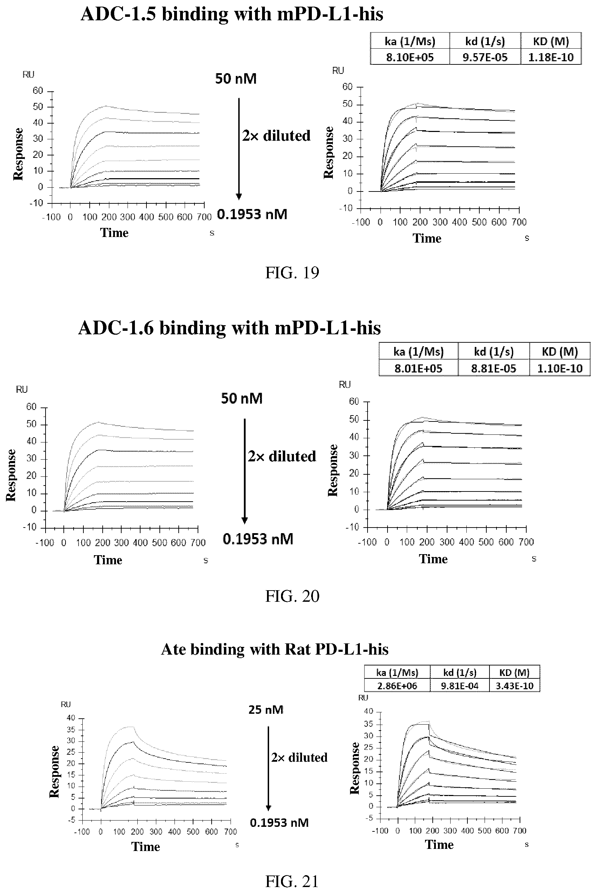

FIG. 19 shows the assay results of the affinity of ADC-1.5 for mPD-L1-his;
FIG. 20 shows the assay results of the affinity of ADC-1.6 for mPD-L1-his;
FIG. 21 shows the assay results of the affinity of the Ate antibody for Rat PD-L1-his;

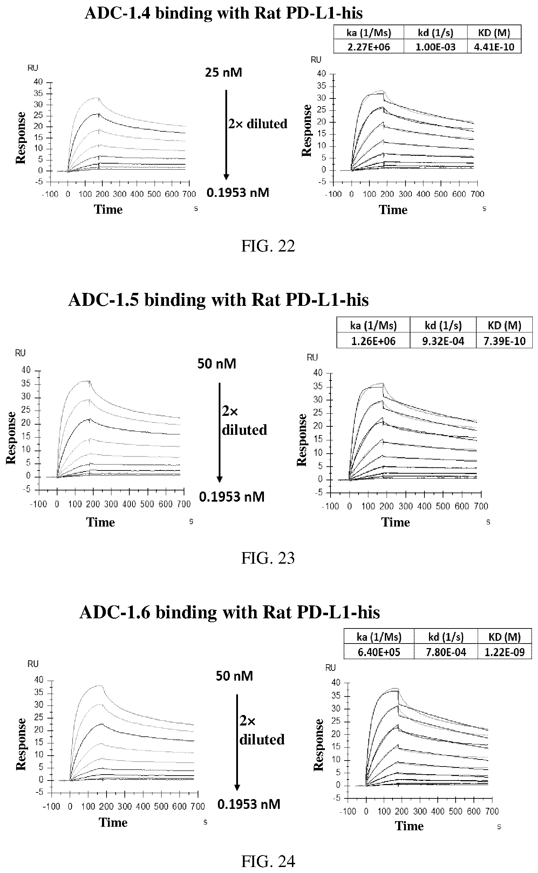

FIG. 22 shows the assay results of the affinity of ADC-1.4 for Rat PD-L1-his;
FIG. 23 shows the assay results of the affinity of ADC-1.5 for Rat PD-L1-his;
FIG. 24 shows the assay results of the affinity of ADC-1.6 for Rat PD-L1-his;

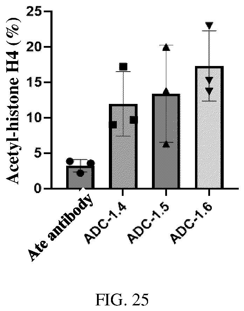

FIG. 25 shows the assay results of the effects of ADC-1.4, ADC-1.5, and ADC-1.6 on the acetylation level of histone H4 in NCI-H292 cells;

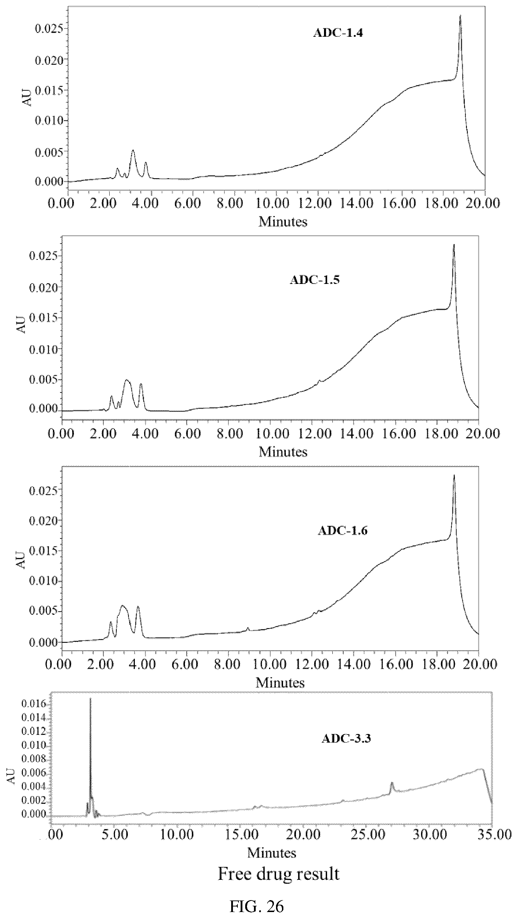

FIG. 26 shows the free toxin detection results for ADC-1.4, ADC-1.5, ADC-1.6, and ADC-3.3;

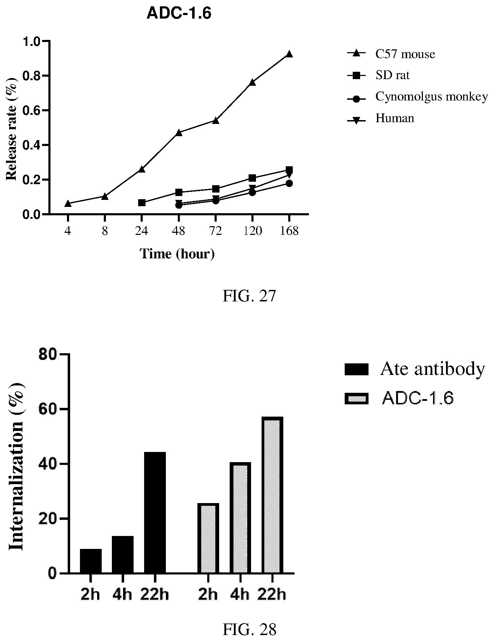

FIG. 27 shows the assay results of the stability of ADC-1.6 in human, cynomolgus monkey, SD rat, and C57 mouse plasma;
FIG. 28 shows the assay results of the internalization rate of ADC-1.6 in tumor cells;

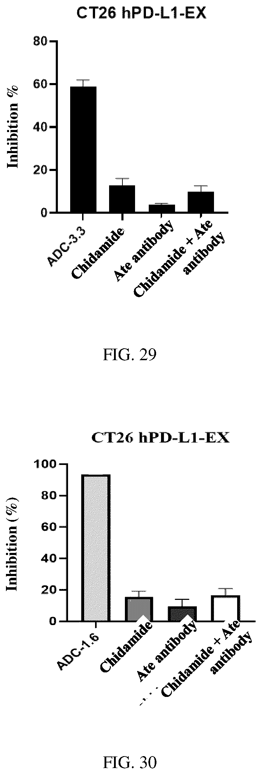

FIG. 29 shows the results of the cell proliferation inhibitory effect of ADC-3.3 on the colorectal cancer cell line CT26 hPD-L1-EX;
FIG. 30 shows the results of the cell proliferation inhibitory effect of ADC-1.6 on the colorectal cancer cell line CT26 hPD-L1-EX;

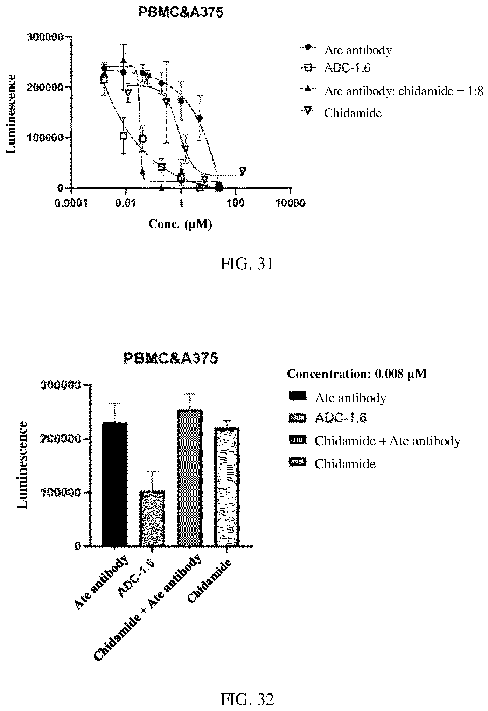

FIG. 31 shows the results of the cell proliferation inhibitory effect of ADC-1.6 on the human malignant melanoma cell line A375 in a co-culture system with human peripheral blood mononuclear cells (PBMCs) and the human malignant melanoma cell line A375;
FIG. 32 shows the results of the cell proliferation inhibitory effect of ADC-1.6 on the human malignant melanoma cell line A375 in a co-culture system with human peripheral blood mononuclear cells (PBMCs) and the human malignant melanoma cell line A375 (sample concentration: 0.008 µM);

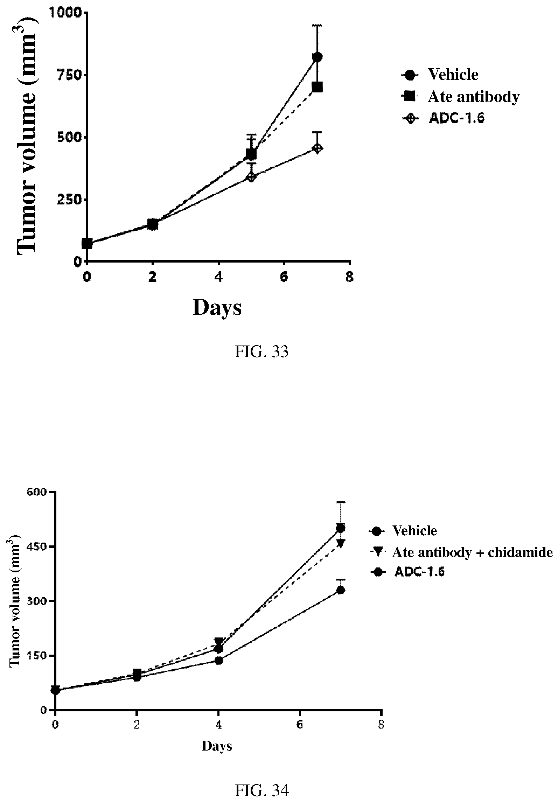

FIG. 33 shows the results of the tumor inhibition rates of ADC-1.6 and a naked antibody in a C57BL/6J mouse MC38 tumor model; and
FIG. 34 shows the results of the tumor inhibition rates of ADC-1.6 and combined use in the C57BL/6J mouse MC38 tumor model.

### DETAILED DESCRIPTION

The present disclosure will be further illustrated with reference to the following specific examples. It should be understood that these examples are merely intended to illustrate the present disclosure rather than limit the scope of the present disclosure.

Unless otherwise specified, the following terms and phrases as used herein are intended to have the following meanings. When a trademark name is used herein, the trademark name includes the product formula, generic drug, and active ingredient of the product with the trademark name, unless otherwise indicated in the context.

In the present disclosure, the "antibody" refers to an immunoglobulin, which is a four-peptide-chain structure formed by connecting two identical heavy chains and two identical light chains by interchain disulfide bonds. The heavy chain constant regions of immunoglobulins differ in their amino acid composition and arrangement, and thus in their antigenicity. Accordingly, immunoglobulins can be divided into five classes, otherwise called isotypes of immunoglobulins, namely IgM, IgD, IgG, IgA, and IgE, with their corresponding heavy chains being *µ* chain, *δ* chain, *γ* chain, *α* chain, and *ε* chain, respectively. The Ig of the same class can be divided into different subclasses according to differences in the amino acid composition of the hinge regions and the number and positions of disulfide bonds of the heavy chains; for example, IgG can be divided into IgG1, IgG2, IgG3, and IgG4. Light chains are divided into *κ* chain or *λ* chain according to differences in the constant regions. Each of the five classes of Ig may have a *κ* chain or a *λ* chain.

The term "drug-to-antibody ratio (DAR)" refers to the average number of cytotoxic drug molecules (i.e., D in formula I or formula II) loaded onto each Ab (e.g., antibody) in formula II (e.g., n in formula II), which may be an integer or a decimal. The DAR (e.g., n in general formula II) may range from 0.5 to 8.5 (i.e., may be any integer or decimal selected from 0.5-8.5 (inclusive)) cytotoxic drug molecules (i.e., D in formula I or formula II) loaded per Ab (e.g., antibody) on average; for example, the DAR (e.g., n in formula II) may be 0.5, 1, 1.5, 1.94, 2, 2.5, 3, 3.37, 3.5, 4, 4.08, 4.5, 4.71, 4.74, 4.99, 5, 5.26, 5.43, 5.5, 6, 6.34, 6.46, 6.5, 7, 7.08, 7.5, 7.66, 7.77, 7.80, 7.81, 8, 8.5, or the like.

The term "pharmaceutically acceptable salt" refers to a salt of the compound or conjugate of the present disclosure, which is safe and effective for use in the body of a mammal and possesses the requisite biological activity. For example, an acidic group in the compound or conjugate of the present disclosure forms a salt with a base; non-limiting examples of the salt include: sodium salt, potassium salt, calcium salt, magnesium salt, or the like. For another example, a basic group in the compound or conjugate of the present disclosure forms a salt with an acid; non-limiting examples of the salt include: hydrochloride, hydrobromide, hydroiodide, sulfate, bisulfate, citrate, acetate, succinate, ascorbate, oxalate, nitrate, phosphate, hydrogen phosphate, dihydrogen phosphate, salicylate, hydrogen citrate, tartrate, maleate, fumarate, formate, benzoate, methanesulfonate, ethanesulfonate, benzenesulfonate, and p-toluenesulfonate.

The term "solvate" refers to a pharmaceutically acceptable solvate formed by the compound or conjugate of the present disclosure with one or more solvent molecules; non-limiting examples of the solvent molecules include water, ethanol, acetonitrile, isopropanol, DMSO, and ethyl acetate.

The term "stereoisomer" refers to compound molecules with the same molecular formula and identical connectivity of atoms or substituents, but differing in the spatial arrangement of these atoms or substituents. This phenomenon falls under the category of isomerism in organic chemistry.

The term "tautomer" refers to two isomers containing heteroatoms (e.g., nitrogen, oxygen, or sulfur atoms), whose structural differences lie solely in the migration of a proton and the corresponding double bond, and which coexist in an equilibrium system and interconvert at a relatively high rate. A typical example is the keto-enol tautomerism.

The term "deuterated compound" refers to a structure resulting from replacement of one or more hydrogen atoms in the compound or conjugate of the present disclosure with a deuterium atom.

The term "amino acid residue" refers to an incomplete amino acid structure formed when the amino group of an amino acid has lost one hydrogen atom and the carboxyl group has lost one hydroxyl group; the resulting residue has an amino end and a carbonyl end. In the present disclosure, L₂ is an amino acid residue or a peptide residue consisting of 2-10 (preferably 2-4) amino acid residues, where the 2-10 (preferably 2-4) amino acids may be identical or different in type. For example, if L₂ is a peptide residue consisting of 4 amino acid residues and the amino acids are selected from glycine and phenylalanine, then L₂ may be the following peptide residue: glycine residue-glycine residue-phenylalanine residue-glycine residue (Gly-Gly-Phe-Gly), specifically

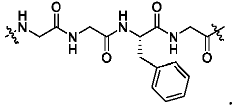

In the present disclosure, unless otherwise explicitly indicated, the descriptive phrase "... be each independently selected from" used throughout the present disclosure may mean that specific items expressed by the same or different symbols in different groups do not affect each other, or that specific items expressed by the same or different symbols in the same group do not affect each other.

The substituents of the compound of the present disclosure are disclosed according to group types or ranges. It is specifically noted that the present disclosure encompasses each independent sub-combination of the individual members of these group types and ranges. For example, the term "C₁-C₆ alkyl" specifically refers to independently disclosed methyl, ethyl, C₃ alkyl, C₄ alkyl, C₅ alkyl, and C₆ alkyl.

In the present disclosure, the "substituted" or "substituted with ..." means that any one or more hydrogens on a specified atom or group are replaced by a choice from the designated groups, provided that the normal valence state of the specified atom is not exceeded.

In the present disclosure, "optionally" means that a selection may or may not be made. For example, "A is pyridyl, and the pyridyl is optionally substituted with 1-2 substituents selected from: C₁-C₄ alkyl" means that A is pyridyl, and the pyridyl may either be unsubstituted or substituted with 1-2 substituents selected from: C₁-C₄ alkyl.

The term "halogen" refers to fluorine, chlorine, bromine, or iodine.

The term "C₁-C₄ alkyl" refers to an alkyl group having 1-4 carbon atoms, preferably "C₁-C₃ alkyl". Examples of the alkyl include, but are not limited to, methyl, ethyl, propyl (e.g., n-propyl and isopropyl), butyl (e.g., n-butyl, isobutyl, and tert-butyl), and the like.

The term "C₁-C₄ haloalkyl" refers to a group obtained by replacing one or more hydrogen atoms in any of the aforementioned C₁-C₄ alkyl with halogen (preferably fluorine); examples of the group include monofluoromethyl, difluoromethyl, difluoroethyl, trifluoromethyl, and the like.

The term "absent" means that the groups on both sides are directly connected. For example, in formula I 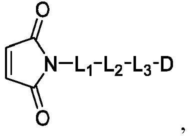 if L₂ is absent, the structural formula of formula I becomes 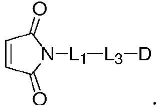 Accordingly, those skilled in the art can understand that if L₂ is present, L₂ has one end connected to L₁ and the other end connected to L₃, but if L₂ is absent, L₁ is directly connected to L₃. Other similar definitions may be understood with reference to the aforementioned content.

The term "carrier" refers to a system that can alter the manner in which the drug gets into a human body and the distribution of the drug in the human body, control the release rate of the drug, and deliver the drug to a targeted organ. The drug carrier release and targeting system can reduce drug degradation and loss, lower side effects, and improve bioavailability. For example, polymeric surfactants that can be used as carriers, due to their unique amphiphilic structures, can self-assemble to form various forms of aggregates, with preferred examples such as micelles, microemulsions, gels, liquid crystals, vesicles, etc. These aggregates possess the capability to encapsulate drug molecules while also exhibiting good permeability for membranes, and therefore can be used as excellent drug carriers.

The term "excipient" refers to an additive in a pharmaceutical formulation other than the active ingredient, also known as an auxiliary material; the auxiliary material includes, but is not limited to: ion exchangers; aluminum oxide; aluminum stearate; lecithin; serum proteins such as human serum albumin; buffering substances such as phosphates; glycerin; sorbic acid; potassium sorbate; partial glyceride mixtures of saturated vegetable fatty acids; water; salts or electrolytes such as protamine sulfate, disodium hydrogen phosphate, potassium hydrogen phosphate, sodium chloride and zinc salts; colloidal silica; magnesium trisilicate; polyvinylpyrrolidone; cellulose-based substances; polyethylene glycol; sodium carboxymethylcellulose, polyacrylates; beeswax; lanolin; and the like.

The term "treat", "treating", or "treatment" usually refers to acquiring desired pharmacological and/or physiological effects. The effects may be prophylactic in terms of completely or partially preventing a disease or a symptom thereof, and/or may be therapeutic in terms of partially or completely stabilizing or curing the disease and/or a side effect arising from the disease. The "treat", "treating", or "treatment" used herein encompasses any treatment to a disease in a patient, including (a) preventing a disease or a symptom in a patient susceptible to the disease or the symptom that has not yet been diagnosed; (b) inhibiting a symptom of a disease, i.e., arresting its progression; or (c) relieving a symptom of a disease, i.e., causing regression of the disease or the symptom. The term "subject" includes a human or a non-human animal. Exemplary human subjects include humans with a disease (e.g., a disease described herein), which are referred to as patients, or normal individuals. In the present disclosure, the term "non-human animal" includes all vertebrates, such as non-mammals (e.g., birds, amphibians, and reptiles) and mammals, such as non-human primates, livestock and/or domesticated animals (e.g., sheep, dogs, cats, cows, pigs, etc.).

The term "effective amount" refers to an amount that is effective to achieve the desired therapeutic or prophylactic effect at the required dose and for the required duration. The "therapeutically effective amount" of the compound or conjugate of the present disclosure may vary depending on factors such as the disease state, age, sex, and body weight of the individual, as well as the ability of the compound or conjugate to elicit a desired response in the individual. The therapeutically effective amount also encompasses an amount of the compound or conjugate where its therapeutically beneficial effects outweigh any toxic or harmful consequences. The "prophylactically effective amount" refers to an amount that is effective to achieve the desired prophylactic effect at the required dose and for the required duration. Typically, but not necessarily, the prophylactically effective amount is lower than the therapeutically effective amount, since the prophylactic dose is administered to the subject prior to the onset of the disease or at an earlier stage of the disease. In the case of cancer, a therapeutically effective amount of a drug may: reduce the number of cancer cells; shrink the tumor volume; inhibit (i.e., slow to some extent, preferably halt) infiltration of cancer cells into surrounding organs; inhibit (i.e., slow to some extent, preferably halt) tumor metastasis; inhibit tumor growth to some extent; and/or alleviate one or more symptoms associated with the cancer to some extent.

The present disclosure will be further described with reference to the following specific examples. It should be understood that these examples are merely intended to illustrate the present disclosure rather than limit the scope of the present disclosure. Experimental procedures without specific conditions in the following examples are generally performed under conventional conditions or under conditions recommended by the manufacturers.

### Example 1: Preparation of Linker-Toxin (Compound 1)

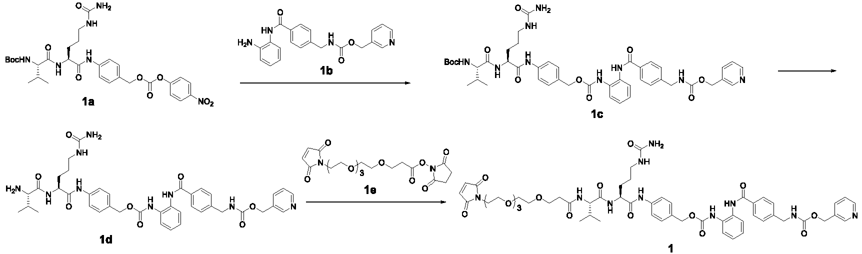

### 1) Preparation of compound 1c:

1-Hydroxybenzotriazole (506 mg, 3.7 mmol) was added to a solution of a compound represented by formula **1a** (805 mg, 1.25 mmol) in N,N-dimethylformamide (12 mL). The mixture was allowed to react at room temperature for 2 h, and then entinostat (1b, 470 mg, 1.25 mmol) was added. The resulting mixture was allowed to react at room temperature for another 72 h. After the reaction was completed, the reaction liquid was concentrated, and the residue was purified by reversed-phase column chromatography (acetonitrile/0.05% aqueous formic acid solution = 0:100% to 50%:50%) to give a compound represented by formula **1c** (200 mg). ESI-MS (m/z): 882.3 [M+H]⁺.

### 2) Preparation of compound 1d:

Under an ice bath, trifluoroacetic acid (2 mL) was slowly added dropwise to a solution of the compound represented by formula **1c** (200 mg, 0.23 mmol) in dichloromethane (10 mL). The mixture was allowed to react for 2 h. After the reaction was completed, the reaction liquid was concentrated, and the residue was purified by reversed-phase column chromatography (acetonitrile/0.05% aqueous formic acid solution = 0:100% to 40%:60%) to give a compound represented by formula **1d** (80 mg). ESI-MS (m/z): 782.4 [M+H]⁺.

### 3) Preparation of compound 1:

1-{15-[(2,5-Dioxotrihydro-*H*-pyrrol-1-yl)oxy]-15-oxy-3,6,9,12-tetraoxy-1-yl}pyrrole-2, 5-dione (**1e**, 26.5 mg, 0.06 mmol) was added to a solution of the compound represented by formula **1d** (80 mg, 0.23 mmol) in N,N-dimethylformamide (10 mL). The mixture was stirred at room temperature for 2 h. The reaction liquid was concentrated, and the residue was purified by reversed-phase column chromatography (acetonitrile/0.05% aqueous formic acid solution = 0:100% to 50%:50%) to give compound 1 represented by formula **1** (27.7 mg). ESI-MS (m/z): 1109.5 [M+H]⁺.

### Example 2: Preparation of Linker-Toxin (Compound 2)

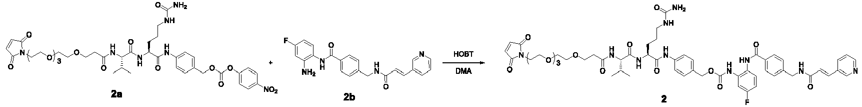

### 4-((17S,20S)-1-(2,5-Dioxo-2,5-dihydro-1H-pyrrol-1-yl)-17-isopropyl-15,18-dioxo-20-(3-ureidopropyl)-3,6,9,12-tetraoxa-16,19-diazacyclo-21-amino)benzyl

(4-nitrophenyl)carbonate **(2a,** 252 mg, 0.29 mmol) and DMA (2 mL) were sequentially added to a 50-mL round-bottom flask. The mixture was purged three times with nitrogen, and HOBT (116 mg, 0.86 mmol) was added thereto. The reaction mixture was stirred at room temperature for 1 h. Chidamide (**2b**, 112 mg, 0.29 mmol) was added to the reaction mixture, and the resulting reaction mixture was stirred at room temperature for 96 h. The resulting reaction liquid was directly purified by reversed-phase silica gel column chromatography (eluent: acetonitrile/0.05% aqueous formic acid solution = 0:100% to 35%:65%) to give compound 2 represented by formula **2** (15 mg). ESI-MS (m/z): 1123.7 [M+H]⁺. ¹H NMR (400 MHz, DMSO-*d*₆) δ 9.99 (s, 1H), 9.76 (s, 1H), 9.17 (s, 1H), 8.78 - 8.75 (m, 2H), 8.56 (dd, *J* = 4.8, 1.6 Hz, 1H), 8.10 (d, *J* = 7.5 Hz, 1H), 8.00 (dt, *J* = 8.0, 1.9 Hz, 1H), 7.93 (d, *J* = 8.2 Hz, 2H), 7.85 (d, *J* = 8.6 Hz, 1H), 7.62 - 7.39 (m, 8H), 7.33 (d, *J* = 8.6 Hz, 2H), 7.01 - 6.96 (m, 2H), 6.82 (d, *J* = 15.9 Hz, 1H), 5.98 (t, *J* = 5.6 Hz, 1H), 5.40 (s, 2H), 5.09 (s, 2H), 4.49 (d, *J* = 5.9 Hz, 2H), 4.38 (q, *J* = 8.1 Hz, 1H), 4.23 (dd, *J* = 8.5, 6.8 Hz, 1H), 3.65 - 3.41 (m, 18H), 3.04 - 2.91 (m, 2H), 2.51 - 2.32 (m, 2H), 1.97 - 1.94 (m, 1H), 1.70 - 1.57 (m, 2H), 1.44 - 1.33 (m, 2H), 0.86 - 0.82 (m, 6H).

### Example 3: Preparation of Linker-Toxin (Compound 3)

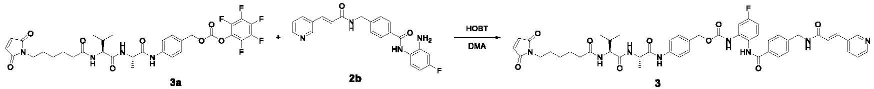

4-((*S*)-2-((*S*)-2-(6-(2,5-Dioxy-2,5-dihydro-1*H*-pyrrol-1-yl)hexanamido)-3-methylbutanam ido)propanamido)benzyl (pentafluorophenyl)carbonate **(3a,** 202 mg, 0.29 mmol) and DMA (2 mL) were sequentially added to a 50-mL round-bottom flask. The mixture was purged three times with nitrogen, and HOBT (116 mg, 0.86 mmol) was added thereto. The reaction mixture was stirred at room temperature for 1 h. Chidamide **(2b,** 112 mg, 0.29 mmol) was added to the reaction mixture, and the resulting reaction mixture was stirred at room temperature for 96 h. The resulting reaction liquid was directly purified by reversed-phase silica gel column chromatography (eluent: acetonitrile/0.05% aqueous formic acid solution = 0:100% to 35%:65%) to give compound 3 represented by formula **3** (22 mg). ESI-MS (m/z): 903.4 [M+H]⁺.

### Example 4: Synthesis of Linker-Toxin (Compound 4)

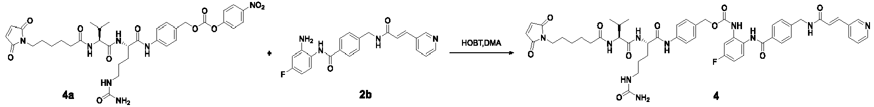

4-((*S*)-2-((*S*)-2-(6-(2,5-Dioxo-2,5-dihydro-1*H*-pyrrol-1-yl)hexanamide)-3-methylbutanam ido)-5-ureidopentanamido)benzyl (4-nitrophenyl)carbonate **(4a,** 200 mg, 0.27 mmol) and anhydrous *N,N*-dimethylacetamide (1 mL) were sequentially added to a 50-mL round-bottom flask. The mixture was purged three times with nitrogen, and HOBT (109 mg, 0.54 mmol) was added thereto. The reaction mixture was stirred at room temperature for 2 h, and chidamide **(2b,** 105 mg, 0.27 mmol) was added thereto. The resulting reaction mixture was stirred at room temperature for 72 h. The mixture was then concentrated by evaporation under reduced pressure to remove the solvent, and the resulting residue was purified by reversed-phase silica gel column chromatography (acetonitrile/0.05% aqueous formic acid solution = 0:100% to 35%:65%) to give compound 4 represented by formula **4** (18 mg). ESI-MS (m/z): 989.5 [M+H]⁺.

### Example 5: Preparation of Linker-Toxin (Compound 5)

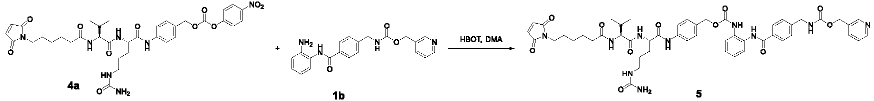

This example was conducted by following the procedures in Example 4, except that the chidamide **(2b)** in Example 4 was replaced by entinostat (**1b**), thus giving compound 5 represented by formula **5** (65 mg). ESI-MS (m/z): 975.5 [M+H]⁺.

### Example 6: Preparation of Linker-Toxin (Compound 6)

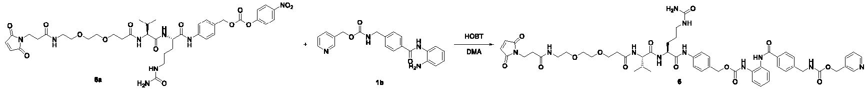

This example was conducted by following the procedures in Example 4, except that the 4-((*S*)-2-((*S*)-2-(6-(2,5-dioxo-2,5-dihydro-1*H*-pyrrol-1-yl)hexanamide)-3-methylbutanami do)-5-ureidopentanamido)benzyl (4-nitrophenyl)carbonate **(4a)** in Example 4 was replaced by 4-((2*S*,5*S*)-19-(2,5-dioxo-2,5-dihydro-1*H*-pyrrol-1-yl)-5-isopropyl-4,7,17-trioxo-2-(3-urei dopropyl)-10,13-dioxo-3,6,16-triazanamide)benzyl (4-nitrophenyl)carbonate **(6a),** thus giving compound 6 represented by formula 6 (55 mg). ESI-MS (m/z): 1092.5 [M+H]⁺.

### Example 7: Preparation of Linker-Toxin (Compound 7)

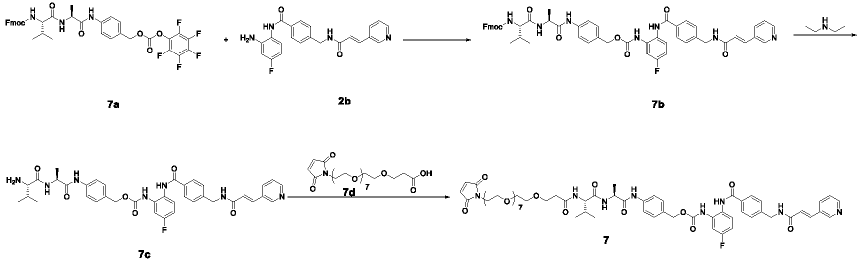

### Preparation of compound 7b:

Chidamide **(2b,** 107.6 mg, 0.28 mmol) and 1-hydroxybenzotriazole (18.6 mg, 0.14 mmol) were added to a solution of compound **7a** (200 mg, 0.28 mmol) in *N,N*-dimethylacetamide (0.5 mL). After completion of the addition, the mixture was allowed to react at 25 °C for 48 h. After the reaction was completed, the reaction liquid was purified by reversed-phase column chromatography (acetonitrile:0.05% aqueous ammonium bicarbonate solution = 0:100% to 40%:60%) to give **7b** (94 mg). ESI-MS (m/z): 933.4 [M+H]⁺.

### Preparation of compound 7c:

Diethylamine (0.2 mL, 2.76 mmol) was added to a solution of compound **7b** (94 mg) in N,N-dimethylformamide (0.8 mL). The reaction liquid was allowed to react at 25 °C overnight. After the reaction was completed, the reaction liquid was purified by reversed-phase column chromatography (acetonitrile:0.05% aqueous formic acid solution = 0:100% to 40%:60%) to give **7c** (51 mg). ESI-MS (m/z): 710.28 [M+H]⁺.

### Preparation of compound 7:

Compound **7d** (18 mg, 0.04 mmol), *O*-(7-azabenzotriazol-1-yl)-*N,N,N',N*'-tetramethyluronium hexafluorophosphate (13 mg, 0.04 mmol), and *N,N*-diisopropylethylamine (5 mg, 0.04 mmol) were added to a solution of compound **7c** (29 mg, 0.04 mmol) in *N,N*-dimethylformamide (0.5 mL). After completion of the addition, the mixture was allowed to react at 25 °C overnight. After the reaction was completed, the reaction liquid was purified by reversed-phase preparative liquid chromatography (acetonitrile:0.05% aqueous formic acid solution = 0:100% to 40%:60%) to give compound 7 represented by formula 7 (15 mg). ESI-MS (m/z): 1213.8 [M+H]⁺.

### Example 8: Preparation of Linker-Toxin (Compound 8)

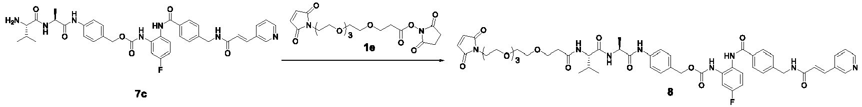

Compound 7c (29 mg, 0.04 mmol), compound **1e** (18 mg, 0.04 mmol), and N,N-dimethylacetamide (0.5 mL) were sequentially added to a 5-mL eggplant-shaped flask equipped with a stir bar. *N,N*-Diisopropylethylamine (6 mg, 0.05 mmol) was then added to the mixture. After completion of the addition, the resulting mixture was stirred at room temperature for 3 h. The resulting reaction liquid was purified by reversed-phase column chromatography (eluent: acetonitrile:0.05% aqueous formic acid solution = 0:100% to 45%:65%) to give compound **8** represented by formula 8 (20 mg, 0.02 mmol, 54.3%). ESI-MS *(m*/*z):* 1037.5 [M+H]⁺. ¹H NMR (400 MHz, DMSO) *δ* 9.96 (s, 1H), 9.79 (s, 1H), 9.21 (s, 1H), 8.82 - 8.76 (m, 2H), 8.58 (dd, *J* = 4.8, 1.6 Hz, 1H), 8.49 (s, 1H), 8.20 (d, *J =* 7.2 Hz, 1H), 8.04 - 7.84 (m, 4H), 7.61 - 7.44 (m, 8H), 7.35 (d, *J =* 8.8 Hz, 2H), 7.03 - 6.98 (m, 2H), 6.84 (d, *J =* 16 Hz, 1H), 5.11 (s, 2H), 4.51 (d, *J =* 6 Hz, 2H), 4.44 - 4.36 (m, 1H), 4.22 (dd, *J* = 8.8, 6.8 Hz, 1H), 3.65 - 3.44 (m, 18H), 2.49 - 2.33 (m, 2H), 2.02 - 1.94 (m, 1H), 1.32 (d, *J =* 7.2 Hz, 3H), 0.87 (dd, *J* = 15.2, 6.8 Hz, 6H).

### Example 9: Preparation of Linker-Toxin (Compound 9)

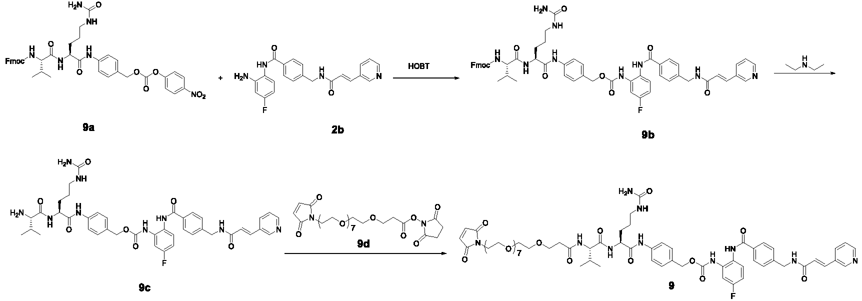

### Preparation of compound 9b:

Chidamide **(2b,** 2545.6 mg, 6.52 mmol) and HOBt (528.6 mg, 3.91 mmol) were added to a solution of compound **9a** (5000 mg, 6.52 mmol) in *N,N*-dimethylacetamide (50 mL). After completion of the addition, the mixture was allowed to react at 25 °C for 72 h. After the reaction was completed, the reaction liquid was purified by reversed-phase column chromatography (acetonitrile:0.05% aqueous ammonium bicarbonate solution = 0:100% to 40%:60%) to give compound **9b** (750 mg). ESI-MS (m/z): 509.7 [M/2+H]⁺.

### Preparation of compound 9c:

Diethylamine (0.5 mL) was added to a solution of compound **9b** (550 mg, 0.54 mmol) in *N,N*-dimethylformamide (5 mL). The reaction mixture was allowed to react at 25 °C overnight. After the reaction was completed, the reaction liquid was purified by reversed-phase preparative liquid chromatography to give compound **9c.** ESI-MS (m/z): 398.91 [M/2+H]⁺.

### Preparation of compound 9:

Compound **9d** (25 mg, 0.04 mmol) and *N*,*N*-diisopropylethylamine (0.02 mL, 0.11 mmol) were added to a solution of compound **9c** (31 mg, 0.04 mmol) in *N,N*-dimethylacetamide (2 mL). After completion of the addition, the mixture was allowed to react at 25 °C for 18 h. After the reaction was completed, the reaction liquid was purified by reversed-phase column chromatography (acetonitrile:0.05% aqueous formic acid solution = 0:100% to 50%:50%) to give compound **9** represented by formula 9 (3 mg). ESI-MS *(m*/*z):* 650.33 [M/2+H]⁺.

### Example 10: Preparation of Antibody-Drug Conjugates

### 10.1. Preparation of anti-PD-L1 antibody atezolizumab (Ate antibody):

The antibody gene for atezolizumab (DrugBank Accession Number: DB11595) was subjected to CHO codon optimization and synthesis by Sangon Biotech (Shanghai) Co., Ltd., and then constructed into a plasmid, thus giving the expression plasmid pXC-atezolizumab.

CHO cells were transfected with the plasmid using electroporation. After cell screening, culture for expression, and collection of cell fermentation broth, affinity chromatography was performed to give an atezolizumab antibody (abbreviated as Ate antibody or Ate in the present application).

**Table 1-1. Atezolizumab antibody sequences:**

| | | |
|---|---|---|
| Heavy chain amino acids | SEQ ID NO:1 | EVQLVESGGGLVQPGGSLRLSCAASGFTFSDSWIHWVRQA PGKGLEWVAWISPYGGSTYYADSVKGRFTISADTSKNTAYL QMNSLRAEDTAVYYCARRHWPGGFDYWGQGTLVTVSSAS TKGPSVFPLAPSSKSTSGGTAALGCLVKDYFPEPVTVSWNS GALTSGVHTFPAVLQSSGLYSLSSVVTVPSSSLGTQTYICNV NHKPSNTKVDKKVEPKSCDKTHTCPPCPAPELLGGPSVFLF PPKPKDTLMISRTPEVTCVVVDVSHEDPEVKFNWYVDGVE VHNAKTKPREEQYASTYRVVSVLTVLHQDWLNGKEYKCK VSNKALPAPIEKTISKAKGQPREPQVYTLPPSREEMTKNQV SLTCLVKGFYPSDIAVEWESNGQPENNYKTTPPVLDSDGSF FLYSKLTVDKSRWQQGNVFSCSVMHEALHNHYTQKSLSLS PGK |
| Light chain amino acids | SEQ ID NO:2 | DIQMTQSPSSLSASVGDRVTITCRASQDVSTAVAWYQQKPG KAPKLLIYSASFLYSGVPSRFSGSGSGTDFTLTISSLQPEDFA TYYCQQYLYHPATFGQGTKVEIKRTVAAPSVFIFPPSDEQLK SGTASVVCLLNNFYPREAKVQWKVDNALQSGNSQESVTE QDSKDSTYSLSSTLTLSKADYEKHKVYACEVTHQGLSSPVT KSFNRGEC |

### 10.2. Preparation of anti-PD-L1 antibody avelumab (Ave):

The antibody gene for avelumab (DrugBank Accession Number: DB11945) was subjected to CHO codon optimization and synthesis by Sangon Biotech (Shanghai) Co., Ltd., and then constructed into a plasmid, thus giving the expression plasmid pXC-avelumab.

CHO cells were transfected with the plasmid using electroporation. After cell screening, culture for expression, and collection of cell fermentation broth, affinity chromatography was performed to give an avelumab antibody (abbreviated as Ave antibody or Ave in the present application).

**Table 1-2. Avelumab antibody sequences:**

| | | |
|---|---|---|
| Heavy chain amino acids | SEQ ID NO:3 | EVQLLESGGGLVQPGGSLRLSCAASGFTFSSYIMMWVRQA PGKGLEWVSSIYPSGGITFYADTVKGRFTISRDNSKNTLYLQ MNSLRAEDTAVYYCARIKLGTVTTVDYWGQGTLVTVSSAS TKGPSVFPLAPSSKSTSGGTAALGCLVKDYFPEPVTVSWNS GALTSGVHTFPAVLQSSGLYSLSSVVTVPSSSLGTQTYICNV NHKPSNTKVDKKVEPKSCDKTHTCPPCPAPELLGGPSVFLF PPKPKDTLMISRTPEVTCVVVDVSHEDPEVKFNWYVDGVE VHNAKTKPREEQYNSTYRVVSVLTVLHQDWLNGKEYKCK VSNKALPAPIEKTISKAKGQPREPQVYTLPPSRDELTKNQVS LTCLVKGFYPSDIAVEWESNGQPENNYKTTPPVLDSDGSFFL YSKLTVDKSRWQQGNVFSCSVMHEALHNHYTQKSLSLSPG K |
| Light chain amino acids | SEQ ID NO:4 | QSALTQPASVSGSPGQSITISCTGTSSDVGGYNYVSWYQQH PGKAPKLMIYDVSNRPSGVSNRFSGSKSGNTASLTISGLQA EDEADYYCSSYTSSSTRVFGTGTKVTVLGQPKANPTVTLFP PSSEELQANKATLVCLISDFYPGAVTVAWKADGSPVKAGVE TTKPSKQSNNKYAASSYLSLTPEQWKSHRSYSCQVTHEGST VEKTVAPTECS |

### 10.3. Antibody-drug conjugation reaction

### Preparation of ADC-1.1:

### Preparation of salt buffered solution:

Buffer-1: 4.65 g of L-histidine was dissolved in 1 L of ultrapure water, and the solution was adjusted to about pH 5.50 using 0.1 mol/L glacial acetic acid, sterilized by filtration through a 0.22-µm membrane filter, then transferred into a bottle, and stored at 4 °C for a short period of time until use.

Buffer-2: 8.96 g of Na₂HPO₄·12H₂O and 3.90 g of NaH₂PO₄·2H₂O were separately dissolved in 500 mL of ultrapure water, adjusted to pH 8.0 (±0.05) by mutual titration, sterilized by filtration through a 0.22-µm membrane filter, then transferred into a bottle, and stored at 4 °C for a short period of time until use.

Antibody buffer exchange: The Ate antibody stock solution was thawed slowly at 4 °C and exchanged into Buffer-1 via the ultrafiltration method to achieve a final concentration of >10 mg/mL, and its concentration was measured using a UV spectrophotometer.

Antibody reduction: The antibody (1 eq) was accurately taken using a pipetting gun, and supplemented with a certain amount of Buffer-1 to ensure that the final concentration of the antibody was about 5 mg/mL. Then, 8 eq of 10 mmol/L TCEP solution was added, and the mixture was thoroughly mixed at a constant temperature of 25 °C and allowed to react for 120 min.

Antibody conjugation: The required volume of an organic solvent (DMA or DMSO) was accurately taken to make it account for 10% of the total volume. The small-molecule payload of formula 2 (10 eq) was added to the organic solvent, and the mixture was thoroughly mixed and then added slowly to the reduced antibody reaction liquid. The resulting mixture was stirred slowly at 25 °C for conjugation reaction for 60 min.

Product primary purification: After the reaction was terminated, the product was exchanged into Buffer-1 via the ultrafiltration method, and stored at 4 °C for short-term storage or at -80 °C for long-term storage until use.

DAR value determination: The DAR value was determined using the RP-HPLC method. The detection conditions were as follows: chromatographic column: PLRP-S 8 µm 4.6 × 150 mm; column temperature: 80 °C; flow rate: 0.8 mL/min; sample injection amount: 20 µg; detection wavelength: 214 & 280 nm; gradient elution: 0 min 30% B, 5 min 35% B, 25 min 45% B, 26-30 min 90% B, and 31-40 min 30% B, where B was ACN containing 0.1% TFA. Sample treatment: The sample was diluted to 3 mg/mL with the corresponding buffer, and DTT with a final concentration of 20 mM was added. The mixture was thoroughly mixed and then directly injected for analysis.

Average DAR value calculation: (1) The peak area percentages of L0 and L1 (the sum of the peak area percentages of L0 + L1 equals 100%) were calculated, and the peak area percentages of H0, H1, H2, and H3 (the sum of the peak area percentages of H0 + H1 + H2 + H3 equals 100%) were calculated. (2) The weighted percentage of each peak was calculated as: peak area percentage × number of conjugated drug molecules; for example, H3 weighted ratio = H3 peak area percentage × 3. (3) Average DAR value = sum of weighted percentages of all peaks × 2 / 100.

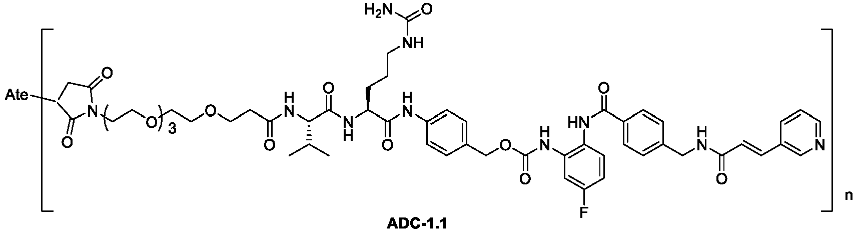

n was measured to be 7.08. See FIG. 1-1 for details.

### Preparation of ADC-1.2:

The preparation of ADC-1.2 was conducted by following the preparation procedures and DAR value determination method for ADC-1.1, except that the Ate antibody was replaced by the Ave antibody, 4 eq of 10 mmol/L TCEP solution was added during antibody reduction, and 6 eq of small molecule was added during antibody conjugation.

All other conditions remained unchanged. 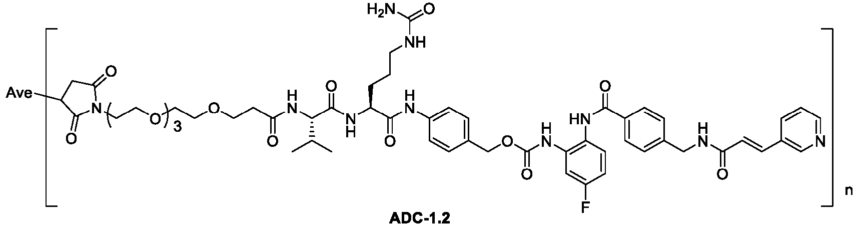 n was measured to be 3.37. See FIG. 1-2 for details.

### Preparation of ADC-1.3:

The preparation of ADC-1.3 was conducted by following the preparation procedures and DAR value determination method for ADC-1.1, except that the Ate antibody was replaced by the Ave antibody.

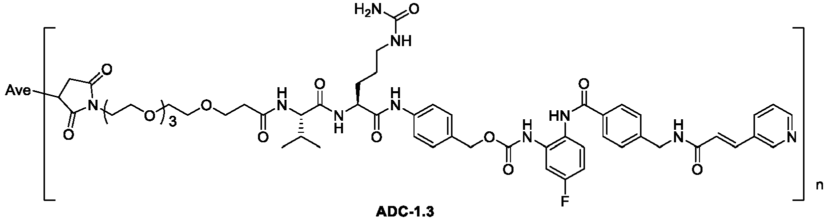

n was measured to be 4.74. See FIG. 1-3 for details.

### Preparation of ADC-1.4:

The preparation of ADC-1.4 was conducted by following the preparation procedures and DAR value determination method for ADC-1.1, except that 1.2 eq of TCEP was added during antibody reduction and 4 eq of small molecule payload was added during conjugation. 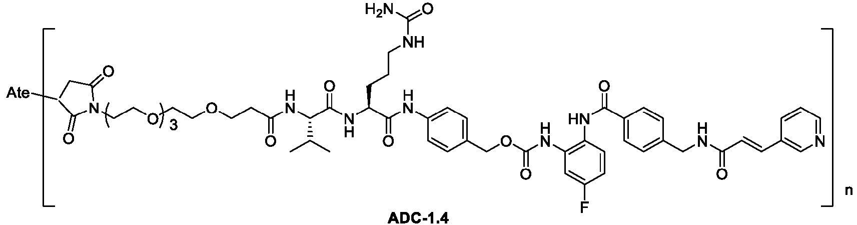 n was measured to be 1.94. See FIG. 1-4 for details.

### Preparation of ADC-1.5:

The preparation of ADC-1.5 was conducted by following the preparation procedures and DAR value determination method for ADC-1.1, except that 4 eq of TCEP was added during antibody reduction and 6 eq of small molecule payload was added during conjugation. 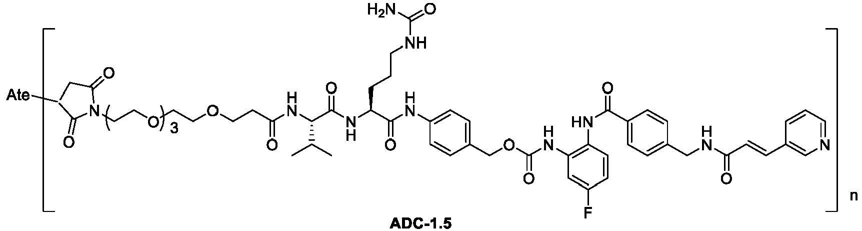 n was measured to be 4.71. See FIG. 1-5 for details.

### Preparation of ADC-1.6:

The preparation of ADC-1.6 was conducted by following the preparation procedures and DAR value determination method for ADC-1.1, except that 10 eq of TCEP was added during antibody reduction and 14 eq of small molecule payload was added during conjugation. 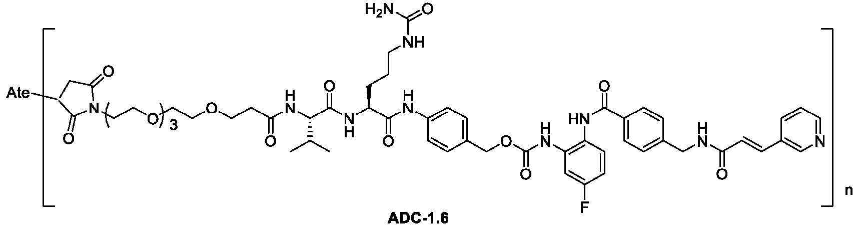 n was measured to be 7.66. See FIG. 1-6 for details.

### Preparation of ADC-2:

The preparation of ADC-2 was conducted by following the preparation procedures and DAR value determination method for ADC-1.1, except that formula 2 was replaced by the structure represented by formula 3. 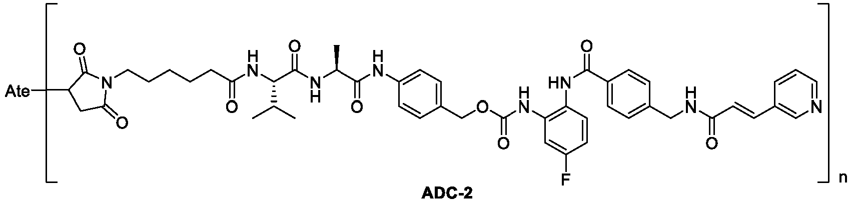 n was measured to be 6.34. See FIG. 2 for details.

### Preparation of ADC-3.1:

The preparation of ADC-3.1 was conducted by following the preparation procedures and DAR value determination method for ADC-1.1, except that formula 2 was replaced by the structure represented by formula 4. 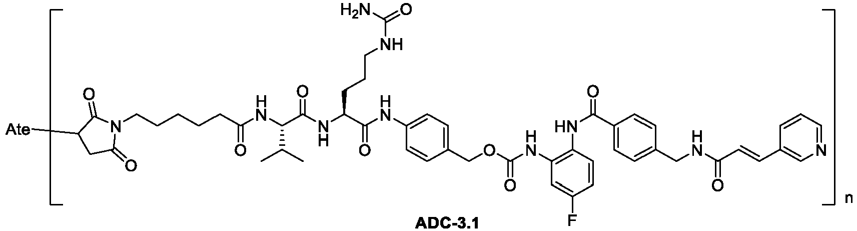 n was measured to be 6.46. See FIG. 3-1 for details.

### Preparation of ADC-3.2:

The preparation of ADC-3.2 was conducted by following the preparation procedures and DAR value determination for ADC-3.1, except that 4 eq of 10 mmol/L TCEP solution was added during antibody reduction and 6 eq of small molecule was added during antibody conjugation. 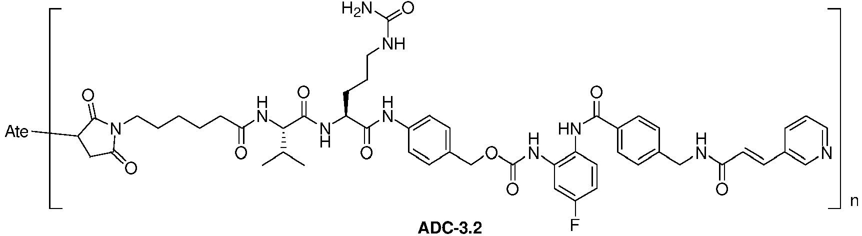 n was measured to be 4.99. See FIG. 3-2 for details.

### Preparation of ADC-3.3:

The preparation of ADC-3.3 was conducted by following the preparation procedures and DAR value determination method for ADC-3.1, except that 12 eq of 10 mmol/L TCEP solution was added during antibody reduction and 14 eq of small molecule was added during antibody conjugation. All other conditions remained unchanged. 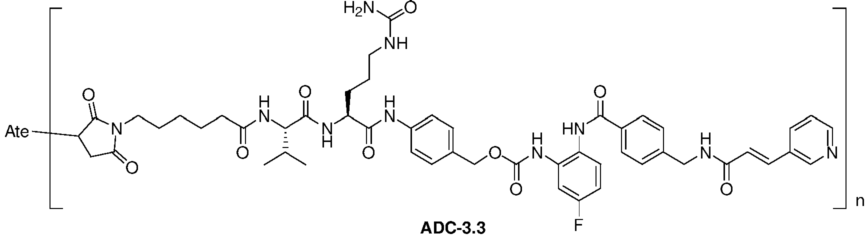 n was measured to be 7.80. See FIG. 3-3 for details.

### Preparation of ADC-3.4:

The preparation of ADC-3.4 was conducted by following the preparation procedures and DAR value determination method for ADC-3.1, except that the Ate antibody was replaced by the Ave antibody, 4 eq of 10 mmol/L TCEP solution was added during antibody reduction, and 6 eq of small molecule was added during antibody conjugation. All other conditions remained unchanged. 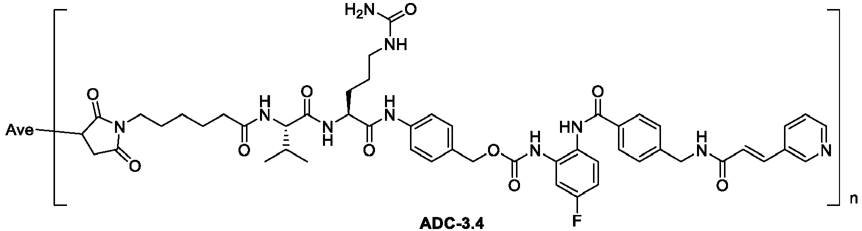 n was measured to be 4.08. See FIG. 3-4 for details.

### Preparation of ADC-4:

The preparation of ADC-4 was conducted by following the preparation procedures and DAR value determination method for ADC-1.1, except that formula 2 was replaced by the structure represented by formula 5. All other conditions remained unchanged. 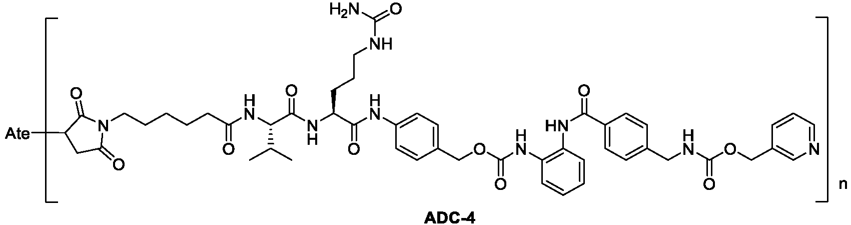 n was measured to be 5.43. See FIG. 4 for details.

### Preparation of ADC-5:

The preparation of ADC-5 was conducted by following the preparation procedures and DAR value determination method for ADC-1.1, except that the small molecule of formula 2 was replaced by a small molecule of formula 7, 12 eq of TCEP was added during antibody reduction, and 10 eq of small molecule payload was added during conjugation. 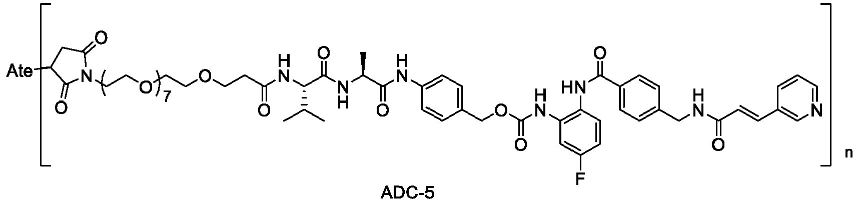 n was measured to be 7.77. See FIG. 5 for details.

### Preparation of ADC-6:

The preparation of ADC-6 was conducted by following the preparation procedures and DAR value determination method for ADC-1.1, except that the small molecule of formula 2 was replaced by a small molecule of formula 8, 12 eq of TCEP was added during antibody reduction, and 10 eq of small molecule payload was added during conjugation. 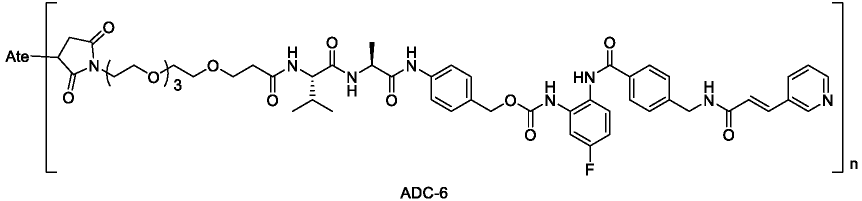 n was measured to be 7.66. See FIG. 6 for details.

### Preparation of ADC-7:

The preparation of ADC-7 was conducted by following the preparation procedures and DAR value determination method for ADC-1.1, except that the small molecule of formula 2 was replaced by a small molecule of formula 9, 12 eq of TCEP was added during antibody reduction, and 10 eq of small molecule payload was added during conjugation. 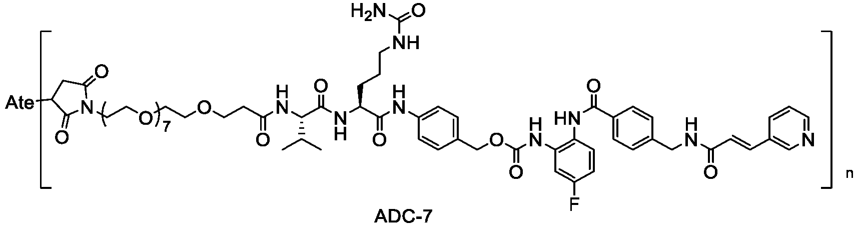 n was measured to be 7.50. See FIG. 7 for details.

### Example 11: Detection of Antibody-Drug Conjugate Aggregates

ADC detection instrument: Thermo vanquishi core; chromatographic column: BioCore SEC-300 5 µm 4.6 × 300 mm; mobile phase: 1× PBS; flow rate: 0.3 mL/min; isocratic elution; analysis time: 20 min; detection wavelength: 280 nm. The results are shown in Table 2.

**Table 2. Comparison of antibody-drug conjugate samples with naked antibody SEC**

| Sample name | Aggregation (%) | Main Peak (%) | Fragment (%) |
|---|---|---|---|
| Ate antibody | 3.25 | 96.75 | / |
| ADC-1.4 | 5.09 | 93.73 | 1.18 |
| ADC-1.5 | 4.62 | 94.27 | 1.11 |
| ADC-1.6 | 1.96 | 96.32 | 1.71 |
| ADC-3.3 | 3.87 | 96.13 | / |
| ADC-5 | 1.79 | 91.42 | 6.79 |
| ADC-6 | 2.19 | 93.73 | 4.08 |
| ADC-7 | 1.73 | 92.76 | 5.51 |

In the table, "/" means absent or undetectable.

The results show that the purity of the antibody-drug conjugate samples (e.g., ADC-1.4, ADC-1.5, ADC-1.6, ADC-3.3, ADC-5, ADC-6, and ADC-7) was comparable to that of the corresponding naked antibody.

### Example 12: Assay for Affinity of Antibody-Drug Conjugate Samples

### Assay for affinity by SPR method

Biacore T200 was used to measure the affinity of the antibody-drug conjugate samples (e.g., ADC-1.4, ADC-1.5, ADC-1.6, and ADC-3.3)/naked antibody (e.g., the Ate antibody) for Cyno PD-L1-his, hPD-L1-his, mPD-L1-his, and Rat PD-L1-his.

The procedures were as follows: A CM5 chip was activated with N-hydroxysuccinimide (NHS) and 1-ethyl-3-(3-dimethylaminopropyl)carbodiimide hydrochloride (EDC) at a 1:1 (v/v) ratio. Then, an anti-his antibody was immobilized, and the chip was blocked.

1 µg/mL hPD-L1-his was captured onto the chip surface. ADC-3.3 and the Ate antibody were 2-fold diluted with 1× PBST to give a total of 9 concentration points, with the dilution range being 25 nM-0.39 nM. The program was set. The samples were then loaded, and the program was executed. Data were analyzed using the Biacore Evaluation Software with the 1:1 binding model for kinetic fitting. The results are shown in Table 3-1 and FIG. 8.

**Table 3-1. Affinity comparison of antibody-drug conjugate sample (e.g., ADC-3.3) with Ate antibody**

| Ligand | Analyte | ka (1/Ms) | kd (1/s) | KD (M) |
|---|---|---|---|---|
| hPD-L1-His | Ate antibody | 1.26E+06 | <10⁻⁵ | <1x10⁻¹² |
| hPD-L1-His | ADC-3.3 | 1.02E+06 | <10⁻⁵ | <1x10⁻¹² |

2 µg/mL Cyno PD-L1-his was captured onto the chip surface. The antibody-drug conjugate samples and the naked antibody sample were 2-fold diluted with 1× PBST to give a total of 7 concentration points, with the sample concentrations all being 25 nM-0.39 nM. The program was set. The samples were then loaded, and the program was executed. Data were analyzed using the Biacore Evaluation Software with the 1:1 binding model for kinetic fitting. The above steps were repeated. 2 µg/mL hPD-L1-his, 5 µg/mL mPD-L1-his, and 5 µg/mL Rat PD-L1-his were captured onto the chip surface separately. The above 4 samples were 2-fold diluted with 1× PBST. For the Ate antibody and ADC-1.4, 8 concentration points were set, with the concentration range being 25 nM-0.39 nM. For ADC-1.5 and ADC-1.6, 9 points were set, with the concentration range being 50 nM-0.19 nM. The program was set. The samples were then loaded, and the program was executed. Data were analyzed using the Biacore Evaluation Software with the 1:1 binding model for kinetic fitting. The results are shown in Table 3-2 and FIGs. 9 to 24.

**Table 3-2. Affinity comparison of antibody-drug conjugate samples with Ate antibody**

| Ligand | Analyte | ka (1/Ms) | kd (1/s) | KD (M) |
|---|---|---|---|---|
| hPD-L1-His | Ate antibody | 2.46E+06 | < 1.00E-05 | < 1.00E-12 |
| hPD-L1-His | ADC-1.4 | 1.88E+06 | < 1.00E-05 | < 1.00E-12 |
| hPD-L1-His | ADC-1.5 | 1.12E+06 | < 1.00E-05 | < 1.00E-12 |
| hPD-L1-His | ADC-1.6 | 1.09E+06 | < 1.00E-05 | < 1.00E-12 |
| Cyno PD-L1-His | Ate antibody | 1.58E+06 | 1.30E-04 | 8.26E-11 |
| Cyno PD-L1-His | ADC-1.4 | 1.28E+06 | 9.49E-05 | 7.43E-11 |
| Cyno PD-L1-His | ADC-1.5 | 1.00E+06 | 4.24E-05 | 4.24E-11 |
| Cyno PD-L1-His | ADC-1.6 | 8.94E+05 | 1.96E-05 | 2.19E-11 |
| mPD-L1-His | Ate antibody | 1.76E+06 | 1.19E-04 | 6.74E-11 |
| mPD-L1-His | ADC-1.4 | 1.39E+06 | 1.09E-04 | 7.87E-11 |
| mPD-L1-His | ADC-1.5 | 8.10E+05 | 9.57E-05 | 1.18E-10 |
| mPD-L1-His | ADC-1.6 | 8.01E+05 | 8.81E-05 | 1.10E-10 |
| Rat-PD-L1-His | Ate antibody | 2.86E+06 | 9.81E-04 | 3.43E-10 |
| Rat-PD-L1-His | ADC-1.4 | 2.27E+06 | 1.00E-03 | 4.41E-10 |
| Rat-PD-L1-His | ADC-1.5 | 1.26E+06 | 9.32E-04 | 7.39E-10 |
| Rat-PD-L1-His | ADC-1.6 | 6.40E+05 | 7.80E-04 | 1.22E-09 |

As can be seen from the results, the affinity of the antibody-drug conjugate samples of the present application (e.g., ADC-1.4, ADC-1.5, ADC-1.6, and ADC-3.3) for Cyno PD-L1-his, hPD-L1-his, mPD-L1-his, and Rat PD-L1-his was comparable to that of the naked antibody (e.g., the Ate antibody).

### Example 13: Effects of Antibody-Drug Conjugate Samples on Total Acetylation Level of Cells

Assay for effects of ADC molecules of the present application (e.g., ADC-1.4, ADC-1.5, and ADC-1.6) on acetylation level of histone H4 in NCI-H292 cells
(1) NCI-H292 cells were collected, and 20 µL was taken for cell counting. (2) The cells were resuspended to 1 × 10⁵ cells/mL. (3) The cell suspension was added to a 48-well plate at 100 µl/well, and the cells were cultured at 37 °C with 5% CO₂ overnight. (4) ADC molecules were diluted to predetermined concentrations (ensuring that the concentration of chidamide carried by the added ADC molecules remained consistent at 19 µM) and added to the corresponding wells at 100 µL/well, and the cells were cultured at 37 °C with 5% CO₂ for 48 h. (5) The supernatant was aspirated and discarded, trypsin was added at 100 µL/well for digestion, and then the digestion was stopped with 300 µL of culture medium. The cells were resuspended, transferred to 1.5-mL centrifuge tubes, and centrifuged at 300 rcf for 5 min at room temperature. (6) 200 µL of fixation solution (4% paraformaldehyde) was added to each tube, and the mixture was immediately pipetted 2-3 times and then incubated at 4 °C for 15 min. (7) The mixture in each tube was washed once with 1 mL of PBS and centrifuged at 700 rcf for 5 min, and then the supernatant was discarded. (8) 50 µL of membrane permeabilization solution (1% Triton X-100) was added to each tube, and the mixture was immediately pipetted 2-3 times and then incubated at room temperature for 15 min. (9) Milli-Mark^{®} Anti-acetyl-Histone H4 Antibody-PE was added at 0.5 µL/tube, and the mixture was incubated at room temperature in the dark for 1 h. (10) The mixture in each tube was washed once with 1 mL of membrane permeabilization solution and centrifuged at 900 rcf for 5 min, and then the supernatant was discarded. (11) 100 µL of membrane permeabilization solution was added to each tube, and the mixture was thoroughly mixed by pipetting and then analyzed using a flow cytometer. (12) Data were processed through Graphpad Prism 9.0. The results are shown in FIG. 25.

As can be seen from the results, the antibody-drug conjugate (ADC) samples of the present application (e.g., ADC-1.4, ADC-1.5, and ADC-1.6) exhibited a significant promoting effect on acetylation of histone H4 in NCI-H292 cells.

### Example 14: Free Toxin Detection

Free linker-toxin in the antibody-drug conjugate samples (e.g., ADC-1.4, ADC-1.5, ADC-1.6, and ADC-3.3) was detected by the RP-HPLC method.

### HPLC analysis conditions:

Instrument: Waters e2695
Chromatographic column: XBridge^{®} C18 3.5 µm, 4.6 × 150 mm column
Mobile phase A: 0.1% TFA in water
Mobile phase B: 0.1% TFA in ACN
Detection wavelength: 254 nm
Flow rate: 0.5 mL/min
Elution gradient: 0-30 min 10%-80% B, 31-35 min 10% B
Sample treatment: 85 µg of a test sample to be tested (e.g., ADC-1.4, ADC-1.5, ADC-1.6, or ADC-3.3) was taken and mixed with 3 µL of DMSO for 5 min, and then 60 µL of saturated sodium chloride supernatant (dissolved in 30% methanol in acetonitrile) was added to the test sample. The mixture was thoroughly mixed for 10 min and centrifuged at 2000 rpm for 2 min. The supernatant was collected for testing.
Data analysis: Linker-toxin solutions at concentrations of 0.05 µg/mL, 0.1 µg/mL, 0.2 µg/mL, 0.5 µg/mL, 1 µg/mL, 2 µg/mL, and 5 µg/mL were injected as standards, and standard curves were plotted. If free linker-toxin was present in the sample, its peak area was substituted into the linear equation to calculate the concentration. The results are shown in FIG. 26.

As can be seen from the results, free linker-toxin was not detected in the antibody-drug conjugate (ADC) samples of the present application (e.g., ADC-1.4, ADC-1.5, ADC-1.6, and ADC-3.3).

### Example 15: Assay for Plasma Stability

Assay for plasma stability of antibody-drug conjugate sample (e.g., ADC-1.6) of the present application in plasma of different species

The specific procedures were as follows:
The antibody-drug conjugate sample was prepared into a stock solution at a concentration of 1 mg/mL using sterile PBS. The solution was prepared immediately before use. The preparation process was completed in a sterile environment, and all reagent bottles or containers were sterilized.

Plasma from various species (human, cynomolgus monkey, SD rat, and C57 mouse) was gently and thoroughly mixed at room temperature. Then, 3600 µL of plasma from each species was taken using a pipette, and 400 µL of test sample working solution was added to prepare a sample with a final test sample concentration of 100 µg/mL. Then, the prepared sample was gently shaken and thoroughly mixed for later use.

The incubation experiment had a final reaction system volume of 200 µL, with 2 replicates per time point. Samples were collected after incubation under sterile conditions at 37 °C for 0 h, 4 h, 8 h, 24 h, 48 h, 72 h, 120 h, and 168 h. All operations were performed under sterile conditions in the dark. Samples from each termination time point were frozen at -70 °C to -90 °C.

After sampling was completed, the samples were analyzed using the RP-HPLC method.

### HPLC analysis conditions:

Instrument: AB SCIEX TRIPLE QUADTM 4500 liquid chromatography-mass spectrometry system
Chromatographic column: Bridge BEH C18, 2.5 µm, 2.1 × 50 mm, Waters
Mobile phase A: 0.1% FA and 2 mmol/L aqueous ammonium formate solution
Mobile phase B: CAN
Strong needle wash (SNW): MeOH:ACN:IPA:DMSO = 1:1:1:1 (v/v/v/v) (containing 0.5% FA)
Weak needle wash (WNW): MeOH:H₂O = 1:1 (v/v)

### Sample treatment procedures:

The samples were thoroughly mixed by vortexing. According to the Plate map, 20 µL of each sample was transferred to a 96-well plate. 20 µL of ACN:H₂O = 1:1 (v/v) was added to DB and Carryover samples, and 20 µL of internal standard working solution (1000.000 ng/mL tolbutamide internal standard working solution) was added to the remaining wells. After vortexing for 1 min, 160 µL of ACN:MeOH = 1:1 containing 0.1% FA was added to all wells. The plate was sealed with a film, and the mixture was thoroughly mixed by vortexing at 1000 rpm for 5 min and then centrifuged at 4700 g for 10 min at 4 °C. According to the Plate map, 100 µL of supernatant from each well was transferred to a new 96-well plate. The plate was then sealed with a film, and the samples were injected for analysis. The calculation results are shown in Table 4 and FIG. 27.

**Table 4. Plasma stability of antibody-drug conjugate sample**

| Name of test sample | Species | Incubation time | Incubation concentration | Dissociation rate (%) |
|---|---|---|---|---|
| Ate antibody | C57 mouse | 168h | 100 µg/mL | NA |
| | SD rat | | | NA |
| | Cynomolgus monkey | | | NA |
| | Human | | | NA |
| ADC-1.6 | C57 mouse | | | 0.926 |
| | SD rat | | | 0.256 |
| | Cynomolgus monkey | | | 0.178 |
| | Human | | | 0.226 |

In the table, "NA" means undetectable.

As can be seen from the results, no small-molecule drugs were detected for the Ate antibody in plasma from different species, and the antibody-drug conjugate (ADC) sample of the present application (e.g., ADC-1.6) showed a dissociation rate of <1.0% after 168 h of incubation at 37 °C in plasma from the four species (human, cynomolgus monkey, SD rat, and C57 mouse), indicating overall stability.

### Example 16: Assay for Internalization of Antibody-Drug Conjugate (ADC) Sample in Tumor Cells

Assay for internalization of antibody-drug conjugate sample (e.g., ADC-1.6) in cells of human lung cancer cell line NCI-H292
(1) NCI-H292 cells were collected, and 20 µL was taken for cell counting. (2) The cells were resuspended to 6 × 10⁶ cells/mL, and the cell suspension was added into two 1.5-mL EP tubes at 100 µL/tube. (3) Diluted antibody-drug conjugate (ADC) group and antibody group samples were added separately at 100 µL/tube, achieving a final sample concentration of 150 nM. The mixtures were thoroughly mixed and then incubated at 4 °C in the dark for 1 h. (4) The mixtures were washed twice with pre-cooled PBS and centrifuged at 1200 rpm for 5 min at 4 °C, and then the supernatants were discarded. (5) Complete medium was added at 300 µL/tube to resuspend the cells, and the cell suspensions were separately transferred to new EP tubes at 150 µL/tube, with one tube kept at 4 °C and the other incubated at 37 °C. (6) After 2 h, 4 h, and 22 h, 50 µL of cell suspension was collected from each of the 4 °C and 37 °C tubes, transferred to new EP tubes, mixed with 1 mL of pre-cooled PBS, and centrifuged at 1200 rpm for 5 min at 4 °C, and then the supernatants were discarded. (7) A second antibody was added at 0.5 µL/tube, and the mixtures were thoroughly mixed and then incubated at 4 °C in the dark for 30 min. (8) The mixtures were washed once with pre-cooled PBS and centrifuged at 1200 rpm for 5 min at 4 °C, and then the supernatants were discarded. (9) Pre-cooled PBS was then added at 100 µL/tube to resuspend the cells, and the cell suspensions were analyzed on a machine. (10) The internalization rate was calculated using the following formula: Internalization (%) = positive rate₄ °_{C} (%) - positive rate₃₇ °_{C} (%), where the positive rate₄ °_{C} (%) represents the positive rate of cells incubated at 4 °C, and the positive rate₃₇ °_{C} (%) represents the positive rate of cells incubated at 37 °C. The data were processed by Graphpad Prism 9.0, and the results are shown in FIG. 28.

As can be seen from the results, the tumor cell internalization rates of both the antibody-drug conjugate (ADC) (e.g., ADC-1.6) group and the antibody (e.g., the Ate antibody) group increased over time, but the tumor cell internalization rate of the antibody-drug conjugate (ADC) group was significantly superior to that of the antibody group.

### Example 17: Inhibitory Effect of Antibody-Drug Conjugate Sample on Tumor Cells

17.1. Assay for inhibition of CT26-hPD-L1 cell proliferation by ADC molecules of the present application (e.g., ADC-3.3)
(1) CT26 hPD-L1-EX cells were collected, and 20 µL was taken for cell counting. (2) The cells were resuspended to 0.5 × 10⁵ cells/mL and 0.5 × 10⁵ cells/mL (5 mL each). (3) The cell suspensions were respectively added to two 96-well plates at 50 µL/well, followed by overnight culture at 37 °C with 5% CO₂. (4) The ADC sample was diluted according to predetermined concentrations and added to the well plates at 100 µL/well. (5) The cells were cultured at 37 °C with 5% CO₂ for 48 h. (6) 50 µL of culture medium was first aspirated and discarded, and then an MTS reagent was added at 20 µL/well.The cells were cultured at 37 °C with 5% CO₂ for another 1-4 h. (7) Readings were taken at 490 nm. The results are shown in FIG. 29 and Table 5.

**Table 5. Inhibition of CT26 cells by antibody-drug conjugate sample ADC-3.3**

| | Sample name | | ADC-3.3 | Chidamide | Ate antibody | Chi+Ate | medium |
|---|---|---|---|---|---|---|---|
| Concentration (5 µM) | CT26hPD-L1-EX | OD₄₉₀ | 0.4713 | 1.0283 | 1.1715 | 1.117 | 1.2257 |
| | | | 0.527 | 1.0841 | 1.1587 | 1.0675 | 1.2282 |
| | | Inhibition % | 61.55 % | 16.11 % | 4.42 % | 8.87 % | 0.00 % |
| | | | 57.09 % | 11.73 % | 5.66 % | 13.08 % | 0.00 % |

As can be seen from the results, the antibody-drug conjugate (ADC) sample of the present application (e.g., ADC-3.3) demonstrated a significant inhibitory effect on CT26-hPD-L1 cell proliferation, and its inhibitory effect was significantly superior to that of the small-molecule drug (e.g., chidamide) alone, the naked antibody (e.g., the Ate antibody) alone, and the combined use of the small-molecule drug and the naked antibody (e.g., chidamide +Ate antibody, i.e., Chi + Ate).

17.2. Assay for inhibition of colorectal cancer cell line CT26 cell proliferation by ADC molecules of the present application (e.g., ADC-1.6)
(1) CT26 hPD-L1-EX cells were collected, and 20 µL was taken for cell counting. (2) The cells were resuspended to 2 × 10⁵ cells/mL. (3) The cell suspension was added to a 96-well plate at 50 µL/well (1 × 10⁴ cells/well), followed by overnight culture at 37 °C with 5% CO₂. (4) The next day, the 96-well plate was taken out. The test sample was diluted with complete medium containing 10% FBS and then added to the plate at 50 µL/well to achieve a final sample concentration of 10 µM. The day of test sample addition was designated as day 0. The cells were cultured at 37 °C with 5% CO₂ for another 2 days. (5) Day 2: The cell growth status was observed under a microscope, and then CCK8 was added at 10 µL/well. The cells were then cultured at 37 °C with 5% CO₂ for another 4 h. (6) Absorbance values at 450 nm (OD₄₅₀) were measured in real-time using a microplate reader. The results are shown in FIG. 30 and Table 6.

**Table 6. Inhibition of CT26 cells by antibody-drug conjugate sample ADC-1.6**

| | Sample name | | ADC-1.6 | Chidamide | Ate antibody | Chi+Ate | medium |
|---|---|---|---|---|---|---|---|
| Concentration (10 µM) | CT26hPD-L1-EX | OD₄₅₀ | 0.1460 | 1.9014 | 2.1282 | 1.9207 | 2.2628 |
| | | | 0.1437 | 1.9554 | 1.9824 | 1.9038 | 2.2164 |
| | | | 0.1488 | 1.7934 | 1.9349 | 1.7512 | 2.1988 |
| | | Inhibition % | 93.44 % | 14.58 % | 4.39 % | 13.72 % | 0.00 % |
| | | | 93.54 % | 12.16 % | 10.94 % | 14.47 % | 0.00 % |
| | | | 93.32 % | 19.43 % | 13.08 % | 21.33 % | 0.00 % |

As can be seen from the results, the antibody-drug conjugate (ADC) (e.g., ADC-1.6) group demonstrated a greater inhibitory effect on CT26 hPD-L1-EX cell proliferation compared to the small-molecule drug (e.g., chidamide) group, the antibody (e.g., the Ate antibody) group, and the combined use (e.g., chidamide + Ate antibody, i.e., Chi + Ate) group.

17.3. Assay of inhibitory effect of ADC molecules of the present application (e.g., ADC-1.6) on human malignant melanoma cell line A375 in co-culture system with human peripheral blood mononuclear cells (PBMCs) and human malignant melanoma cell line A375

The specific procedures were as follows:
Cryopreserved PBMCs were taken out from a liquid nitrogen tank and added to 8 mL of pre-heated RPMI 1640 medium, and the mixture was centrifuged at 500 g for 10 min. The supernatant was discarded, and the PBMCs were resuspended in a small amount of RPMI 1640 medium. Then, the cell suspension was 10-fold diluted, and 20 µL of dilution was taken for cell counting. The cells were resuspended to 5E6 cells/mL. The A375-hPDL1-Luciferase cell suspension was collected, and 20 µL was taken for cell counting. The cells were resuspended to 1E6 cells/mL. The PBMC and A375-hPDL1-Luciferase cell suspensions were mixed at equal volumes to prepare a cell mixture with an E:T ratio of 5:1, and the mixture was adjusted to a total volume of 1500 µL using RPMI 1640 complete medium. Matrigel was thawed on ice, and 1500 µL of the thawed Matrigel was mixed with the cell mixture. The resulting mixture was added to a black 384-well plate at 20 µL/well, which was then placed at 37 °C for 10 min for solidification. ADC-1.6 and the Ate antibody were diluted to 5 µM, 1 µM, 0.2 µM, 0.04 µM, 0.008 µM, 0.0016 µM, and 0 µM dilutions, and chidamide was diluted to 200 µM, 40 µM, 8 µM, 1.6 µM, 0.32 µM, 0.064 µM, 0.0128 µM, and 0 µM dilutions, with 8 concentration gradients and 3 replicates per sample. The dilutions were added to the 384-well plate at 30 µL/well, and the cells were cultured at 37 °C with 5% CO₂ for 6 days. Then, the 384-well plate was taken out, and Bio-Lite Luciferase Assay System was added at 20 µL/well.The mixture was incubated for 5-10 min and then assayed using a multi-mode microplate reader. The results are shown in FIG. 31, FIG. 32, and Table 7.

**Table 7. Inhibition of A375 cells by antibody-drug conjugate sample ADC-1.6**

| | Ate antibody | ADC-1.6 | Combo (Chidamide:Ate antibody = 8:1) | Chidamide |
|---|---|---|---|---|
| IC50 (µM) | 1.09E+08 | <0.01 | 0.03206 | 0.8232 |

As can be seen from the results, the IC₅₀ value of the antibody-drug conjugate (ADC) (e.g., ADC-1.6) group was < 0.01 µM, while the IC₅₀ values of the Ate antibody group, the chidamide group, and the combined use (chidamide:Ate antibody = 8:1) group were all > 0.03 µM. The antibody-drug conjugate (ADC) (e.g., ADC-1.6) group showed a significant proliferation inhibitory effect on A375-hPDL1-Luciferase cells at lower concentrations compared to the small molecule (e.g., chidamide) group, the antibody (e.g., the Ate antibody) group, and the combined use (e.g., chidamide + Ate antibody, i.e., Chi + Ate) group. At a concentration of 0.008 µM, the antibody-drug conjugate (ADC) (e.g., ADC-1.6) group demonstrated a significantly greater inhibitory effect on A375-hPDL1-Luciferase cell proliferation compared to the small-molecule drug (e.g., chidamide) group, the antibody (e.g., the Ate antibody) group, and the combined use (e.g., chidamide + Ate antibody, i.e., Chi + Ate) group.

### Example 18: Anti-Tumor Efficacy of Antibody-Drug Conjugate (ADC) Sample

*In-vivo* pharmacodynamic study of antibody-drug conjugate (ADC) of the present application (e.g., ADC-1.6) using C57BL/6J mouse MC38 tumor model

18.1. The specific procedures were as follows: On the day of cell inoculation, each C57BL/6J mouse was subcutaneously inoculated with 3 × 10⁵ MC38 cells (0.1 mL/mouse). When the average tumor volume in mice reached about 70-80 mm³, grouping was performed. A total of 24 mice were divided into 3 groups, and drug administration began on the day of grouping. The administration regimen was three times per week, and the route of administration was intraperitoneal injection. The naked antibody group and the drug conjugate ADC sample group were intraperitoneally injected with 10 mg/kg Ate antibody and ADC-1.6, respectively. The vehicle control group was intraperitoneally injected with an equivalent volume of PBS solution. After the start of administration, the body weight and tumor volume were measured three times per week. The tumor volume was calculated as: tumor volume (mm³) = 0.5 × long diameter of tumor × (short diameter of tumor)². The experiment was terminated on day 7 post-administration. All mice were euthanized, and tumor tissues were collected and weighed. The *in-vivo* anti-tumor effect of the antibody-drug conjugate (ADC) sample is shown in FIG. 33.

As can be seen from the results, on day 7 post-administration, the tumor volume in the group treated with the antibody-drug conjugate sample ADC-1.6 of the present application was 455.74 mm³, while the tumor volumes in the naked antibody group and the vehicle control group were 701.09 mm³ and 821.79 mm³, respectively. The tumor growth inhibition rate TGI (%) of the antibody-drug conjugate sample ADC-1.6 group was 48.97%, showing a significant difference compared to the vehicle control group (P < 0.05). In addition, the inhibitory effect of the ADC group was significantly superior to that of the Ate antibody group (TGI = 16.26%).

18.2. The specific procedures were as follows: On the day of cell inoculation, each C57BL/6J mouse was subcutaneously inoculated with 3 × 10⁵ MC38 cells (0.1 mL/mouse). When the average tumor volume in mice reached about 50-60 mm³, grouping was performed. A total of 24 mice were divided into 3 groups, and drug administration began on the day of grouping. The administration regimen was three times per week, and the route of administration was intraperitoneal injection. The combined use (Ate antibody + chidamide) group was intraperitoneally injected with 10 mg/kg mAb and 0.2 mg/kg chidamide. The drug conjugate ADC sample group was intraperitoneally injected with 10 mg/kg ADC-1.6. The vehicle control group was intraperitoneally injected with an equivalent volume of PBS solution. After the start of administration, the body weight and tumor volume were measured three times per week. The tumor volume was calculated as: tumor volume (mm³) = 0.5 × long diameter of tumor × (short diameter of tumor)². The experiment was terminated on day 7 post-administration. All mice were euthanized, and tumor tissues were collected and weighed. The *in-vivo* anti-tumor effect of the antibody-drug conjugate (ADC) sample is shown in FIG. 34.

As can be seen from the results, on day 7 post-administration, the tumor volume in the group treated with the antibody-drug conjugate sample ADC-1.6 of the present application was 329.63 mm³, while the tumor volumes in the combined use group and the vehicle control group were 457.35 mm³ and 499.89 mm³, respectively. The TGI (%) of the antibody-drug conjugate sample ADC-1.6 group was 38.26%, showing a significant difference compared to the vehicle control group (P < 0.05). In addition, the inhibitory effect of the ADC group was significantly superior to that of the combined use group (TGI = 9.63%).

The present disclosure has been illustrated through various specific examples. However, it is appreciated by those of ordinary skill in the art that the present disclosure is not limited to the specific embodiments. Various changes or modifications can be made by those of ordinary skill in the art within the scope of the present disclosure, and the technical features mentioned in the specification can be combined with one another without departing from the spirit and scope of the present disclosure. Such changes and modifications are all within the scope of the present disclosure.

## Claims

1. A compound represented by formula I, a stereoisomer thereof, a tautomer thereof, a pharmaceutically acceptable salt thereof, a deuterated compound thereof, or a solvate thereof: 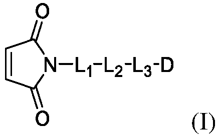 wherein
L₁ is selected from 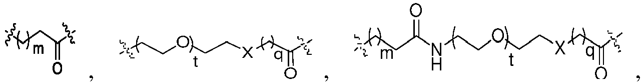 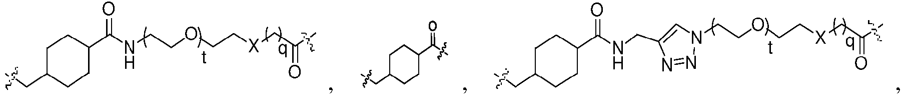 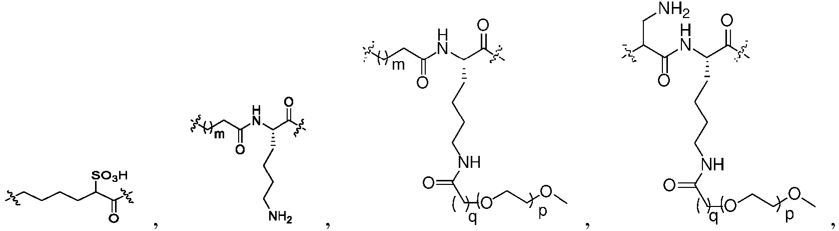 and 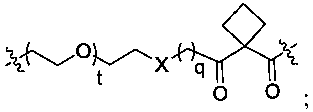
preferably, the carbon end of L₁ is linked to the N of succinimide, and the carbonyl end of L₁ is linked to L₂;
m and t are each independently selected from: 0, 1, 2, 3, 4, 5, 6, 7, and 8;
q is 0, 1, 2, or 3;
p is 0, 1, 2, 3, 4, 5, 6, 7, 8, 9, 10, 11, 12, 13, 14, or 15;
X is selected from: CH₂, O, and NH;
L₂ is absent, or is an amino acid residue, or is a peptide residue consisting of 2-10 amino acid residues;
preferably, the amino end of L₂ is linked to L₁, and the carbonyl end of L₂ is linked to L₃;
preferably, the amino acid includes, but is not limited to, phenylalanine (F), glycine (G), valine (V), lysine (K), serine (S), glutamic acid (E), asparagine (N), arginine (R), alanine (A), citrulline, and cysteine (C);
L₃ is absent, or is selected from 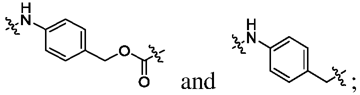
preferably, the amino end of L₃ is linked to L₂, and the carbonyl end or carbon end of L₃ is linked to D;
D is a histone deacetylase inhibitor drug selected from a thiol-based histone deacetylase inhibitor, a hydroxamic acid-based histone deacetylase inhibitor, and a benzamide-based histone deacetylase inhibitor.

2. The compound, the stereoisomer thereof, the tautomer thereof, the pharmaceutically acceptable salt thereof, the deuterated compound thereof, or the solvate thereof according to claim 1, wherein
L₁ is selected from: 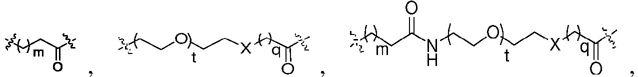 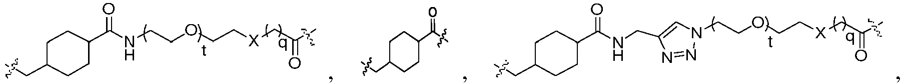 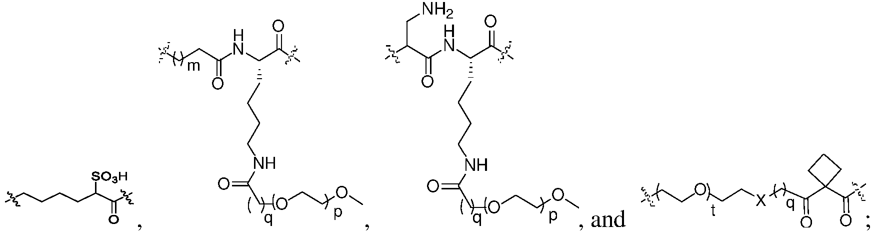
preferably, L₁ is selected from: 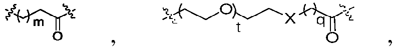 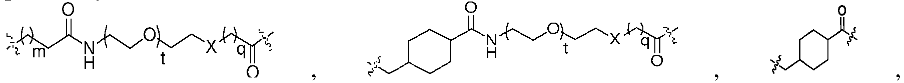 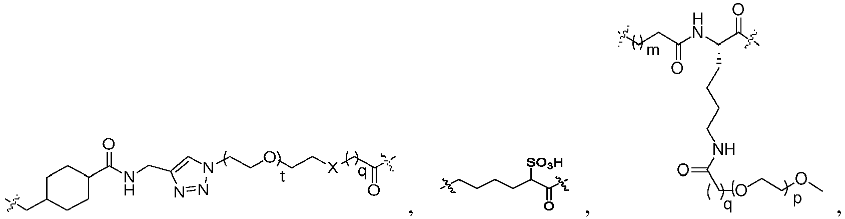 and 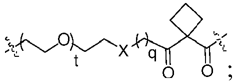
preferably, m is selected from: 0, 1, 2, 3, 4, and 5; more preferably, m is selected from 1, 3, and 4;
preferably, t is selected from: 0, 1, 2, 3, 4, 5, 6, and 7; more preferably, t is selected from 1, 2, 3, and 7;
preferably, q is selected from 0, 1, and 2;
preferably, p is selected from 10, 11, 12, 13, 14, and 15; more preferably, p is selected from 11, 12, and 13; further preferably, p is 12;
preferably, X is selected from O and NH;
more preferably, L₁ is selected from 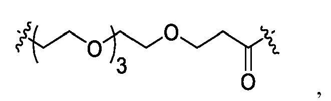 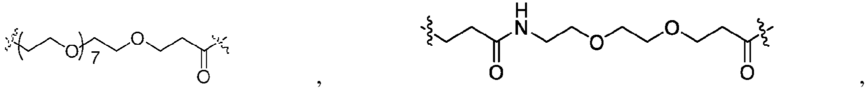 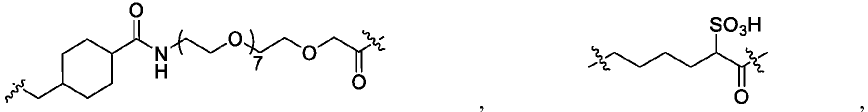 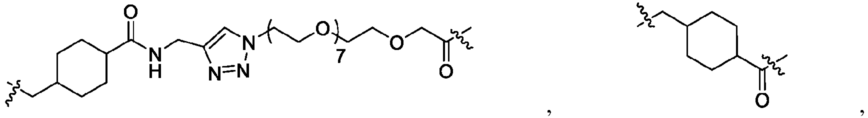 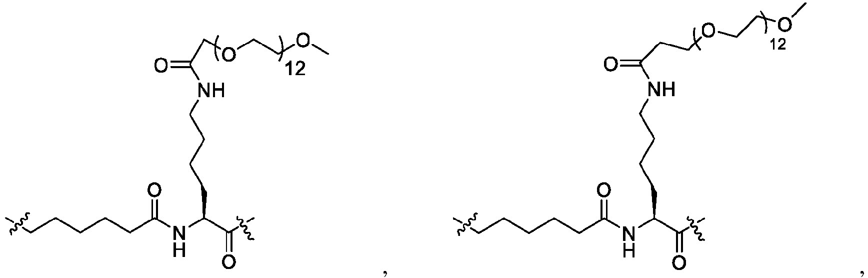 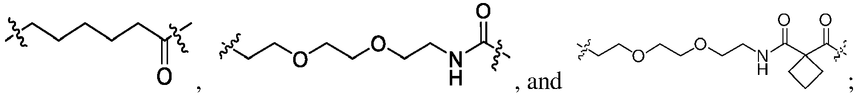
more preferably, L₁ is selected from 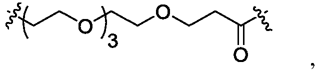 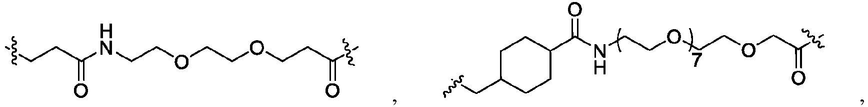 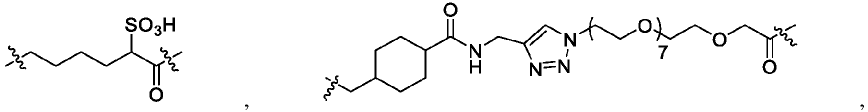 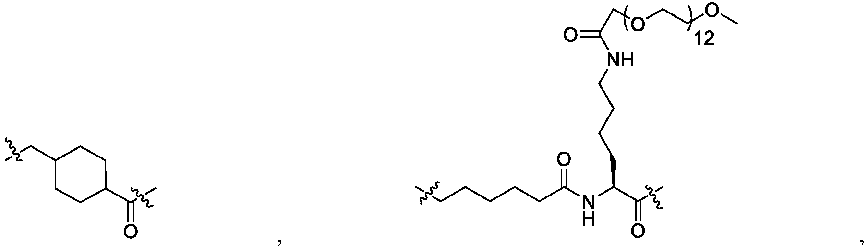 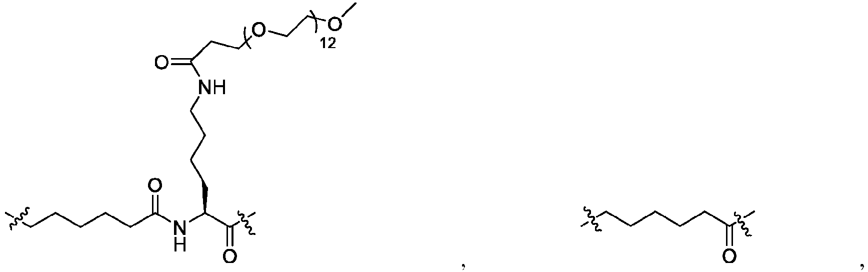 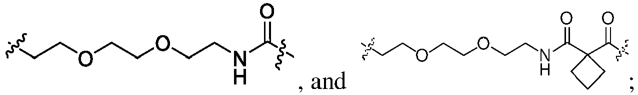
further preferably, L₁ is selected from 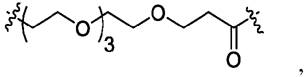 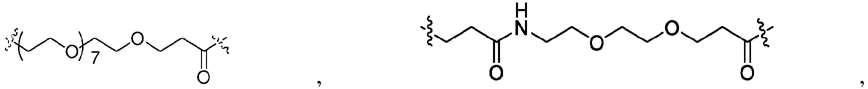 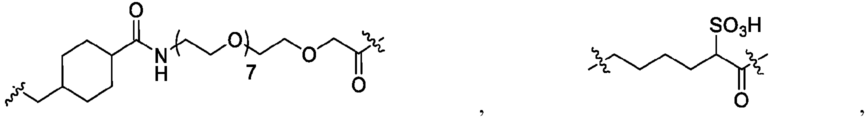 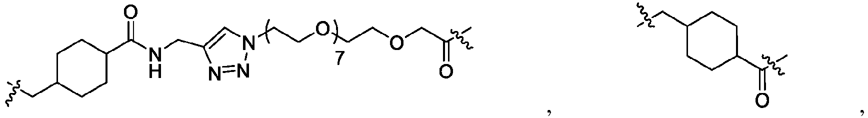 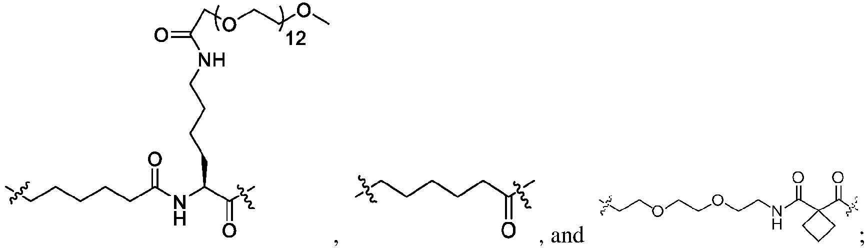
still further preferably, L₁ is 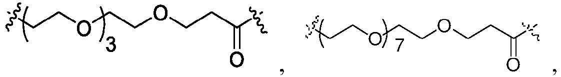 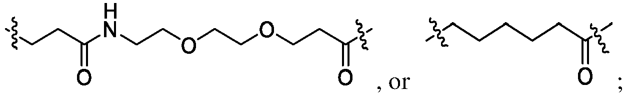
still further preferably, L₁ is

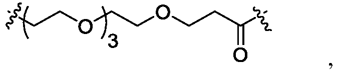

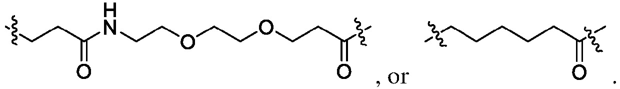

3. The compound, the stereoisomer thereof, the tautomer thereof, the pharmaceutically acceptable salt thereof, the deuterated compound thereof, or the solvate thereof according to any one of claims 1-2, wherein
L₂ is absent, or is an amino acid residue, or is a peptide residue consisting of 2-4 amino acid residues;
preferably, the amino acid includes, but is not limited to, phenylalanine (F), glycine (G), valine (V), alanine (A), citrulline, and cysteine;
more preferably, the amino acid includes, but is not limited to, phenylalanine (F), glycine (G), valine (V), alanine (A), and citrulline;
preferably, L₂ is absent, or is a citrulline residue, or is selected from the following peptide residues: valine residue-alanine residue, valine residue-citrulline residue, glycine amino-glycine residue-phenylalanine residue-glycine residue, valine residue-alanine residue-phenylalanine residue-glycine residue, glycine residue-glycine residue-glycine residue, glycine residue-phenylalanine residue-glycine residue, and valine residue-cysteine-phenylalanine residue-glycine residue;
more preferably, L₂ is absent, or is a citrulline residue, or is selected from the following peptide residues: valine residue-alanine residue, valine residue-citrulline residue, glycine amino-glycine residue-phenylalanine residue-glycine residue, and valine residue-alanine residue-phenylalanine residue-glycine residue;
further preferably, L₂ is absent, or is selected from 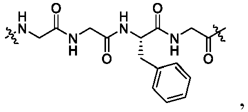 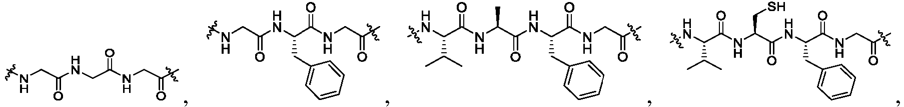 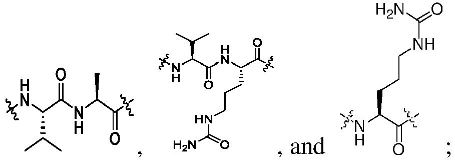
still further preferably, L₂ is absent, or is selected from 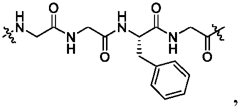 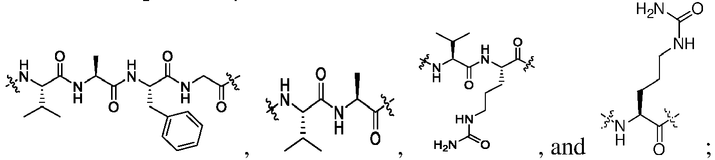
or still further preferably, L₂ is absent, or is selected from 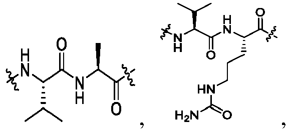 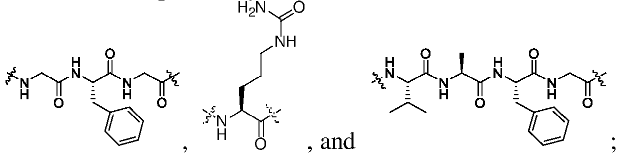
most preferably, L₂ is absent, or is selected from

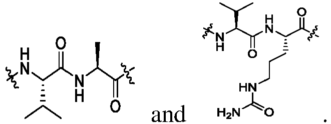

4. The compound, the stereoisomer thereof, the tautomer thereof, the pharmaceutically acceptable salt thereof, the deuterated compound thereof, or the solvate thereof according to any one of claims 1-3, wherein
D is a benzamide-based histone deacetylase inhibitor;
preferably, D is selected from 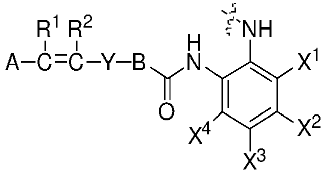 and 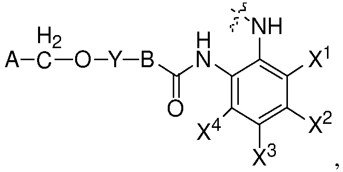 wherein
A is phenyl or pyridyl, and the phenyl or pyridyl is each independently and optionally substituted with 1-4 substituents selected from: halogen, C₁-C₄ alkyl (e.g., methyl and ethyl), and C₁-C₄ haloalkyl (e.g., trifluoromethyl);
preferably, A is phenyl or pyridyl, and the phenyl or pyridyl is each independently and optionally substituted with 1-2 substituents selected from: halogen, C₁-C₄ alkyl (e.g., methyl and ethyl), and trifluoromethyl;
more preferably, A is pyridyl, and the pyridyl is optionally substituted with 1-2 substituents selected from: C₁-C₄ alkyl;
further preferably, A is pyridyl;
most preferably, A is 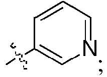
B is phenylene;
preferably, B is 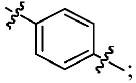
Y is -CO-NH-CH₂-;
preferably, the methylene end of Y is linked to B, and the carbonyl end of Y is linked to the alkenyl carbon or O;
R¹ and R² are each independently selected from hydrogen and C₁-C₄ alkyl;
preferably, R¹ and R² are both hydrogen;
any one of X¹, X², X³ , and X⁴ is selected from hydrogen, halogen, and C₁-C₄ alkyl, and the remaining three are hydrogen;
preferably, any one of X¹, X², X³, and X⁴ is selected from hydrogen and halogen, and the remaining three are hydrogen;
more preferably, any one of X¹, X², X³, and X⁴ is selected from hydrogen and fluorine, and the remaining three are hydrogen;
most preferably, X² is selected from hydrogen and fluorine, and X¹, X³, and X⁴ are hydrogen;
more preferably, D is selected from 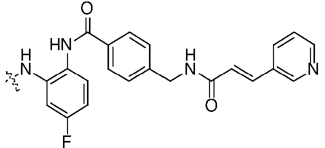 and

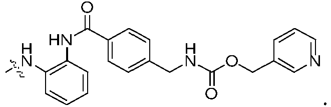

5. The compound, the stereoisomer thereof, the tautomer thereof, the pharmaceutically acceptable salt thereof, the deuterated compound thereof, or the solvate thereof according to any one of claims 1-4, wherein the compound is selected from:

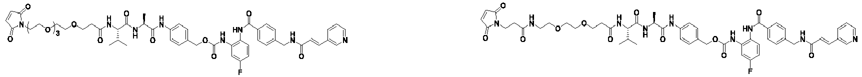

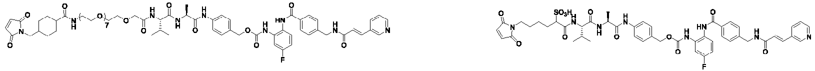

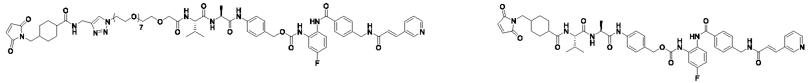

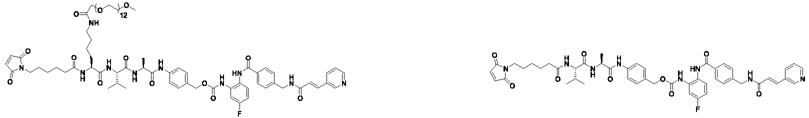

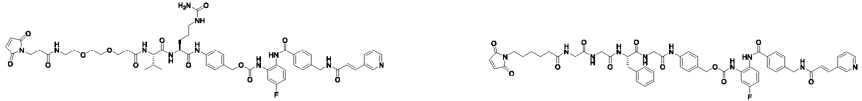

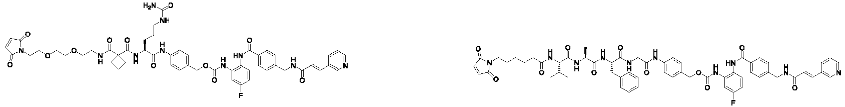

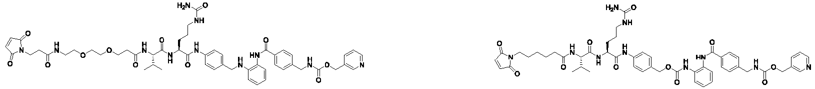

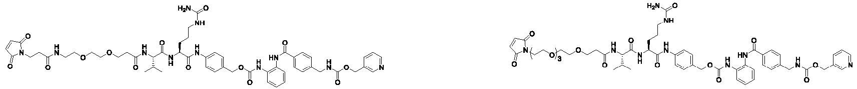

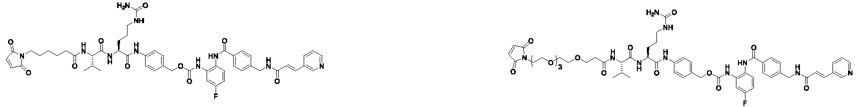

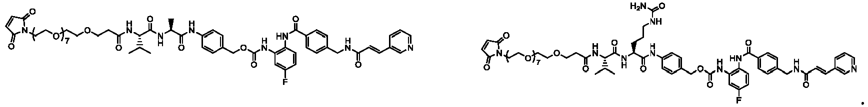

6. Use of the compound, the stereoisomer thereof, the tautomer thereof, the pharmaceutically acceptable salt thereof, the deuterated compound thereof, or the solvate thereof according to any one of claims 1-5 in the preparation of a ligand-drug conjugate (e.g., an antibody-drug conjugate).

7. A ligand-drug conjugate represented by formula II, a stereoisomer thereof, a tautomer thereof, a pharmaceutically acceptable salt thereof, a deuterated compound thereof, or a solvate thereof: 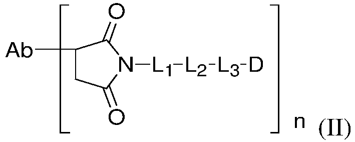 wherein
Ab is a ligand selected from a protein, an antibody, a polypeptide, an enzyme, and a small molecule;
n is a number between 0.5 and 8.5; for example, n is a number between 0.8 and 5, a number between 1 and 4, a number between 2 and 6, a number between 3 and 7, a number between 4 and 8, a number between 3.5 and 8.5, a number between 3.5 and 4.5, or a number between 6.5 and 8.5; preferably, n is about 2, 3, 4, 5, 6, 7, or 8; more preferably, n is about 3, 4, 5, 6, 7, or 8;
L₁ is selected from 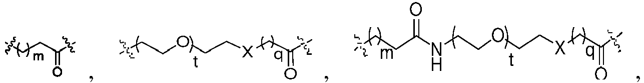 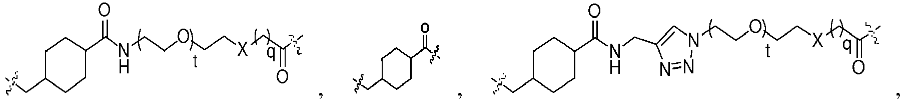 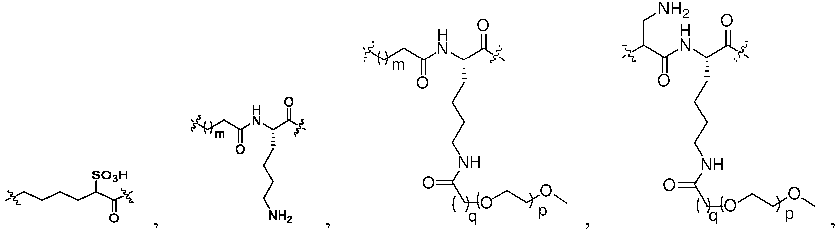 and 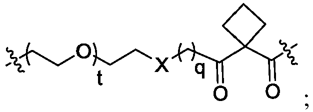
preferably, the carbon end of L₁ is linked to the N of succinimide, and the carbonyl end of L₁ is linked to L₂;
m and t are each independently selected from: 0, 1, 2, 3, 4, 5, 6, 7, and 8;
q is 0, 1, 2, or 3;
p is 0, 1, 2, 3, 4, 5, 6, 7, 8, 9, 10, 11, 12, 13, 14, or 15;
X is selected from: CH₂, O, and NH;
L₂ is absent, or is an amino acid residue, or is a peptide residue consisting of 2-10 amino acid residues;
preferably, the amino end of L₂ is linked to L₁, and the carbonyl end of L₂ is linked to L₃;
preferably, the amino acid includes, but is not limited to, phenylalanine (F), glycine (G), valine (V), lysine (K), serine (S), glutamic acid (E), asparagine (N), arginine (R), alanine (A), citrulline, and cysteine (C);
L₃ is absent, or is selected from 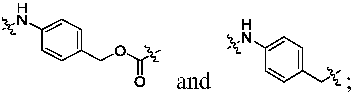 preferably, the amino end of L₃ is linked to L₂, and the carbonyl end or carbon end of L₃ is linked to D;
D is a histone deacetylase inhibitor drug selected from a thiol-based histone deacetylase inhibitor, a hydroxamic acid-based histone deacetylase inhibitor, and a benzamide-based histone deacetylase inhibitor.

8. The ligand-drug conjugate, the stereoisomer thereof, the tautomer thereof, the pharmaceutically acceptable salt thereof, the deuterated compound thereof, or the solvate thereof according to claim 7, wherein
L₁ is selected from: 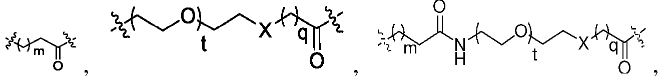 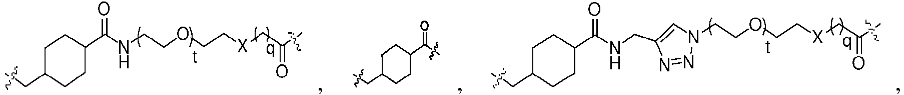 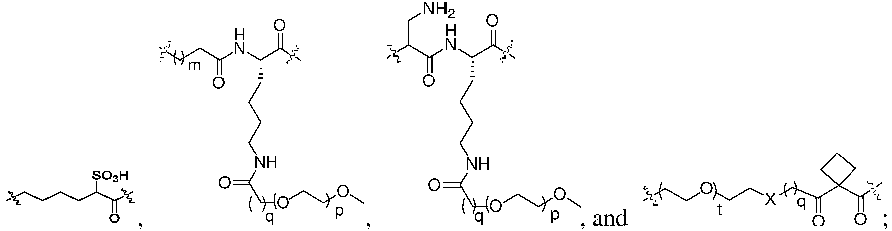
preferably, L₁ is selected from: 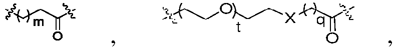 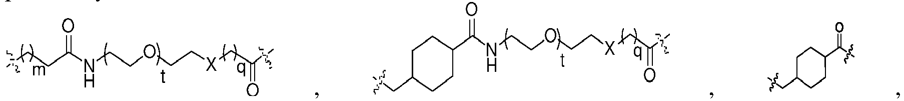 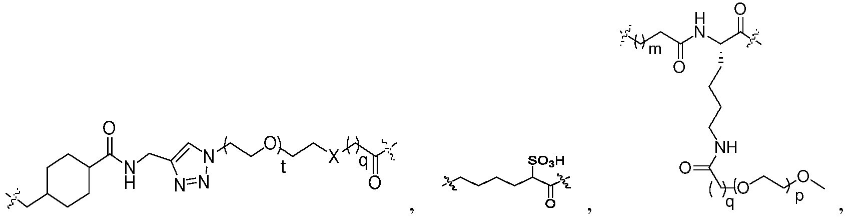 and 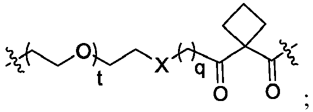
preferably, m is selected from: 0, 1, 2, 3, 4, and 5; more preferably, m is selected from 1, 3, and 4;
preferably, t is selected from: 0, 1, 2, 3, 4, 5, 6, and 7; more preferably, t is selected from 1, 2, 3, and 7;
preferably, q is selected from 0, 1, and 2;
preferably, p is selected from 10, 11, 12, 13, 14, and 15; more preferably, p is selected from 11, 12, and 13; further preferably, p is 12;
preferably, X is selected from O and NH;
more preferably, L₁ is selected from 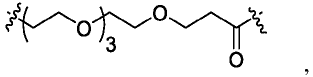 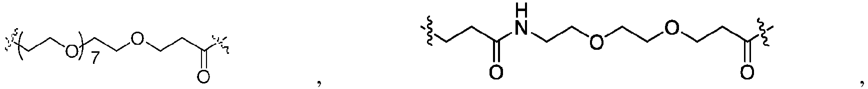 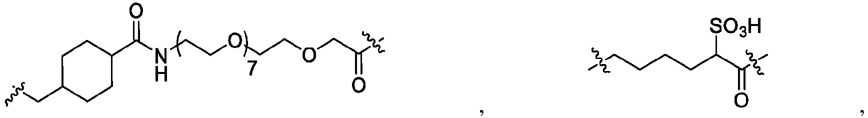 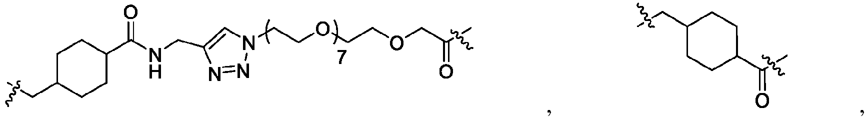 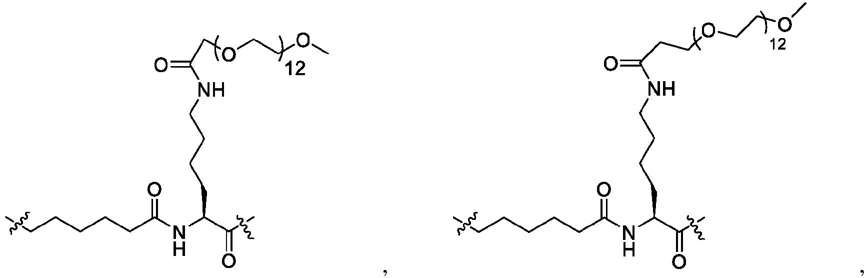 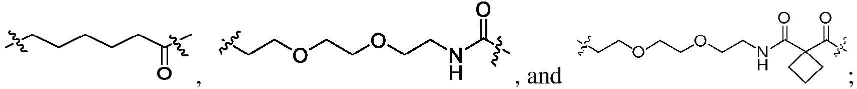
more preferably, L₁ is selected from 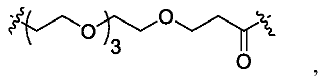 , 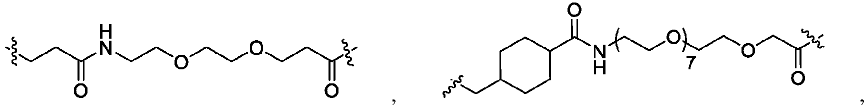 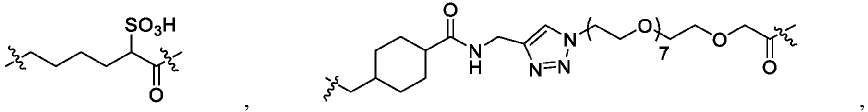 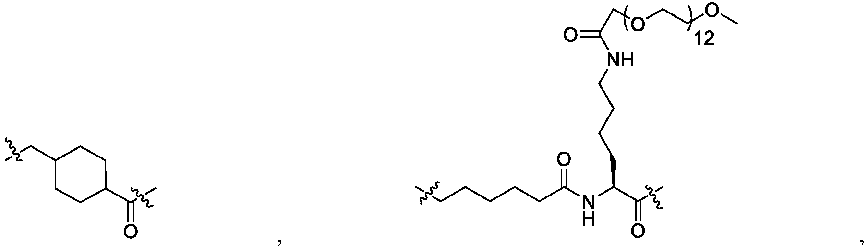 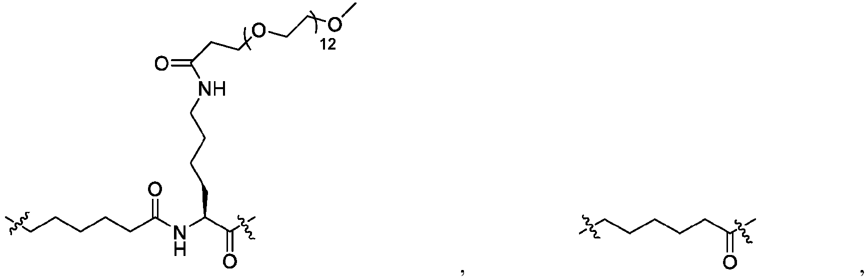 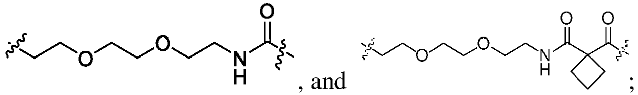
further preferably, L₁ is selected from 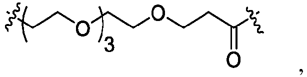 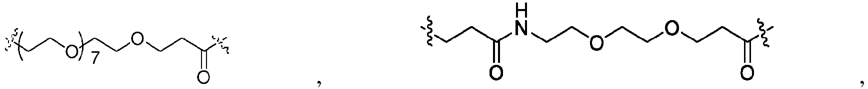 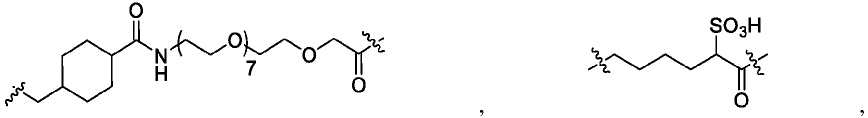 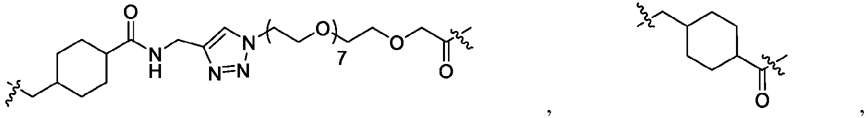 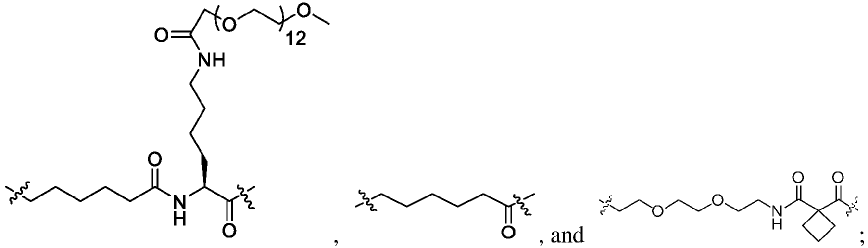
still further preferably, L₁ is 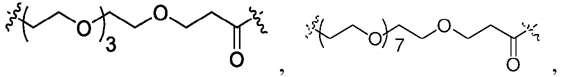 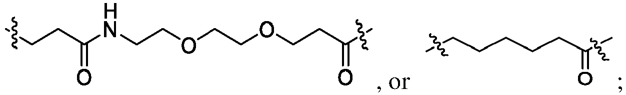
still further preferably, L₁ is

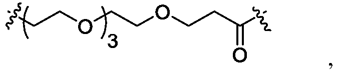

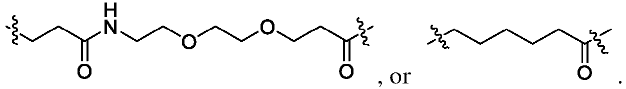

9. The ligand-drug conjugate, the stereoisomer thereof, the tautomer thereof, the pharmaceutically acceptable salt thereof, the deuterated compound thereof, or the solvate thereof according to any one of claims 7-8, wherein
L₂ is absent, or is an amino acid residue, or is a peptide residue consisting of 2-4 amino acid residues;
preferably, the amino acid includes, but is not limited to, phenylalanine (F), glycine (G), valine (V), alanine (A), citrulline, and cysteine;
more preferably, the amino acid includes, but is not limited to, phenylalanine (F), glycine (G), valine (V), alanine (A), and citrulline;
preferably, L₂ is absent, or is a citrulline residue, or is selected from the following peptide residues: valine residue-alanine residue, valine residue-citrulline residue, glycine amino-glycine residue-phenylalanine residue-glycine residue, valine residue-alanine residue-phenylalanine residue-glycine residue, glycine residue-glycine residue-glycine residue, glycine residue-phenylalanine residue-glycine residue, and valine residue-cysteine-phenylalanine residue-glycine residue;
more preferably, L₂ is absent, or is a citrulline residue, or is selected from the following peptide residues: valine residue-alanine residue, valine residue-citrulline residue, glycine amino-glycine residue-phenylalanine residue-glycine residue, and valine residue-alanine residue-phenylalanine residue-glycine residue;
further preferably, L₂ is absent, or is selected from 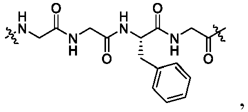 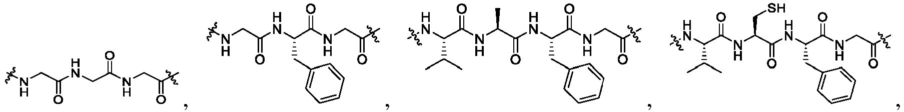 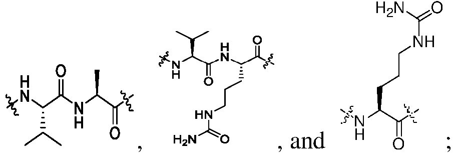
still further preferably, L₂ is absent, or is selected from 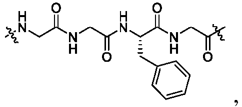 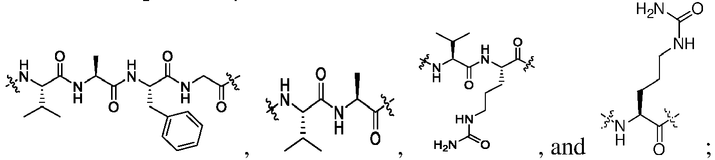 or still further preferably, L₂ is absent, or is selected from 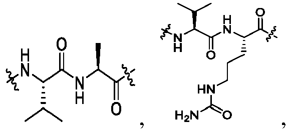 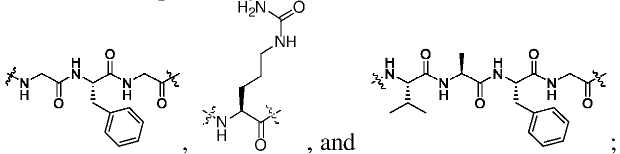
most preferably, L₂ is absent, or is selected from

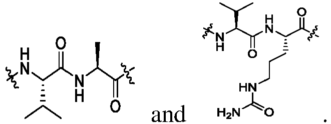

10. The ligand-drug conjugate, the stereoisomer thereof, the tautomer thereof, the pharmaceutically acceptable salt thereof, the deuterated compound thereof, or the solvate thereof according to any one of claims 7-9, wherein
D is a benzamide-based histone deacetylase inhibitor;
preferably, D is selected from 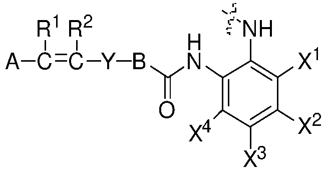 and 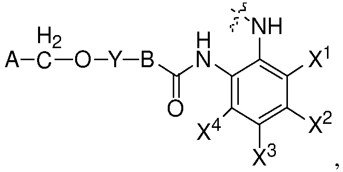 wherein
A is phenyl or pyridyl, and the phenyl or pyridyl is each independently and optionally substituted with 1-4 substituents selected from: halogen, C₁-C₄ alkyl (e.g., methyl and ethyl), and C₁-C₄ haloalkyl (e.g., trifluoromethyl);
preferably, A is phenyl or pyridyl, and the phenyl or pyridyl is each independently and optionally substituted with 1-2 substituents selected from: halogen, C₁-C₄ alkyl (e.g., methyl and ethyl), and trifluoromethyl;
more preferably, A is pyridyl, and the pyridyl is optionally substituted with 1-2 substituents selected from: C₁-C₄ alkyl;
further preferably, A is pyridyl;
most preferably, A is 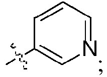
B is phenylene;
preferably, B is 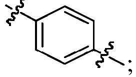
Y is -CO-NH-CH₂-;
preferably, the methylene end of Y is linked to B, and the carbonyl end of Y is linked to the alkenyl carbon or O;
R¹ and R² are each independently selected from hydrogen and C₁-C₄ alkyl;
preferably, R¹ and R² are both hydrogen;
any one of X¹, X², X³, and X⁴ is selected from hydrogen, halogen, and C₁-C₄ alkyl, and the remaining three are hydrogen;
preferably, any one of X¹, X², X³, and X⁴ is selected from hydrogen and halogen, and the remaining three are hydrogen;
more preferably, any one of X¹, X², X³, and X⁴ is selected from hydrogen and fluorine, and the remaining three are hydrogen;
most preferably, X² is selected from hydrogen and fluorine, and X¹, X³, and X⁴ are hydrogen;
more preferably, D is selected from 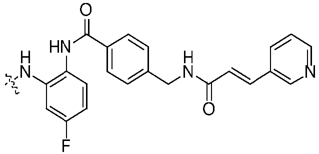 and

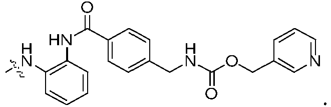

11. The ligand-drug conjugate, the stereoisomer thereof, the tautomer thereof, the pharmaceutically acceptable salt thereof, the deuterated compound thereof, or the solvate thereof according to any one of claims 7-10, wherein
Ab is an antibody selected from, without limitation: an anti-EGFR antibody, an anti-CD20 antibody, and an anti-PD-L1 antibody;
preferably, Ab is selected from, without limitation, cetuximab, panitumumab, necitumumab, rituximab, tositumomab (tositumomab + iodine 131 tositumomab), ofatumumab, obinutuzumab, ocrelizumab, atezolizumab, avelumab, durvalumab, and cemiplimab (cemiplimab-rwlc);
further preferably, Ab is atezolizumab or avelumab.

12. The ligand-drug conjugate, the stereoisomer thereof, the tautomer thereof, the pharmaceutically acceptable salt thereof, the deuterated compound thereof, or the solvate thereof according to any one of claims 7-11, wherein the ligand-drug conjugate is selected from:

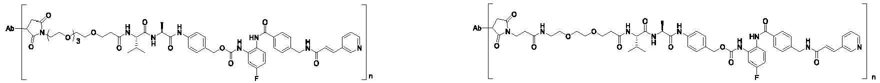

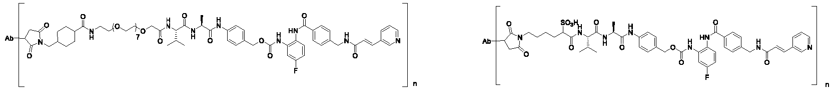

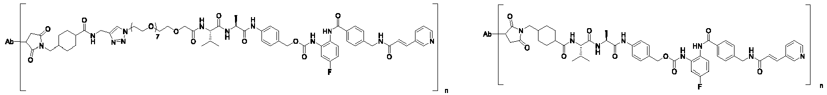

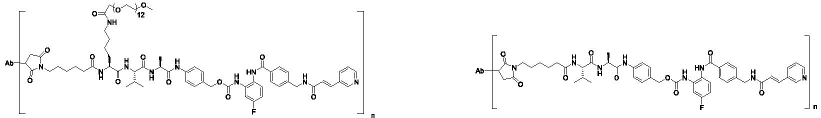

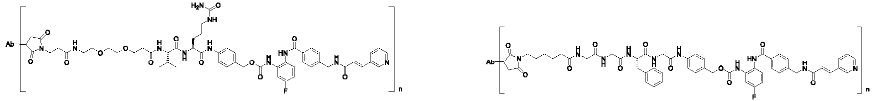

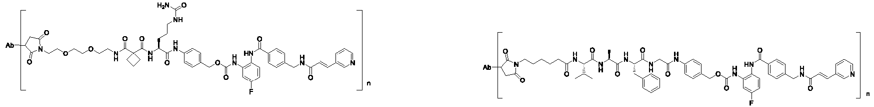

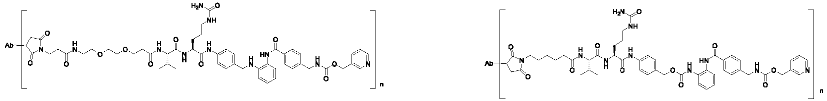

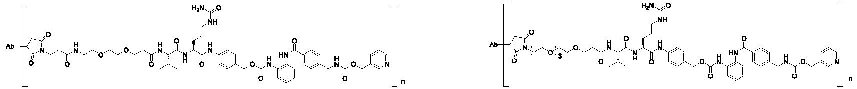

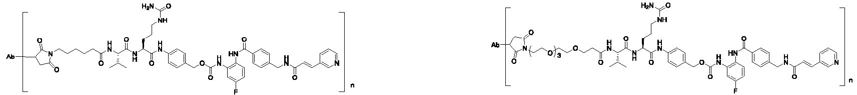

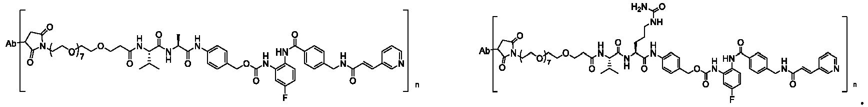

13. The ligand-drug conjugate, the stereoisomer thereof, the tautomer thereof, the pharmaceutically acceptable salt thereof, the deuterated compound thereof, or the solvate thereof according to any one of claims 7-12, wherein the ligand-drug conjugate is selected from:

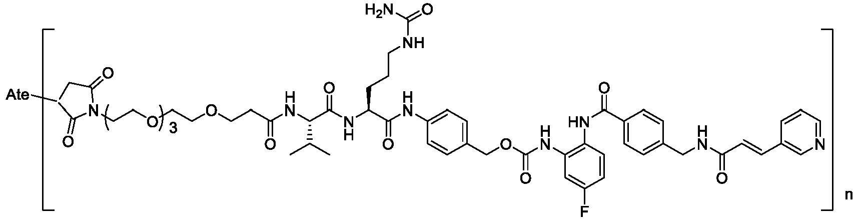

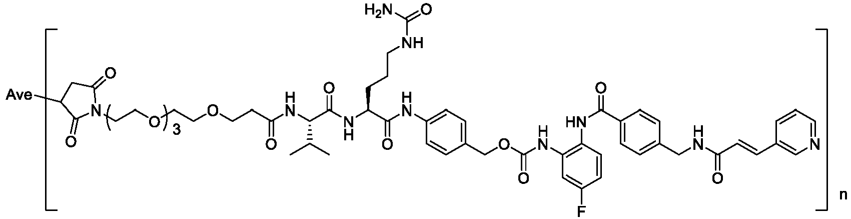

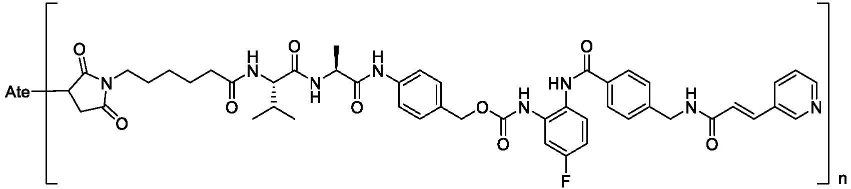

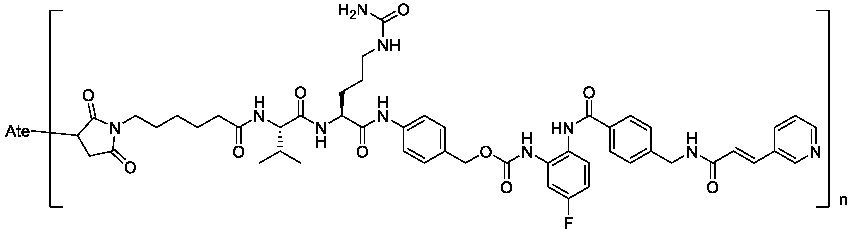

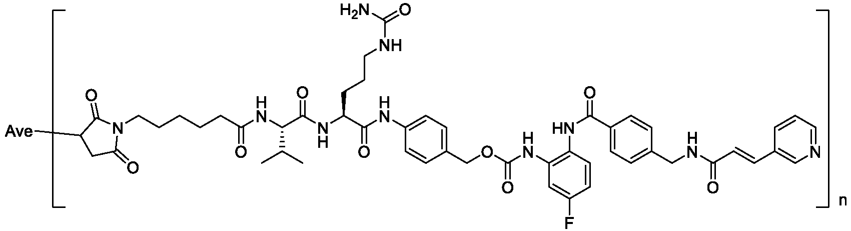

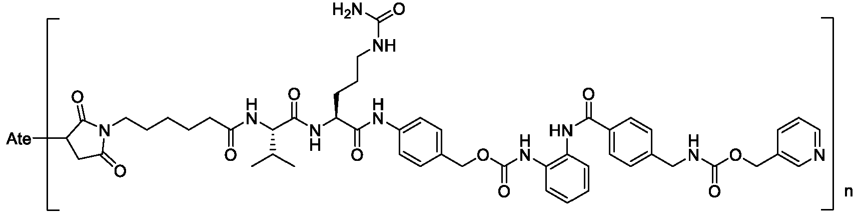

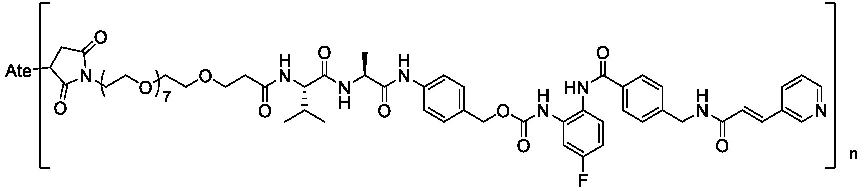

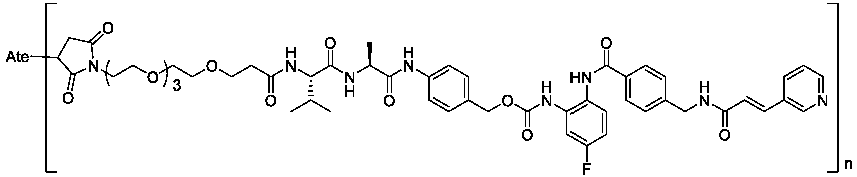

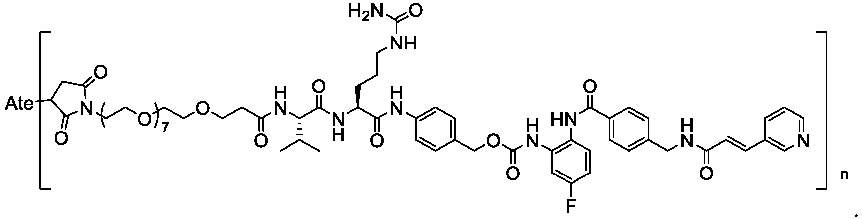

14. A pharmaceutical composition, comprising at least one of the compound, the stereoisomer thereof, the tautomer thereof, the pharmaceutically acceptable salt thereof, the deuterated compound thereof, or the solvate thereof according to any one of claims 1-5, or comprising at least one of the ligand-drug conjugate, the stereoisomer thereof, the tautomer thereof, the pharmaceutically acceptable salt thereof, the deuterated compound thereof, or the solvate thereof according to any one of claims 7-13, and optionally further comprising a pharmaceutically acceptable carrier or excipient.

15. Use of the compound, the stereoisomer thereof, the tautomer thereof, the pharmaceutically acceptable salt thereof, the deuterated compound thereof, or the solvate thereof according to any one of claims 1-5, or the ligand-drug conjugate, the stereoisomer thereof, the tautomer thereof, the pharmaceutically acceptable salt thereof, the deuterated compound thereof, or the solvate thereof according to any one of claims 7-13, or the pharmaceutical composition according to claim 14 in the preparation of a medicament for treating or preventing a disease, and
preferably, the disease is a tumor.
